# EUROPEAN PATENT APPLICATION

(11) **EP 2 420 573 A1**
(43) Date of publication of application: **22.02.2012**
(21) Application number: 10764307.4
(22) Date of filing: 19.02.2010
(51) Int. Cl.: C12N 15/09, C12N 1/19, C12N 1/21, C12N 5/10, C12N 9/02, C12Q 1/66

(54) **STABLE ARTIFICIAL BIOLUMINESCENT ENZYME HAVING SUPER-HIGH BRIGHTNESS**

(30) Priority: 17.04.2009 JP 2009101025
(71) Applicant: National Institute of Advanced Industrial Science and Technology, Tokyo 100-8921 (JP)
(72) Inventor: KIM SungBae, Tsukuba-shi Ibaraki 305-8569 (JP); TAO Hiroaki, Tsukuba-shi Ibaraki 305-8569 (JP); SATO Moritoshi, Tokyo 113-0023 (JP)
(74) Representative: HOFFMANN EITLE
(86) International application number: PCT/JP2010/052511
(87) International publication number: WO 2010/119721

(57) **Abstract**

This invention provides a luciferase variant that is produced by genetic engineering modification of a marine luciferase such as Gaussia luciferase, which has bioluminescence intensity, and has high bioluminescence stability or bioluminescence shifted to the longer wavelength side. Specifically disclosed is a luciferase variant with improved optical property obtained by replacing at least one amino acid residue among the amino acid sequence of a marine luciferase at positions corresponding to positions 89 to 118 in the amino acid sequence of Gaussia luciferase (GLuc), wherein an amino acid residue at a position corresponding to at least one position selected from positions 89, 90, 95, 97, 100, 108, 112, 115, and 118 in the amino acid sequence of GLuc is replaced by way of conservative amino acid replacement. The above-mentioned replacement in a marine luciferase improves enzymatic activity of the luciferase. Also disclosed is a bioluminescent probe having an improved optical property, which is produced using the luciferase variant of the present invention.

SEQUENCE LISTING

<110>  National Institute of Advanced Industrial Science and Technology

<120>  STABLE ARTIFICIAL LUCIFERASE HAVING SUPER-HIGH LUMINANCE

<130>  2009002915

<150>  JP2009-101025
<151>  2009-04-17

<150>  PCT/JP2010/052511
<151>  2010-02-19

<160>  46

<170>  PatentIn version 3.1

<210>  1
<211>  185
<212>  PRT
<213>  Artificial

<220>
<223>  GLuc mutant(I90L) version 1.0

<400>  1

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Leu Pro Gly Arg Cys His Thr
                85                  90                  95

Tyr Glu Gly Asp Lys Glu Ser Ala Gln Gly Gly Ile Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp

145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp
            180                 185

<210>  2
<211>  189
<212>  PRT
<213>  Artificial

<220>
<223>  GLuc mutant(I90L) version 1.1\201i+KDEL\201j

<400>  2

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Leu Pro Gly Arg Cys His Thr
                85                  90                  95

Tyr Glu Gly Asp Lys Glu Ser Ala Gln Gly Gly Ile Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  3
<211>  170
<212>  PRT
<213>  Artificial

<220>
<223>  GLuc mutant (I90L) version 1.2\201i-Signal peptide\201j

<400>  3

Met Gly Lys Pro Thr Glu Asn Asn Glu Asp Phe Asn Ile Val Ala Val
1               5                   10                  15

Ala Ser Asn Phe Ala Thr Thr Asp Leu Asp Ala Asp Arg Gly Lys Leu
            20                  25                  30

Pro Gly Lys Lys Leu Pro Leu Glu Val Leu Lys Glu Met Glu Ala Asn
        35                  40                  45

Ala Arg Lys Ala Gly Cys Thr Arg Gly Cys Leu Ile Cys Leu Ser His
    50                  55                  60

Ile Lys Cys Thr Pro Lys Met Lys Lys Phe Leu Pro Gly Arg Cys His
65                  70                  75                  80

Thr Tyr Glu Gly Asp Lys Glu Ser Ala Gln Gly Gly Ile Gly Glu Ala
                85                  90                  95

Ile Val Asp Ile Pro Glu Ile Pro Gly Phe Lys Asp Leu Glu Pro Met
            100                 105                 110

Glu Gln Phe Ile Ala Gln Val Asp Leu Cys Val Asp Cys Thr Thr Gly
        115                 120                 125

Cys Leu Lys Gly Leu Ala Asn Val Gln Cys Ser Asp Leu Leu Lys Lys
    130                 135                 140

Trp Leu Pro Gln Arg Cys Ala Thr Phe Ala Ser Lys Ile Gln Gly Gln
145                 150                 155                 160

Val Asp Lys Ile Lys Gly Ala Gly Gly Asp
                165                 170

<210>  4
<211>  174
<212>  PRT
<213>  Artificial

<220>
<223>  GLuc mutant (I90L) version 1.3\201i-Signal peptide, +KDEL)

<400>  4

Met Gly Lys Pro Thr Glu Asn Asn Glu Asp Phe Asn Ile Val Ala Val
1               5                   10                  15

Ala Ser Asn Phe Ala Thr Thr Asp Leu Asp Ala Asp Arg Gly Lys Leu
            20                  25                  30

Pro Gly Lys Lys Leu Pro Leu Glu Val Leu Lys Glu Met Glu Ala Asn
        35                  40                  45

Ala Arg Lys Ala Gly Cys Thr Arg Gly Cys Leu Ile Cys Leu Ser His
    50                  55                  60

Ile Lys Cys Thr Pro Lys Met Lys Lys Phe Leu Pro Gly Arg Cys His
65                  70                  75                  80

Thr Tyr Glu Gly Asp Lys Glu Ser Ala Gln Gly Gly Ile Gly Glu Ala
                85                  90                  95

Ile Val Asp Ile Pro Glu Ile Pro Gly Phe Lys Asp Leu Glu Pro Met
            100                 105                 110

Glu Gln Phe Ile Ala Gln Val Asp Leu Cys Val Asp Cys Thr Thr Gly
        115                 120                 125

Cys Leu Lys Gly Leu Ala Asn Val Gln Cys Ser Asp Leu Leu Lys Lys
    130                 135                 140

Trp Leu Pro Gln Arg Cys Ala Thr Phe Ala Ser Lys Ile Gln Gly Gln
145                 150                 155                 160

Val Asp Lys Ile Lys Gly Ala Gly Gly Asp Lys Asp Glu Leu
                165                 170


<210>  5
<211>  6
<212>  PRT
<213>  Artificial

<220>
<223>  His-Tag

<400>  5

His His His His His His
1               5


<210>  6
<211>  8
<212>  PRT
<213>  Artificial

<220>
<223>  FLAG-Tag\201iepitope\201j

<400>  6

Asp Tyr Lys Asp Asp Asp Asp Lys
1               5


<210>  7
<211>  10
<212>  PRT
<213>  Artificial

<220>
<223>  Myc-Tag\201iepitope\201j

<400>  7

Glu Gln Lys Leu Ile Ser Glu Glu Asp Leu
1               5                   10


<210>  8
<211>  9
<212>  PRT
<213>  Artificial

<220>
<223>  HA-Tag\201iepitope\201j

<400>  8

Tyr Pro Tyr Asp Val Pro Asp Tyr Ala
1               5


<210>  9
<211>  14
<212>  PRT
<213>  Artificial

<220>
<223>  V5-Tag\201iepitope\201j

<400>  9

Gly Lys Pro Ile Pro Asn Pro Leu Leu Gly Leu Asp Ser Thr
1               5                   10


<210>  10
<211>  11
<212>  PRT
<213>  Artificial

<220>
<223>  T7-Tag\201iepitope\201j

<400>  10

Met Ala Ser Met Thr Gly Gly Gln Gln Met Gly
1               5                   10

<210>  11
<211>  11
<212>  PRT
<213>  Artificial

<220>
<223>  Membrane-localization signal

<400>  11

Met Leu Cys Cys Met Arg Arg Thr Lys Gln Val
1               5                   10


<210>  12
<211>  10
<212>  PRT
<213>  Artificial

<220>
<223>  Cytosol-localization signal

<400>  12

Gly Arg Lys Lys Arg Arg Gln Arg Arg Arg
1               5                   10


<210>  13
<211>  8
<212>  PRT
<213>  Artificial

<220>
<223>  Nucleus-localization signal

<400>  13

Asp Pro Lys Lys Lys Arg Lys Val
1               5


<210>  14
<211>  4
<212>  PRT
<213>  Artificial

<220>
<223>  ER-localization signal

<400>  14

Lys Asp Glu Leu
1


<210>  15
<211>  189
<212>  PRT
<213>  Artificial

<220>
<223>  GLuc mutant (F89W)

<400>  15

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Trp Ile Pro Gly Arg Cys His Thr
                85                  90                  95

Tyr Glu Gly Asp Lys Glu Ser Ala Gln Gly Gly Ile Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185


<210>  16
<211>  189
<212>  PRT
<213>  Artificial

<220>
<223>  GLuc mutant (H95E)

<400>  16

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala

20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Ile Pro Gly Arg Cys Glu Thr
                85                  90                  95

Tyr Glu Gly Asp Lys Glu Ser Ala Gln Gly Gly Ile Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  17
<211>  189
<212>  PRT
<213>  Artificial

<220>
<223>  GLuc mutant (Y97W)

<400>  17

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Ile Pro Gly Arg Cys His Thr
                85                  90                  95

Trp Glu Gly Asp Lys Glu Ser Ala Gln Gly Gly Ile Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  18
<211>  189
<212>  PRT
<213>  Artificial

<220>
<223>  GLuc mutant (F89W/I90L); named 8990

<400>  18

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Trp Leu Pro Gly Arg Cys His Thr
                85                  90                  95

Tyr Glu Gly Asp Lys Glu Ser Ala Gln Gly Gly Ile Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  19
<211>  189
<212>  PRT
<213>  Artificial

<220>
<223>  GLuc mutant (I90L/I115L)

<400>  19

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Leu Pro Gly Arg Cys His Thr
                85                  90                  95

Tyr Glu Gly Asp Lys Glu Ser Ala Gln Gly Gly Ile Gly Glu Ala Ile
            100                 105                 110

Val Asp Leu Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  20
<211>  570
<212>  DNA
<213>  Artificial

<220>
<223>  A human codon-optimized GLuc variant cDNA (F89W/I90L/H95E/Y97W);
       Mon3

<400>  20
atgggcgtga aggtcctgtt cgccctgatc tgcatcgccg tggccgaggc caagcccacc      60

gagaacaacg aggacttcaa cattgtggcc gtggccagca actttgcgac cacggacctc     120

gacgccgatc gcgggaagtt gcccggaaag aagctgccgc tggaggtcct caaggagatg     180

gaggccaacg cccggaaggc cggctgcacc aggggatgcc tgatctgcct gtcccacatt     240

aagtgcacgc ccaagatgaa gaagtggctc cccggccgct gcgagacctg ggagggagac     300

aaggagtccg cccagggcgg tatcggagag gcgattgtcg atatcccgga gataccgggg     360

tttaaggatt tggagcccat ggagcagttc atcgcgcagg tcgatctttg cgtggactgc     420

acgacgggct gcctcaaggg gctcgccaac gtgcagtgct ccgacctgct caagaagtgg     480

ctaccgcagc gctgcgcgac cttcgccagc aagattcagg gacaggtgga caagatcaag     540

ggggccggcg gatccaagga cgagctgtga                                       570

<210>  21
<211>  189
<212>  PRT
<213>  Artificial

<220>
<223>  GLuc mutant (H95Q/D100N)

<400>  21

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Ile Pro Gly Arg Cys Gln Thr
                85                  90                  95

Tyr Glu Gly Asn Lys Glu Ser Ala Gln Gly Gly Ile Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  22
<211>  189
<212>  PRT
<213>  Artificial

<220>
<223>  GLuc mutant (I90V)

<400>  22

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro

35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Val Pro Gly Arg Cys His Thr
                85                  90                  95

Tyr Glu Gly Asn Lys Glu Ser Ala Gln Gly Gly Ile Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  23
<211>  189
<212>  PRT
<213>  Artificial

<220>
<223>  GLuc mutant (I108V)

<400>  23

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Ile Pro Gly Arg Cys His Thr
                85                  90                  95

Tyr Glu Gly Asn Lys Glu Ser Ala Gln Gly Gly Val Gly Glu Ala Ile
            100                 105                 110

Val Asp Ile Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  24
<211>  189
<212>  PRT
<213>  Artificial

<220>
<223>  GLuc mutant (I118V)

<400>  24

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Ile Pro Gly Arg Cys His Thr
                85                  90                  95

Tyr Glu Gly Asn Lys Glu Ser Ala Gln Gly Gly Ile Gly Glu Ala Ile
            100                 105                 110

Val Asp Ile Pro Glu Val Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185


<210>  25
<211>  189
<212>  PRT
<213>  Artificial

<220>
<223>  GLuc mutant (I90L/I115V)

<400>  25

Met Gly Val Lys Val Leu Phe Ala Leu Ile Cys Ile Ala Val Ala Glu
1               5                   10                  15

Ala Lys Pro Thr Glu Asn Asn Glu Asp Phe Asn Ile Val Ala Val Ala
            20                  25                  30

Ser Asn Phe Ala Thr Thr Asp Leu Asp Ala Asp Arg Gly Lys Leu Pro
        35                  40                  45

Gly Lys Lys Leu Pro Leu Glu Val Leu Lys Glu Met Glu Ala Asn Ala
    50                  55                  60

Arg Lys Ala Gly Cys Thr Arg Gly Cys Leu Ile Cys Leu Ser His Ile
65                  70                  75                  80

Lys Cys Thr Pro Lys Met Lys Lys Phe Leu Pro Gly Arg Cys His Thr
                85                  90                  95

Tyr Glu Gly Asn Lys Glu Ser Ala Gln Gly Gly Ile Gly Glu Ala Ile
            100                 105                 110

Val Asp Val Pro Glu Ile Pro Gly Phe Lys Asp Leu Glu Pro Met Glu
        115                 120                 125

Gln Phe Ile Ala Gln Val Asp Leu Cys Val Asp Cys Thr Thr Gly Cys
    130                 135                 140

Leu Lys Gly Leu Ala Asn Val Gln Cys Ser Asp Leu Leu Lys Lys Trp
145                 150                 155                 160

Leu Pro Gln Arg Cys Ala Thr Phe Ala Ser Lys Ile Gln Gly Gln Val
                165                 170                 175

Asp Lys Ile Lys Gly Ala Gly Gly Asp Lys Asp Glu Leu
            180                 185

<210>  26
<211>  214
<212>  PRT
<213>  Artificial

<220>
<223>  MpLuc1 mutant (Y113F)

<400>  26

Met Met Glu Ile Gln Val Leu Phe Ala Leu Ile Cys Phe Ala Leu Val
1               5                   10                  15

Gln Ala Asn Pro Thr Glu Asn Lys Asp Asp Ile Asp Ile Val Gly Val
            20                  25                  30

Glu Gly Lys Phe Gly Thr Thr Asp Leu Glu Thr Asp Leu Phe Thr Ile
        35                  40                  45

Val Glu Asp Met Asn Val Ile Ser Arg Asp Thr Asn Leu Ala Asn Ser
    50                  55                  60

Asp Ala Asp Arg Gly Lys Met Pro Gly Lys Lys Leu Pro Leu Glu Val
65                  70                  75                  80

Leu Ile Glu Met Glu Ala Asn Ala Arg Lys Ala Gly Cys Thr Arg Gly
                85                  90                  95

Cys Leu Ile Cys Leu Ser Lys Ile Lys Cys Thr Ala Lys Met Lys Val
            100                 105                 110

Phe Ile Pro Gly Arg Cys His Asp Tyr Gly Gly Asp Lys Lys Thr Gly
        115                 120                 125

Gln Ala Gly Ile Val Gly Ala Ile Val Asp Ile Pro Glu Ile Ser Gly
    130                 135                 140

Phe Lys Glu Leu Gly Pro Met Glu Gln Phe Ile Ala Gln Val Asp Leu

145                 150                 155                 160

Cys Ala Asp Cys Thr Thr Gly Cys Leu Lys Gly Leu Ala Asn Val Lys
                165                 170                 175

Cys Ser Ala Leu Leu Lys Lys Trp Leu Pro Asp Arg Cys Ala Ser Phe
            180                 185                 190

Ala Asp Lys Ile Gln Ser Glu Val Asp Asn Ile Lys Gly Leu Ala Gly
        195                 200                 205

Asp Arg Lys Asp Glu Leu
    210

<210>  27
<211>  214
<212>  PRT
<213>  Artificial

<220>
<223>  MpLuc1 mutant (I114L)

<400>  27

Met Met Glu Ile Gln Val Leu Phe Ala Leu Ile Cys Phe Ala Leu Val
1               5                   10                  15

Gln Ala Asn Pro Thr Glu Asn Lys Asp Asp Ile Asp Ile Val Gly Val
            20                  25                  30

Glu Gly Lys Phe Gly Thr Thr Asp Leu Glu Thr Asp Leu Phe Thr Ile
        35                  40                  45

Val Glu Asp Met Asn Val Ile Ser Arg Asp Thr Asn Leu Ala Asn Ser
    50                  55                  60

Asp Ala Asp Arg Gly Lys Met Pro Gly Lys Lys Leu Pro Leu Glu Val
65                  70                  75                  80

Leu Ile Glu Met Glu Ala Asn Ala Arg Lys Ala Gly Cys Thr Arg Gly
                85                  90                  95

Cys Leu Ile Cys Leu Ser Lys Ile Lys Cys Thr Ala Lys Met Lys Val
            100                 105                 110

Tyr Leu Pro Gly Arg Cys His Asp Tyr Gly Gly Asp Lys Lys Thr Gly
        115                 120                 125

Gln Ala Gly Ile Val Gly Ala Ile Val Asp Ile Pro Glu Ile Ser Gly
    130                 135                 140

Phe Lys Glu Leu Gly Pro Met Glu Gln Phe Ile Ala Gln Val Asp Leu
145                 150                 155                 160

Cys Ala Asp Cys Thr Thr Gly Cys Leu Lys Gly Leu Ala Asn Val Lys
                165                 170                 175

Cys Ser Ala Leu Leu Lys Lys Trp Leu Pro Asp Arg Cys Ala Ser Phe
            180                 185                 190

Ala Asp Lys Ile Gln Ser Glu Val Asp Asn Ile Lys Gly Leu Ala Gly
        195                 200                 205

Asp Arg Lys Asp Glu Leu
    210

<210>  28
<211>  210
<212>  PRT
<213>  Artificial

<220>
<223>  MpLuc1 mutant (Y113W/I114L/H119E/Y121W); MpLuc4

<400>  28

Met Met Glu Ile Gln Val Leu Phe Ala Leu Ile Cys Phe Ala Leu Val
1               5                   10                  15

Gln Ala Asn Pro Thr Glu Asn Lys Asp Asp Ile Asp Ile Val Gly Val
            20                  25                  30

Glu Gly Lys Phe Gly Thr Thr Asp Leu Glu Thr Asp Leu Phe Thr Ile
        35                  40                  45

Val Glu Asp Met Asn Val Ile Ser Arg Asp Thr Asn Leu Ala Asn Ser
    50                  55                  60

Asp Ala Asp Arg Gly Lys Met Pro Gly Lys Lys Leu Pro Leu Glu Val
65                  70                  75                  80

Leu Ile Glu Met Glu Ala Asn Ala Arg Lys Ala Gly Cys Thr Arg Gly
                85                  90                  95

Cys Leu Ile Cys Leu Ser Lys Ile Lys Cys Thr Ala Lys Met Lys Val
            100                 105                 110

Trp Leu Pro Gly Arg Cys Glu Asp Trp Gly Gly Asp Lys Lys Thr Gly
        115                 120                 125

Gln Ala Gly Ile Val Gly Ala Ile Val Asp Ile Pro Glu Ile Ser Gly
    130                 135                 140

Phe Lys Glu Leu Gly Pro Met Glu Gln Phe Ile Ala Gln Val Asp Leu
145                 150                 155                 160

Cys Ala Asp Cys Thr Thr Gly Cys Leu Lys Gly Leu Ala Asn Val Lys
                165                 170                 175

Cys Ser Ala Leu Leu Lys Lys Trp Leu Pro Asp Arg Cys Ala Ser Phe
            180                 185                 190

Ala Asp Lys Ile Gln Ser Glu Val Asp Asn Ile Lys Gly Leu Ala Gly
        195                 200                 205

Asp Arg
    210

<210>  29
<211>  219
<212>  PRT
<213>  Artificial

<220>
<223>  MLuc mutant (I123L)

<400>  29

Met Asp Ile Lys Val Val Phe Thr Leu Val Phe Ser Ala Leu Val Gln
1               5                   10                  15

Ala Lys Ser Thr Glu Phe Asp Pro Asn Ile Asp Ile Val Gly Leu Glu
            20                  25                  30

Gly Lys Phe Gly Ile Thr Asn Leu Glu Thr Asp Leu Phe Thr Ile Trp
        35                  40                  45

Glu Thr Met Glu Val Met Ile Lys Ala Asp Ile Ala Asp Thr Asp Arg
    50                  55                  60

Ala Ser Asn Phe Val Ala Thr Glu Thr Asp Ala Asn Arg Gly Lys Met
65                  70                  75                  80

Pro Gly Lys Lys Leu Pro Leu Ala Val Ile Met Glu Met Glu Ala Asn
                85                  90                  95

Ala Phe Lys Ala Gly Cys Thr Arg Gly Cys Leu Ile Cys Leu Ser Lys
            100                 105                 110

Ile Lys Cys Thr Ala Lys Met Lys Val Tyr Leu Pro Gly Arg Cys His
        115                 120                 125

Asp Tyr Gly Gly Asp Lys Lys Thr Gly Gln Ala Gly Ile Val Gly Ala
    130                 135                 140

Ile Val Asp Ile Pro Glu Ile Ser Gly Phe Lys Glu Met Ala Pro Met
145                 150                 155                 160

Glu Gln Phe Ile Ala Gln Val Asp Arg Cys Ala Ser Cys Thr Thr Gly
                165                 170                 175

Cys Leu Lys Gly Leu Ala Asn Val Lys Cys Ser Glu Leu Leu Lys Lys
            180                 185                 190

Trp Leu Pro Asp Arg Cys Ala Ser Phe Ala Asp Lys Ile Gln Lys Glu
        195                 200                 205

Val His Asn Ile Lys Gly Met Ala Gly Asp Arg
    210                 215

<210>  30
<211>  219
<212>  PRT
<213>  Artificial

<220>
<223>  MLuc mutant (Y122W/I123L/H128E/Y130W); MLuc4

<400>  30

Met Asp Ile Lys Val Val Phe Thr Leu Val Phe Ser Ala Leu Val Gln
1               5                   10                  15

Ala Lys Ser Thr Glu Phe Asp Pro Asn Ile Asp Ile Val Gly Leu Glu
            20                  25                  30

Gly Lys Phe Gly Ile Thr Asn Leu Glu Thr Asp Leu Phe Thr Ile Trp
        35                  40                  45

Glu Thr Met Glu Val Met Ile Lys Ala Asp Ile Ala Asp Thr Asp Arg
    50                  55                  60

Ala Ser Asn Phe Val Ala Thr Glu Thr Asp Ala Asn Arg Gly Lys Met
65                  70                  75                  80

Pro Gly Lys Lys Leu Pro Leu Ala Val Ile Met Glu Met Glu Ala Asn
                85                  90                  95

Ala Phe Lys Ala Gly Cys Thr Arg Gly Cys Leu Ile Cys Leu Ser Lys
            100                 105                 110

Ile Lys Cys Thr Ala Lys Met Lys Val Trp Leu Pro Gly Arg Cys Glu
        115                 120                 125

Asp Trp Gly Gly Asp Lys Lys Thr Gly Gln Ala Gly Ile Val Gly Ala

130                 135                 140

Ile Val Asp Ile Pro Glu Ile Ser Gly Phe Lys Glu Met Ala Pro Met
145                 150                 155                 160

Glu Gln Phe Ile Ala Gln Val Asp Arg Cys Ala Ser Cys Thr Thr Gly
                165                 170                 175

Cys Leu Lys Gly Leu Ala Asn Val Lys Cys Ser Glu Leu Leu Lys Lys
            180                 185                 190

Trp Leu Pro Asp Arg Cys Ala Ser Phe Ala Asp Lys Ile Gln Lys Glu
        195                 200                 205

Val His Asn Ile Lys Gly Met Ala Gly Asp Arg
    210                 215

<210>  31
<211>  195
<212>  PRT
<213>  Artificial

<220>
<223>  Obelia luciferase mutant (L95I/A96L/K101E/W130L); OLuc4

<400>  31

Met Ser Ser Lys Tyr Ala Val Lys Leu Lys Thr Asp Phe Asp Asn Pro
1               5                   10                  15

Arg Trp Ile Lys Arg His Lys His Met Phe Asp Phe Leu Asp Ile Asn
            20                  25                  30

Gly Asn Gly Lys Ile Thr Leu Asp Glu Ile Val Ser Lys Ala Ser Asp
        35                  40                  45

Asp Ile Cys Ala Lys Leu Glu Ala Thr Pro Glu Gln Thr Lys Arg His
    50                  55                  60

Gln Val Cys Val Glu Ala Phe Phe Arg Gly Cys Gly Met Glu Tyr Gly
65                  70                  75                  80

Lys Glu Ile Ala Phe Pro Gln Phe Leu Asp Gly Trp Lys Gln Ile Leu
                85                  90                  95

Thr Ser Glu Leu Glu Lys Leu Ala Arg Asn Glu Pro Thr Leu Ile Arg
            100                 105                 110

Glu Trp Gly Asp Ala Val Phe Asp Ile Phe Asp Lys Asp Gly Ser Gly
        115                 120                 125

Thr Ile Thr Leu Asp Glu Trp Lys Ala Tyr Gly Lys Ile Ser Gly Ile
    130                 135                 140

Ser Pro Ser Gln Glu Asp Cys Glu Ala Thr Phe Arg His Cys Asp Leu
145                 150                 155                 160

Asp Asn Ser Gly Asp Leu Asp Val Asp Glu Met Thr Arg Gln His Leu
                165                 170                 175

Gly Phe Trp Tyr Thr Leu Asp Pro Glu Ala Asp Gly Leu Tyr Gly Asn
            180                 185                 190

Gly Val Pro
        195

<210>  32
<211>  196
<212>  PRT
<213>  Artificial

<220>
<223>  aqualine luciferase mutant (L96I/A97L/E102H/Y104W); ALuc4

<400>  32

Met Thr Ser Lys Gln Tyr Ser Val Lys Leu Thr Ser Asp Phe Asp Asn
1               5                   10                  15

Pro Arg Trp Ile Gly Arg His Lys His Met Phe Asn Phe Leu Asp Val
            20                  25                  30

Asn His Asn Gly Lys Ile Ser Leu Asp Glu Met Val Tyr Lys Ala Ser
        35                  40                  45

Asp Ile Val Ile Asn Asn Leu Gly Ala Thr Pro Glu Gln Ala Lys Arg
    50                  55                  60

His Lys Asp Ala Val Glu Ala Phe Phe Gly Gly Ala Gly Met Lys Tyr
65                  70                  75                  80

Gly Val Glu Thr Asp Trp Pro Ala Tyr Ile Glu Gly Trp Lys Lys Ile
                85                  90                  95

Leu Thr Asp Glu Leu His Lys Trp Ala Lys Asn Glu Pro Thr Leu Ile
            100                 105                 110

Arg Ile Trp Gly Asp Ala Leu Phe Asp Ile Val Asp Lys Asp Gln Asn
        115                 120                 125

Gly Ala Ile Thr Leu Asp Glu Trp Lys Ala Tyr Thr Lys Ala Ala Gly
    130                 135                 140

Ile Ile Gln Ser Ser Glu Asp Trp Glu Glu Thr Phe Arg Val Cys Asp
145                 150                 155                 160

Ile Asp Glu Ser Gly Gln Leu Asp Val Asp Glu Met Thr Arg Gln His
                165                 170                 175

Leu Gly Phe Trp Tyr Thr Met Asp Pro Ala Cys Glu Lys Leu Tyr Gly
            180                 185                 190

Gly Ala Val Pro
        195

<210>  33
<211>  189
<212>  PRT
<213>  Artificial

<220>
<223>  MpLuc2 luciferase mutant (F92W/I93L/H98E/Y100W); MpLuc5

<400>  33

Met Gly Val Lys Leu Ile Phe Ala Val Leu Cys Val Ala Ala Ala Gln
1               5                   10                  15

Ala Ala Thr Ile Asn Glu Asn Phe Glu Asp Ile Asp Val Val Ala Ile
            20                  25                  30

Gly Gly Ser Phe Ala Leu Asp Val Asp Ala Asn Arg Gly Gly His Gly
        35                  40                  45

Gly His Pro Gly Lys Lys Met Pro Lys Glu Val Leu Val Glu Met Glu
    50                  55                  60

Ala Asn Ala Lys Arg Ala Gly Cys His Arg Gly Cys Leu Ile Cys Leu
65                  70                  75                  80

Ser His Ile Lys Cys Thr Lys Lys Met Lys Lys Trp Leu Pro Gly Arg
                85                  90                  95

Cys Glu Ser Trp Glu Gly Asp Lys Asp Ser Ala Gln Gly Gly Ile Gly
            100                 105                 110

Glu Glu Ile Val Asp Met Pro Glu Ile Pro Gly Phe Lys Asp Lys Glu
        115                 120                 125

Pro Met Asp Gln Phe Ile Ala Gln Val Asp Leu Cys Val Asp Cys Thr
    130                 135                 140

Thr Gly Cys Leu Lys Gly Leu Ala Asn Val His Cys Ser Ala Leu Leu
145                 150                 155                 160

Lys Lys Trp Leu Pro Ser Arg Cys Lys Thr Phe Ala Ser Lys Ile Gln
                165                 170                 175

Ser Gln Val Asp Thr Ile Lys Gly Leu Ala Gly Asp Arg
            180                 185

<210>  34
<211>  196
<212>  PRT
<213>  Artificial

<220>
<223>  Oplophorus luciferase mutant (I103L/F104W/Y108W/V110L); OpLuc4

<400>  34

Met Ala Tyr Ser Thr Leu Phe Ile Ile Ala Leu Thr Ala Val Val Thr
1               5                   10                  15

Gln Ala Ser Ser Thr Gln Lys Ser Asn Leu Thr Phe Thr Leu Ala Asp
            20                  25                  30

Phe Val Gly Asp Trp Gln Gln Thr Ala Gly Tyr Asn Gln Asp Gln Val
        35                  40                  45

Leu Glu Gln Gly Gly Leu Ser Ser Leu Phe Gln Ala Leu Gly Val Ser
    50                  55                  60

Val Thr Pro Ile Gln Lys Val Val Leu Ser Gly Glu Asn Gly Leu Lys
65                  70                  75                  80

Ala Asp Ile His Val Ile Ile Pro Tyr Glu Gly Leu Ser Gly Phe Gln
                85                  90                  95

Met Gly Leu Ile Glu Met Leu Trp Lys Val Val Trp Pro Leu Asp Asp
            100                 105                 110

His His Phe Lys Ile Ile Leu His Tyr Gly Thr Leu Val Ile Asp Gly
        115                 120                 125

Val Thr Pro Asn Met Ile Asp Tyr Phe Gly Arg Pro Tyr Pro Gly Ile
    130                 135                 140

Ala Val Phe Asp Gly Lys Gln Ile Thr Val Thr Gly Thr Leu Trp Asn
145                 150                 155                 160

Gly Asn Lys Ile Tyr Asp Glu Arg Leu Ile Asn Pro Asp Gly Ser Leu
                165                 170                 175

Leu Phe Arg Val Thr Ile Asn Gly Val Thr Gly Trp Arg Leu Cys Glu

180                 185                 190

Asn Ile Leu Ala
        195

<210>  35
<211>  198
<212>  PRT
<213>  Artificial

<220>
<223>  Pleuromamma luciferase mutant (T101W/W102L/E109H/D111W); PLuc4

<400>  35

Met Leu Arg Asn Cys Ala Arg Lys Gln Glu Gln Val Cys Ala Asp Val
1               5                   10                  15

Thr Glu Met Lys Cys Gln Ala Val Ala Trp Ala Asp Cys Gly Pro Arg
            20                  25                  30

Phe Asp Ser Thr Gly Arg Asn Arg Cys Gln Val Gln Tyr Lys Asp Tyr
        35                  40                  45

Ala Tyr Lys Ser Cys Val Glu Val Asp Tyr Thr Val Pro His Arg Lys
    50                  55                  60

Gln Val Pro Glu Cys Lys Gln Val Thr Lys Asp Asn Cys Val Thr Asp
65                  70                  75                  80

Trp Glu Val Asp Ala Asn Gly Asn Lys Val Trp Gly Gly Thr Glu Lys
                85                  90                  95

Cys Thr Pro Val Trp Leu Glu Glu Cys Asn Ile Val His Lys Trp Val
            100                 105                 110

Asp Phe Pro Thr Val Lys Thr Glu Cys Gly Ile Leu Ser His Leu Lys
        115                 120                 125

Tyr Ala Asp Phe Ile Glu Gly Pro Ser His Ser Leu Ser Met Arg Thr
    130                 135                 140

Asn Cys Gln Val Lys Ser Ser Leu Asp Cys Arg Pro Val Lys Thr Arg
145                 150                 155                 160

Lys Cys Ala Thr Val Glu Tyr His Glu Cys Ser Met Lys Pro Gln Glu
                165                 170                 175

Asp Cys Ser Pro Val Thr Val His Ile Pro Asp Gln Glu Lys Val His
            180                 185                 190

Gln Lys Lys Cys Leu Thr
        195

<210>  36
<211>  311
<212>  PRT
<213>  Artificial

<220>
<223>  Renilla luciferase mutant (F105W/E106L/Y129L/Y131W); RLuc4

<400>  36

Met Ala Ser Lys Val Tyr Asp Pro Glu Gln Arg Lys Arg Met Ile Thr
1               5                   10                  15

Gly Pro Gln Trp Trp Ala Arg Cys Lys Gln Met Asn Val Leu Asp Ser
            20                  25                  30

Phe Ile Asn Tyr Tyr Asp Ser Glu Lys His Ala Glu Asn Ala Val Ile
        35                  40                  45

Phe Leu His Gly Asn Ala Ala Ser Ser Tyr Leu Trp Arg His Val Val
    50                  55                  60

Pro His Ile Glu Pro Val Ala Arg Cys Ile Ile Pro Asp Leu Ile Gly
65                  70                  75                  80

Met Gly Lys Ser Gly Lys Ser Gly Asn Gly Ser Tyr Arg Leu Leu Asp
                85                  90                  95

His Tyr Lys Tyr Leu Thr Ala Trp Trp Leu Leu Ile Asn Ile Pro Lys
            100                 105                 110

Lys Ile Ile Phe Val Gly His Asp Trp Gly Ala Cys Leu Ala Phe His
        115                 120                 125

Leu Ser Trp Glu His Gln Asp Lys Ile Lys Ala Ile Val His Ala Glu
    130                 135                 140

Ser Val Val Asp Val Ile Glu Ser Trp Asp Glu Trp Pro Asp Ile Glu
145                 150                 155                 160

Glu Asp Ile Ala Leu Ile Lys Ser Glu Glu Gly Glu Lys Met Val Leu
                165                 170                 175

Glu Asn Asn Phe Phe Val Glu Thr Met Leu Pro Ser Lys Ile Met Arg
            180                 185                 190

Lys Leu Glu Pro Glu Glu Phe Ala Ala Tyr Leu Glu Pro Phe Lys Glu
        195                 200                 205

Lys Gly Glu Val Arg Arg Pro Thr Leu Ser Trp Pro Arg Glu Ile Pro
    210                 215                 220

Leu Val Lys Gly Gly Lys Pro Asp Val Val Gln Ile Val Arg Asn Tyr
225                 230                 235                 240

Asn Ala Tyr Leu Arg Ala Ser Asp Asp Leu Pro Lys Met Phe Ile Glu
                245                 250                 255

Ser Asp Pro Gly Phe Phe Ser Asn Ala Ile Val Glu Gly Ala Lys Lys
            260                 265                 270

Phe Pro Asn Thr Glu Phe Val Lys Val Lys Gly Leu His Phe Ser Gln
        275                 280                 285

Glu Asp Ala Pro Asp Glu Met Gly Lys Tyr Ile Lys Ser Phe Val Glu
    290                 295                 300

Arg Val Leu Lys Asn Glu Gln
305                 310

<210>  37
<211>  660
<212>  DNA
<213>  Artificial

<220>
<223>  A human codon-optimized MLuc variant cDNA; 4 mutated

<400>  37
atggacataa aggtggtgtt tacgctggta tttagcgcac tggttcaggc caaatcgacg      60

gagtttgatc cgaatattga cattgttgga ttggaaggca aattcggcat taccaacctg     120

gaaaccgatc tgttcaccat ctgggaaacg atggaggtca tgatcaaagc cgacattgcc     180

gataccgatc gggcttcgaa ctttgttgct acagaaacgg atgcaaatcg cgggaaaatg     240

ccgggcaaga aactgccgtt agcggtaatc atggaaatgg aagcgaacgc gttcaaagcg     300

ggttgcacac gtgggtgtct gatctgcttg agcaagatca aatgcacggc caaaatgaaa     360

gtctggttac caggacgttg cgaagattgg ggtggcgaca agaaaaccgg tcaagcaggc     420

attgtgggtg cgattgtgga tatccccgaa atttctggct tcaaagagat ggcgccgatg     480

gaacagttca ttgcccaagt tgaccgctgt gcgagttgca ctactggctg tctgaaaggg     540

cttgcgaatg tcaaatgctc agaactgctc aagaaatggc tccctgatcg ttgtgcctcc     600

tttgcagaca aaatccagaa agaggtgcat aacatcaaag gtatggctgg tgatcgctaa     660

<210>  38
<211>  633
<212>  DNA
<213>  Artificial

<220>
<223>  human codon-optimized MpLuc1 cDNA; 4-mutated

<400>  38
atgatggaaa tacaagttct ttttgctctc atttgctttg cattggtgca ggccaatccc      60

actgagaata aggacgacat cgacatcgtt ggagtcgaag gcaagtttgg gaccactgat     120

ctggaaacag acctgttcac catcgttgag gatatgaacg tgatttcccg agacacgaat     180

ctggcgaact ctgacgcaga tagagggaag atgccaggta agaaactccc tctggaagtg     240

ttgatagaga tggaggctaa tgcccggaaa gcaggatgca caagaggctg cctgatttgc     300

ctcagcaaaa tcaagtgtac agccaagatg aaagtctggc tgcccggccg ttgcgaggat     360

tggggcggtg acaagaagac cggacaagcc gggatagtag gtgccatcgt ggatatcccg     420

gagatttccg ggttcaaaga gctgggacct atggaacagt tcattgcgca ggtggacttg     480

tgtgccgact gtaccactgg ctgtctgaaa ggccttgcaa acgtgaaatg ctcagctctg     540

ctcaagaaat ggctgccaga tcgctgtgcc agctttgccg acaagatcca gagtgaagtg     600

gacaacatca agggacttgc tggggatagg tga                                  633


<210>  39
<211>  582
<212>  DNA
<213>  Artificial

<220>
<223>  human codon-optimized MpLuc1 cDNA; 1-mutated variant without a se
       cretion peptide

<400>  39
atgggcccca ctgagaataa ggacgacatc gacatcgttg gagtcgaagg caagtttggg      60

accactgatc tggaaacaga cctgttcacc atcgttgagg atatgaacgt gatttcccga     120

gacacgaatc tggcgaactc tgacgcagat agagggaaga tgccaggtaa gaaactccct     180

ctggaagtgt tgatagagat ggaggctaat gcccggaaag caggatgcac aagaggctgc     240

ctgatttgcc tcagcaaaat caagtgtaca gccaagatga aagtctacct gcccggccgt     300

tgccacgatt atggcggtga caagaagacc ggacaagccg ggatagtagg tgccatcgtg     360

gatatcccgg agatttccgg gttcaaagag ctgggaccta tggaacagtt cattgcgcag     420

gtggacttgt gtgccgactg taccactggc tgtctgaaag gccttgcaaa cgtgaaatgc     480

tcagctctgc tcaagaaatg gctgccagat cgctgtgcca gctttgccga caagatccag     540

agtgaagtgg acaacatcaa gggacttgct ggggataggt ga                        582


<210>  40
<211>  494
<212>  PRT
<213>  Artificial

<220>

<223>  cPresso; an integrated molecule-format probe carrying circularly
       permutated GLuc.

<400>  40

Met Asn Ala Leu Leu Arg Tyr Leu Leu Asp Lys Asp Gly Ser Gly Gly
1               5                   10                  15

Gly Gly Ile Gly Glu Ala Ile Val Asp Ile Pro Glu Ile Pro Gly Phe
            20                  25                  30

Lys Asp Leu Glu Pro Met Glu Gln Phe Ile Ala Gln Val Asp Leu Cys
        35                  40                  45

Val Asp Cys Thr Thr Gly Cys Leu Lys Gly Leu Ala Asn Val Gln Cys
    50                  55                  60

Ser Asp Leu Leu Lys Lys Trp Leu Pro Gln Arg Cys Ala Thr Phe Ala
65                  70                  75                  80

Ser Lys Ile Gln Gly Gln Val Asp Lys Ile Lys Gly Ala Gly Gly Asp
                85                  90                  95

Lys Asp Glu Leu Gly Gly Ser Gly Arg Gly Gly Met Lys Pro Thr Glu
            100                 105                 110

Asn Asn Glu Asp Phe Asn Ile Val Ala Val Ala Ser Asn Phe Ala Thr
        115                 120                 125

Thr Asp Leu Asp Ala Asp Arg Gly Lys Leu Pro Gly Lys Lys Leu Pro
    130                 135                 140

Leu Glu Val Leu Lys Glu Met Glu Ala Asn Ala Arg Lys Ala Gly Cys
145                 150                 155                 160

Thr Arg Gly Cys Leu Ile Cys Leu Ser His Ile Lys Cys Thr Pro Lys
                165                 170                 175

Met Lys Lys Phe Ile Pro Gly Arg Cys His Thr Tyr Glu Gly Asp Lys
            180                 185                 190

Glu Ser Ala Gln Gly Gly Gly Gly Gly Thr Met Asn Leu Glu Ala Arg
        195                 200                 205

Lys Thr Lys Lys Lys Ile Lys Gly Ile Gln Gln Ala Thr Thr Gly Val
    210                 215                 220

Ser Gln Glu Thr Ser Glu Asn Pro Gly Asn Lys Thr Ile Val Pro Ala
225                 230                 235                 240

Thr Leu Pro Gln Leu Thr Pro Thr Leu Val Ser Leu Leu Glu Val Ile
                245                 250                 255

Glu Pro Glu Val Leu Tyr Ala Gly Tyr Asp Ser Ser Val Pro Asp Ser
            260                 265                 270

Thr Trp Arg Ile Met Thr Thr Leu Asn Met Leu Gly Gly Arg Gln Val
        275                 280                 285

Ile Ala Ala Val Lys Trp Ala Lys Ala Ile Pro Gly Phe Arg Asn Leu
    290                 295                 300

His Leu Asp Asp Gln Met Thr Leu Leu Gln Tyr Ser Trp Met Phe Leu
305                 310                 315                 320

Met Ala Phe Ala Leu Gly Trp Arg Ser Tyr Arg Gln Ser Ser Ala Asn
                325                 330                 335

Leu Leu Cys Phe Ala Pro Asp Leu Ile Ile Asn Glu Gln Arg Met Thr
            340                 345                 350

Leu Pro Cys Met Tyr Asp Gln Cys Lys His Met Leu Tyr Val Ser Ser
        355                 360                 365

Glu Leu His Arg Leu Gln Val Ser Tyr Glu Glu Tyr Leu Cys Met Lys
    370                 375                 380

Thr Leu Leu Leu Leu Ser Ser Val Pro Lys Asp Gly Leu Lys Ser Gln
385                 390                 395                 400

Glu Leu Phe Asp Glu Ile Arg Met Thr Tyr Ile Lys Glu Leu Gly Lys
                405                 410                 415

Ala Ile Val Lys Arg Glu Gly Asn Ser Ser Gln Asn Trp Gln Arg Phe
            420                 425                 430

Tyr Gln Leu Thr Lys Leu Leu Asp Ser Met His Glu Val Val Glu Asn
        435                 440                 445

Leu Leu Asn Tyr Cys Phe Gln Thr Phe Leu Asp Lys Thr Met Ser Ile
    450                 455                 460

Glu Phe Pro Glu Met Leu Ala Glu Ile Ile Thr Asn Gln Ile Pro Lys
465                 470                 475                 480

Tyr Ser Asn Gly Asn Ile Lys Lys Leu Leu Phe His Gln Lys
                485                 490

<210>  41

<211>  506
<212>  PRT
<213>  Artificial

<220>
<223>  cPressoMAX; an integrated molecule-format bioluminescent probe ca
       rrying a superluminescent GLuc mutant.

<400>  41

Met Glu Gln Lys Leu Ile Ser Glu Glu Asp Leu His Met Asn Ala Leu
1               5                   10                  15

Leu Arg Tyr Leu Leu Asp Lys Asp Leu Lys Gly Gly Gly Gly Ile Gly
            20                  25                  30

Glu Ala Ile Val Asp Ile Pro Glu Ile Pro Gly Phe Lys Asp Leu Glu
        35                  40                  45

Pro Met Glu Gln Phe Ile Ala Gln Val Asp Leu Cys Val Asp Cys Thr
    50                  55                  60

Thr Gly Cys Leu Lys Gly Leu Ala Asn Val Gln Cys Ser Asp Leu Leu
65                  70                  75                  80

Lys Lys Trp Leu Pro Gln Arg Cys Ala Thr Phe Ala Ser Lys Ile Gln
                85                  90                  95

Gly Gln Val Asp Lys Ile Lys Gly Ala Gly Gly Asp Lys Asp Glu Leu
            100                 105                 110

Gly Asp Ile Gly Arg Gly Gly Met Lys Pro Thr Glu Asn Asn Glu Asp
        115                 120                 125

Phe Asn Ile Val Ala Val Ala Ser Asn Phe Ala Thr Thr Asp Leu Asp
    130                 135                 140

Ala Asp Arg Gly Lys Leu Pro Gly Lys Lys Leu Pro Leu Glu Val Leu
145                 150                 155                 160

Lys Glu Met Glu Ala Asn Ala Arg Lys Ala Gly Cys Thr Arg Gly Cys
                165                 170                 175

Leu Ile Cys Leu Ser His Ile Lys Cys Thr Pro Lys Met Lys Lys Trp
            180                 185                 190

Leu Pro Gly Arg Cys His Thr Tyr Glu Gly Asp Lys Glu Ser Ala Gln
        195                 200                 205

Gly Gly Gly Gly Gly Thr Met Asn Leu Glu Ala Arg Lys Thr Lys Lys
    210                 215                 220

Lys Ile Lys Gly Ile Gln Gln Ala Thr Thr Gly Val Ser Gln Glu Thr
225                 230                 235                 240

Ser Glu Asn Pro Gly Asn Lys Thr Ile Val Pro Ala Thr Leu Pro Gln
                245                 250                 255

Leu Thr Pro Thr Leu Val Ser Leu Leu Glu Val Ile Glu Pro Glu Val
            260                 265                 270

Leu Tyr Ala Gly Tyr Asp Ser Ser Val Pro Asp Ser Thr Trp Arg Ile
        275                 280                 285

Met Thr Thr Leu Asn Met Leu Gly Gly Arg Gln Val Ile Ala Ala Val
    290                 295                 300

Lys Trp Ala Lys Ala Ile Pro Gly Phe Arg Asn Leu His Leu Asp Asp
305                 310                 315                 320

Gln Met Thr Leu Leu Gln Tyr Ser Trp Met Phe Leu Met Ala Phe Ala
                325                 330                 335

Leu Gly Trp Arg Ser Tyr Arg Gln Ser Ser Ala Asn Leu Leu Cys Phe
            340                 345                 350

Ala Pro Asp Leu Ile Ile Asn Glu Gln Arg Met Thr Leu Pro Cys Met
        355                 360                 365

Tyr Asp Gln Cys Lys His Met Leu Tyr Val Ser Ser Glu Leu His Arg
    370                 375                 380

Leu Gln Val Ser Tyr Glu Glu Tyr Leu Cys Met Lys Thr Leu Leu Leu
385                 390                 395                 400

Leu Ser Ser Val Pro Lys Asp Gly Leu Lys Ser Gln Glu Leu Phe Asp
                405                 410                 415

Glu Ile Arg Met Thr Tyr Ile Lys Glu Leu Gly Lys Ala Ile Val Lys
            420                 425                 430

Arg Glu Gly Asn Ser Ser Gln Asn Trp Gln Arg Phe Tyr Gln Leu Thr
        435                 440                 445

Lys Leu Leu Asp Ser Met His Glu Val Val Glu Asn Leu Leu Asn Tyr
    450                 455                 460

Cys Phe Gln Thr Phe Leu Asp Lys Thr Met Ser Ile Glu Phe Pro Glu
465                 470                 475                 480

Met Leu Ala Glu Ile Ile Thr Asn Gln Ile Pro Lys Tyr Ser Asn Gly
                485                 490                 495

Asn Ile Lys Lys Leu Leu Phe His Gln Lys
            500                 505

<210>  42
<211>  453
<212>  PRT
<213>  Artificial

<220>
<223>  SimGR3; an integrated molecule-format probe carrying an LXXLL mot
       if and GLuc.

<400>  42

Met Lys Pro Thr Glu Asn Asn Glu Asp Phe Asn Ile Val Ala Val Ala
1               5                   10                  15

Ser Asn Phe Ala Thr Thr Asp Leu Asp Ala Asp Arg Gly Lys Leu Pro
            20                  25                  30

Gly Lys Lys Leu Pro Leu Glu Val Leu Lys Glu Met Glu Ala Asn Ala
        35                  40                  45

Arg Lys Ala Gly Cys Thr Arg Gly Cys Leu Ile Cys Leu Ser His Ile
    50                  55                  60

Lys Cys Thr Pro Lys Met Lys Lys Phe Ile Pro Gly Arg Cys His Thr
65                  70                  75                  80

Tyr Glu Gly Asp Lys Glu Ser Ala Gln Gly Gly Gly Gly Gly Thr Gln
                85                  90                  95

Leu Thr Pro Thr Leu Val Ser Leu Leu Glu Val Ile Glu Pro Glu Val
            100                 105                 110

Leu Tyr Ala Gly Tyr Asp Ser Ser Val Pro Asp Ser Thr Trp Arg Ile
        115                 120                 125

Met Thr Thr Leu Asn Met Leu Gly Gly Arg Gln Val Ile Ala Ala Val
    130                 135                 140

Lys Trp Ala Lys Ala Ile Pro Gly Phe Arg Asn Leu His Leu Asp Asp
145                 150                 155                 160

Gln Met Thr Leu Leu Gln Tyr Ser Trp Met Phe Leu Met Ala Phe Ala
                165                 170                 175

Leu Gly Trp Arg Ser Tyr Arg Gln Ser Ser Ala Asn Leu Leu Cys Phe
            180                 185                 190

Ala Pro Asp Leu Ile Ile Asn Glu Gln Arg Met Thr Leu Pro Cys Met
        195                 200                 205

Tyr Asp Gln Cys Lys His Met Leu Tyr Val Ser Ser Glu Leu His Arg
    210                 215                 220

Leu Gln Val Ser Tyr Glu Glu Tyr Leu Cys Met Lys Thr Leu Leu Leu
225                 230                 235                 240

Leu Ser Ser Val Pro Lys Asp Gly Leu Lys Ser Gln Glu Leu Phe Asp
                245                 250                 255

Glu Ile Arg Met Thr Tyr Ile Lys Glu Leu Gly Lys Ala Ile Val Lys
            260                 265                 270

Arg Glu Gly Asn Ser Ser Gln Asn Trp Gln Arg Phe Tyr Gln Leu Thr
        275                 280                 285

Lys Leu Leu Asp Ser Met His Glu Val Val Glu Asn Leu Leu Asn Tyr
    290                 295                 300

Cys Phe Gln Thr Phe Leu Asp Lys Thr Met Ser Ile Glu Phe Pro Glu
305                 310                 315                 320

Met Leu Ala Glu Ile Ile Thr Asn Gln Ile Pro Lys Tyr Ser Asn Gly
                325                 330                 335

Asn Ile Lys Lys Leu Leu Phe His Gln Lys Val Asp Gly Gly Asn Gly
            340                 345                 350

Gly Arg Asn Ala Leu Leu Arg Tyr Leu Leu Asp Lys Asp Gly Ser Gly
        355                 360                 365

Gly Gly Gly Ile Gly Glu Ala Ile Val Asp Ile Pro Glu Ile Pro Gly
    370                 375                 380

Phe Lys Asp Leu Glu Pro Met Glu Gln Phe Ile Ala Gln Val Asp Leu
385                 390                 395                 400

Cys Val Asp Cys Thr Thr Gly Cys Leu Lys Gly Leu Ala Asn Val Gln
                405                 410                 415

Cys Ser Asp Leu Leu Lys Lys Trp Leu Pro Gln Arg Cys Ala Thr Phe
            420                 425                 430

Ala Ser Lys Ile Gln Gly Gln Val Asp Lys Ile Lys Gly Ala Gly Gly
        435                 440                 445

Asp Lys Asp Glu Leu
    450

<210>  43
<211>  449
<212>  PRT
<213>  Artificial

<220>
<223>  8990N; an integrated molecule-format probe carrying 8990.

<400>  43

Met Lys Pro Thr Glu Asn Asn Glu Asp Phe Asn Ile Val Ala Val Ala
1               5                   10                  15

Ser Asn Phe Ala Thr Thr Asp Leu Asp Ala Asp Arg Gly Lys Leu Pro
            20                  25                  30

Gly Lys Lys Leu Pro Leu Glu Val Leu Lys Glu Met Glu Ala Asn Ala
        35                  40                  45

Arg Lys Ala Gly Cys Thr Arg Gly Cys Leu Ile Cys Leu Ser His Ile
    50                  55                  60

Lys Cys Thr Pro Lys Met Lys Lys Trp Leu Pro Gly Arg Cys His Thr
65                  70                  75                  80

Tyr Glu Gly Asp Lys Glu Ser Ala Gln Gly Gly Gly Gly Gly Thr Gln
                85                  90                  95

Leu Thr Pro Thr Leu Val Ser Leu Leu Glu Val Ile Glu Pro Glu Val
            100                 105                 110

Leu Tyr Ala Gly Tyr Asp Ser Ser Val Pro Asp Ser Thr Trp Arg Ile
        115                 120                 125

Met Thr Thr Leu Asn Met Leu Gly Gly Arg Gln Val Ile Ala Ala Val
    130                 135                 140

Lys Trp Ala Lys Ala Ile Pro Gly Phe Arg Asn Leu His Leu Asp Asp
145                 150                 155                 160

Gln Met Thr Leu Leu Gln Tyr Ser Trp Met Phe Leu Met Ala Phe Ala
                165                 170                 175

Leu Gly Trp Arg Ser Tyr Arg Gln Ser Ser Ala Asn Leu Leu Cys Phe
            180                 185                 190

Ala Pro Asp Leu Ile Ile Asn Glu Gln Arg Met Thr Leu Pro Cys Met
        195                 200                 205

Tyr Asp Gln Cys Lys His Met Leu Tyr Val Ser Ser Glu Leu His Arg
    210                 215                 220

Leu Gln Val Ser Tyr Glu Glu Tyr Leu Cys Met Lys Thr Leu Leu Leu
225                 230                 235                 240

Leu Ser Ser Val Pro Lys Asp Gly Leu Lys Ser Gln Glu Leu Phe Asp
                245                 250                 255

Glu Ile Arg Met Thr Tyr Ile Lys Glu Leu Gly Lys Ala Ile Val Lys
            260                 265                 270

Arg Glu Gly Asn Ser Ser Gln Asn Trp Gln Arg Phe Tyr Gln Leu Thr
        275                 280                 285

Lys Leu Leu Asp Ser Met His Glu Val Val Glu Asn Leu Leu Asn Tyr
    290                 295                 300

Cys Phe Gln Thr Phe Leu Asp Lys Thr Met Ser Ile Glu Phe Pro Glu
305                 310                 315                 320

Met Leu Ala Glu Ile Ile Thr Asn Gln Ile Pro Lys Tyr Ser Asn Gly
                325                 330                 335

Asn Ile Lys Lys Leu Leu Phe His Gln Lys Val Asp Gly Gly Asn Gly
            340                 345                 350

Gly Arg Asn Ala Leu Leu Arg Tyr Leu Leu Asp Lys Asp Gly Ser Gly
        355                 360                 365

Gly Gly Gly Ile Gly Glu Ala Ile Val Asp Ile Pro Glu Ile Pro Gly
    370                 375                 380

Phe Lys Asp Leu Glu Pro Met Glu Gln Phe Ile Ala Gln Val Asp Leu
385                 390                 395                 400

Cys Val Asp Cys Thr Thr Gly Cys Leu Lys Gly Leu Ala Asn Val Gln
                405                 410                 415

Cys Ser Asp Leu Leu Lys Lys Trp Leu Pro Gln Arg Cys Ala Thr Phe
            420                 425                 430

Ala Ser Lys Ile Gln Gly Gln Val Asp Lys Ile Lys Gly Ala Gly Gly
        435                 440                 445

Asp

<210>  44
<211>  449
<212>  PRT
<213>  Artificial

<220>
<223>  Mon3N; an integrated molecule-format probe carrying Mon3.

<400>  44

Met Lys Pro Thr Glu Asn Asn Glu Asp Phe Asn Ile Val Ala Val Ala
1               5                   10                  15

Ser Asn Phe Ala Thr Thr Asp Leu Asp Ala Asp Arg Gly Lys Leu Pro
            20                  25                  30

Gly Lys Lys Leu Pro Leu Glu Val Leu Lys Glu Met Glu Ala Asn Ala
        35                  40                  45

Arg Lys Ala Gly Cys Thr Arg Gly Cys Leu Ile Cys Leu Ser His Ile
    50                  55                  60

Lys Cys Thr Pro Lys Met Lys Lys Trp Leu Pro Gly Arg Cys Glu Thr
65                  70                  75                  80

Trp Glu Gly Asp Lys Glu Ser Ala Gln Gly Gly Gly Gly Gly Thr Gln
                85                  90                  95

Leu Thr Pro Thr Leu Val Ser Leu Leu Glu Val Ile Glu Pro Glu Val
            100                 105                 110

Leu Tyr Ala Gly Tyr Asp Ser Ser Val Pro Asp Ser Thr Trp Arg Ile
        115                 120                 125

Met Thr Thr Leu Asn Met Leu Gly Gly Arg Gln Val Ile Ala Ala Val
    130                 135                 140

Lys Trp Ala Lys Ala Ile Pro Gly Phe Arg Asn Leu His Leu Asp Asp
145                 150                 155                 160

Gln Met Thr Leu Leu Gln Tyr Ser Trp Met Phe Leu Met Ala Phe Ala
                165                 170                 175

Leu Gly Trp Arg Ser Tyr Arg Gln Ser Ser Ala Asn Leu Leu Cys Phe
            180                 185                 190

Ala Pro Asp Leu Ile Ile Asn Glu Gln Arg Met Thr Leu Pro Cys Met
        195                 200                 205

Tyr Asp Gln Cys Lys His Met Leu Tyr Val Ser Ser Glu Leu His Arg
    210                 215                 220

Leu Gln Val Ser Tyr Glu Glu Tyr Leu Cys Met Lys Thr Leu Leu Leu
225                 230                 235                 240

Leu Ser Ser Val Pro Lys Asp Gly Leu Lys Ser Gln Glu Leu Phe Asp
                245                 250                 255

Glu Ile Arg Met Thr Tyr Ile Lys Glu Leu Gly Lys Ala Ile Val Lys
            260                 265                 270

Arg Glu Gly Asn Ser Ser Gln Asn Trp Gln Arg Phe Tyr Gln Leu Thr
        275                 280                 285

Lys Leu Leu Asp Ser Met His Glu Val Val Glu Asn Leu Leu Asn Tyr
    290                 295                 300

Cys Phe Gln Thr Phe Leu Asp Lys Thr Met Ser Ile Glu Phe Pro Glu
305                 310                 315                 320

Met Leu Ala Glu Ile Ile Thr Asn Gln Ile Pro Lys Tyr Ser Asn Gly
                325                 330                 335

Asn Ile Lys Lys Leu Leu Phe His Gln Lys Val Asp Gly Gly Asn Gly
            340                 345                 350

Gly Arg Asn Ala Leu Leu Arg Tyr Leu Leu Asp Lys Asp Gly Ser Gly
        355                 360                 365

Gly Gly Gly Ile Gly Glu Ala Ile Val Asp Ile Pro Glu Ile Pro Gly
    370                 375                 380

Phe Lys Asp Leu Glu Pro Met Glu Gln Phe Ile Ala Gln Val Asp Leu
385                 390                 395                 400

Cys Val Asp Cys Thr Thr Gly Cys Leu Lys Gly Leu Ala Asn Val Gln
                405                 410                 415

Cys Ser Asp Leu Leu Lys Lys Trp Leu Pro Gln Arg Cys Ala Thr Phe
            420                 425                 430

Ala Ser Lys Ile Gln Gly Gln Val Asp Lys Ile Lys Gly Ala Gly Gly
        435                 440                 445

Asp

<210>  45
<211>  261
<212>  PRT

<213>  Artificial

<220>
<223>  GAL4-linked Src SH2 cDNA for Two hybrid assays

<400>  45

Met Lys Leu Leu Ser Ser Ile Glu Gln Ala Cys Asp Ile Cys Arg Leu
1               5                   10                  15

Lys Lys Leu Lys Cys Ser Lys Glu Lys Pro Lys Cys Ala Lys Cys Leu
            20                  25                  30

Lys Asn Asn Trp Glu Cys Arg Tyr Ser Pro Lys Thr Lys Arg Ser Pro
        35                  40                  45

Leu Thr Arg Ala His Leu Thr Glu Val Glu Ser Arg Leu Glu Arg Leu
    50                  55                  60

Glu Gln Leu Phe Leu Leu Ile Phe Pro Arg Glu Asp Leu Asp Met Ile
65                  70                  75                  80

Leu Lys Met Asp Ser Leu Gln Asp Ile Lys Ala Leu Leu Thr Gly Leu
                85                  90                  95

Phe Val Gln Asp Asn Val Asn Lys Asp Ala Val Thr Asp Arg Leu Ala
            100                 105                 110

Ser Val Glu Thr Asp Met Pro Leu Thr Leu Arg Gln His Arg Ile Ser
        115                 120                 125

Ala Thr Ser Ser Ser Glu Glu Ser Ser Asn Lys Gly Gln Arg Gln Leu
    130                 135                 140

Thr Val Ser Pro Glu Phe Pro Gly Ile Arg Arg Leu Val Ala Pro Ser
145                 150                 155                 160

Asp Ser Ile Gln Ala Glu Glu Trp Tyr Phe Gly Lys Ile Thr Arg Arg
                165                 170                 175

Glu Ser Glu Arg Leu Leu Leu Asn Ala Glu Asn Pro Arg Gly Thr Phe
            180                 185                 190

Leu Val Arg Glu Ser Glu Thr Thr Lys Gly Ala Tyr Cys Leu Ser Val
        195                 200                 205

Ser Asp Phe Asp Asn Ala Lys Gly Leu Asn Val Lys His Tyr Lys Ile
    210                 215                 220

Arg Lys Leu Asp Ser Gly Gly Phe Tyr Ile Thr Ser Arg Thr Gln Phe
225                 230                 235                 240

Asn Ser Leu Gln Gln Leu Val Ala Tyr Tyr Ser Lys His Ala Asp Gly
                245                 250                 255

Leu Cys His Arg Leu
            260

<210>  46
<211>  299
<212>  PRT
<213>  Artificial

<220>
<223>  VP16-linked ER LBD cDNA for Two hybrid assays

<400>  46

Met Thr Ala Pro Pro Thr Asp Val Ser Leu Gly Asp Glu Leu His Leu
1               5                   10                  15

Asp Gly Glu Asp Val Ala Met Ala His Ala Asp Ala Leu Asp Asp Phe
            20                  25                  30

Asp Leu Asp Met Leu Gly Asp Gly Asp Ser Pro Gly Pro Gly Ser Pro
        35                  40                  45

Gly Ile Arg Arg Leu Ser Leu Ala Leu Ser Leu Thr Ala Asp Gln Met
    50                  55                  60

Val Ser Ala Leu Leu Asp Ala Glu Pro Pro Ile Leu Tyr Ser Glu Tyr
65                  70                  75                  80

Asp Pro Thr Arg Pro Phe Ser Glu Ala Ser Met Met Gly Leu Leu Thr
                85                  90                  95

Asn Leu Ala Asp Arg Glu Leu Val His Met Ile Asn Trp Ala Lys Arg
            100                 105                 110

Val Pro Gly Phe Val Asp Leu Thr Leu His Asp Gln Val His Leu Leu
        115                 120                 125

Glu Cys Ala Trp Leu Glu Ile Leu Met Ile Gly Leu Val Trp Arg Ser
    130                 135                 140

Met Glu His Pro Gly Lys Leu Leu Phe Ala Pro Asn Leu Leu Leu Asp
145                 150                 155                 160

Arg Asn Gln Gly Lys Cys Val Glu Gly Met Val Glu Ile Phe Asp Met
                165                 170                 175

Leu Leu Ala Thr Ser Ser Arg Phe Arg Met Met Asn Leu Gln Gly Glu

180                 185                 190

Glu Phe Val Cys Leu Lys Ser Ile Ile Leu Leu Asn Ser Gly Val Tyr
        195                 200                 205

Thr Phe Leu Ser Ser Thr Leu Lys Ser Leu Glu Glu Lys Asp His Ile
    210                 215                 220

His Arg Val Leu Asp Lys Ile Thr Asp Thr Leu Ile His Leu Met Ala
225                 230                 235                 240

Lys Ala Gly Leu Thr Leu Gln Gln Gln His Gln Arg Leu Ala Gln Leu
                245                 250                 255

Leu Leu Ile Leu Ser His Ile Arg His Met Ser Asn Lys Gly Met Glu
            260                 265                 270

His Leu Tyr Ser Met Lys Cys Lys Asn Val Val Pro Leu Tyr Asp Leu
        275                 280                 285

Leu Leu Glu Met Leu Asp Ala His Arg Leu His
    290                 295

## Description

### Technical Field

The present invention relates to the establishment of a method for improving the optical properties and bioluminescence stability of marine luciferase by way of genetic modification, and also relates to a superluminescent and stable artificial luciferase synthesized by the method.

### Background Art

Many molecular phenomena occurring in cells or cell-free systems in nature (for example, conformation changes and phosphorylation of proteins, interaction between proteins, and production of secondary signal transduction substances) are highly important signatures to understand nature and to explore the molecular mechanisms dominating life phenomena. Presently, various methods, including (i) FRET (Non-patent Literature 1 and 2), (ii) BRET (Non-patent Literature 3), and (iii) protein-fragment complementation assay (Non-patent Literature 4 and 5), have been developed to measure such molecular phenomena.
In recent years, the present inventors have conducted research and development regarding bioluminescence imaging using unique molecular design technology. More specifically, the inventors measured translocation of transcription factors into the nucleus and nongenomic protein-protein interactions in the cytosol using protein splicing (Non-patent Literature 6 and 7). Further, the inventors developed a single molecule-format bioluminescent probe in which all of the necessary elements for signal recognition and bioluminescence emission are integrated (Non-patent Literature 8 and 9). Thereafter, the probes were multicolorized, thereby enabling it to simultaneously image multiple signal-transduction processes (Non-patent Literature 10). Moreover, the inventors further developed a circular permutation technique (Non-patent Literature 11) and a molecular design technology using a small luminescent enzyme (Non-patent Literature 12), as methods for improving the ligand sensitivity of the bioluminescent probe itself. These technologies have been used as means for efficiently measuring cellular and noncellular molecular phenomena.
Fluorescence imaging is more widespread than luminescence imaging as a method for exploring intra- or extracellular molecular phenomena. However, fluorescent proteins company autofluorescence, and therefore generate a high background, requiring an external light source. Therefore, fluorescence imaging requires a large instrument such as a fluorescence microscope, and a sophisticated filtering system. Fluorescence imaging also has a drawback in that maturation of fluorescence chromophore takes at least several hours to several days. Further, when using a fluorescence microscope, the observable cell numbers for each measurement are limited, which has hampered quantitative analysis (Non-patent Literature 6).
On the other hand, the bioluminescence imaging using a luciferase has, despite the many advantages, a critical problem of poor bioluminescence intensities of luciferases, which results in hampering popular use of bioluminescence imaging compared to fluorescence imaging. Since the bioluminescence intensity of luciferases is poor, high sensitivity detectors are required, and bioluminescence imaging is considered to be inappropriate for single-cell imaging or exploration of organelle.
Meanwhile, as for fluorescent protein, fluorescent protein with multicolors has been studied well, and many facts regarding the coloring mechanisms thereof have been discovered. Therefore, based on these findings, many fluorescent proteins with diversified fluorescent characteristics have been developed. On the other hand, there exist only a few luciferase that can exhibit multicolor bioluminescence. Therefore, although the advantages of diversifying the colors of bioluminescence, including (i) simultaneous measurements of multiple signals and (ii) high tissue permeability of the bioluminescence of longer wavelengths into living tissues, have been recited, a systematic study for diversifying the colors of bioluminescence based on the luminescence principles thereof have hardly been conducted.
Accordingly, it has been strongly desired to establish a systematic mutagenesis strategy that can exert superluminescence and stable bioluminescence intensity even in luciferases. Further, there has also been an urgent need for systematic studies for a technology for shifting the luminescence of luciferases to the longer wavelength side.

The present inventors have long focused attention on Gaussia luciferase (Patent Literature 1), which is a luciferase derived from a marine animal, advantageously small in size and higher exhibits higher bioluminescence intensities compared to other luciferases, and the inventors have developed various luminescence imaging methods using the Gaussia luciferase (Japanese Patent Application Nos. JP2008-116098, JP2009-025229 and Jap2007-332253). However, despite its relatively high bioluminescence intensities, the bioluminescence of marine animal-derived luciferases including Gaussia luciferase (GLuc) is unstable has exhibits poor protein folding, which results in insufficient bioluminescence intensities. Although many genetically mutated variants of Gaussia luciferase are known, none of the mutants have sufficient bioluminescence intensity and stability (Patent Literature 2 to 6). Although the anchor peptide (JP2009-025229) developed by the present inventors exerts considerably improved optical stability as a luminescent probe, further enhancements in bioluminescence intensity and stability have been desired.

### [Prior Art Documents]

### [Patent Literature]

[Patent Literature I] International Publication No. 1999-49019 (W01999/049019)
[Patent Literature 2] Specification of US Patent No. 6232107
[Patent Literature 3] Specification of US Patent No. 6436682
[Patent Literature 4] Specification of US Patent No. 6780974
[Patent Literature 5] Specification of US Patent No. 7045599
[Patent Literature 6] Specification of US Patent No. 7238497
[Patent Literature 7] International Publication No. 2006-84869 (W02008/084869)

### [Non-patent Literature]

[Non-patent Literature 1] Awais, M.; Sato, M.; Lee, X. F.; Umezawa, Y.; Angew. Chem. Int. Ed. 2006, 45, 2707-2712.
[Non-patent Literature 2] Awais, M.; Sato, M.; Sasaki, K.; Umezawa, Y.; Anal. Chem. 2004, 76, 2181-2186.
[Non-patent Literature 3] Hoshino, H.; Nakajima, Y.; Ohmiya, Y.; Nature Methods 2007, 4, 637-639.
[Non-patent Literature 4] Paulmurugan, R.; Gambhir, S.S.; Anal. Chem. 2005, 77, 1295-1302.
[Non-patent Literature 5] aulmurugan, R.; Gambhir, S.S.; Proc. Natl. Acad. Sci. U.S.A. 2006, 103, 15883-15888.
[Non-patent Literature 6] Kim, S.B.; Ozawa, T.; Watanabe, S.; Umezawa, Y.; Proc. Natl. Acad. Sci. U. S. A. 2004, 101, 11542-11547.
[Non-patent Literature 7] Kim, S.B.; Ozawa, T.; Umezawa, Y.; Anal. Chem. 2005, 77, 6588-6593.
[Non-patent Literature 8] Kim, S.B.; Awais, M.; Sato, M.; Umezawa, Y.; Tao, H.; Anal. Chem. 2007, 79, 1874-1880.
[Non-patent Literature 9] Kim, S.B.; Otani, Y.; Umezawa, Y.; Tao, H.; (2007) Anal. Chem. , 79, 4820- 4826.
[Non-patent Literature 10] Kim, S.B.; Umezawa, Y.; Kanno, K. A.; Tao, H.; ACS Chemical Biology 2008, 3, 359-372.
[Non-patent Literature 11] Kim, S.B.; Sato, M.; Tao, H.; Bioconjugate Chem. 2008, 19, 2480-2486.
[Non-patent Literature 12] Kim, S.B.; Sato, M.; Tao, H.; Anal. Chem. 2009, 81, 67-74.
[Non-patent Literature 13] Matthews, L.; Berry, A.; Tersigni, M.; D'Acquisto, F.; Ianaro, A.; Ray, D.; Endocrinology 2009, 150, 75-86.
[Non-patent Literature 14] Kim, S.B.; Sato, M.; Tao, H.; Anal. Chem. 2009, 81, 67-74.
[Non-patent Literature 15] Loening A.M.; Fenn T.D.; Gambhir S.S.; J. Mol. Biol. 2007, 374 (4), 1017-1028.
[Non-patent Literature 16] Cubitt A.B.; Woollenweber L.A.; Heim R.; Meth. Cell Biol. 1999, 58, 19-30.
[Non-patent Literature 17] Atsushi Miyawaki; Takeharu Nagai; Hideaki Mizuno; Curr. Opinion Chem. Biol. 2003, 7:557-562.
[Non-patent Literature 18] N.C. Shaner; G.H. Patterson; M.W. Davidson; J. Cell Sci., 2007 120 (24), 4247-4260.
[Non-patent Literature 19] Okita K.; Ichisaka T.; Yamanaka S.; Nature, 2007, 448 (7151) 313-317.
[Non-patent Literature 20] Papworth, C.; Bauer, J.C.; Braman, J; Wright, D.A.; Strategies, 1996, 9(3):3-4.

### Summary of Invention

### Technical Problem

An object of the present invention is to establish a method for enhancement of bioluminescence intensity, shifting the bioluminescence to the longer wavelength side, and improvement of bioluminescence stability of luciferase; and to provide a superluminescent and stable artificial luciferase produced by the method. Another object of the present invention is to establish a bioluminescence imaging method using the luciferase.

### Solution to Problem

In order to attain the above object, the present inventors focused their attention on marine luciferases among various luciferases. On the whole, luciferases have variable structures and share no considerable phylogenetical similarities. Therefore, it is difficult to determine generally acceptable rules among luciferases. However, upon spotlight on marine luciferases, they share common substrates, and have significantly high similarities in sequence. Owing to these advantages, the inventors speculated that, if the conventional knowledge regarding marine luciferases were combined, it may be possible to predict the steric-structure of interaction between enzyme and substrate and estimate the active site of luciferase enzyme. On the basis of this speculation, the inventors attempted to enhance bioluminescence intensity, shift the bioluminescence to the longer wavelength side and improve bioluminescence stability of luciferase by genetically modifying the putative active site and vicinity thereof. More specifically, the inventors utilized the gene sequence of Gaussia-derived luciferase (GLuc) (GENE ACCESSION #: FJ010198) which has the smallest molecular weight among the marine luciferases; codon-optimized and modified to create many variants with genetic mutations at the putative active sites or adjacent amino acids. Each variant was observed for its changes in bioluminescence intensity and stability, or tendency to a shift to the longer wavelength side.
The observation revealed that conservative amino acid replacement of at least one of the amino acid residues at a position corresponding to positions 89, 90, 95, 97, 100, 108, 112, 115, and 118 in GLuc successfully improved activity of the luciferases, i.e., enhanced bioluminescence intensity of luciferases with stabilized bioluminescence, or bioluminescence shifted to the longer wavelength side. More specifically, the improvements in enzymatic properties of luciferase such as enhancement in bioluminescence intensity or the shift to the longer wavelength side, are observed through mutating one or more hydrophobic amino acid residues at positions corresponding to 89, 90, 97, 108, 112, 115, and 118 in GLuc to other hydrophobic amino acid residues. In particular, by individually replacing one or more hydrophobic nonpolar aliphatic amino acid residues at a positions corresponding to positions 90, 108, 112, 115, and 118 in GLuc with other hydrophobic nonpolar aliphatic amino acid residues; or replacing aromatic amino residues at a position corresponding to position 89 and/or 97 in GLuc with other aromatic amino residue(s) demonstrated significant enhancement in activity of luminescence enzyme, as substantiated by an enhancement in bioluminescence intensity and stability, and a shift to the longer wavelength side. Further, it was also confirmed that replacement of hydrophilic amino acid residues at a position corresponding to position 95 and/or 100 in GLuc forming a. hydrogen bond with other hydrophilic amino acid residue caused a shift to the longer wavelength side.
Based on these findings, the present invention was completed.
Further, by introducing mutation into Metridia longa (MLuc) and Metridia pacifica (MpLuc1), which are marine luciferases similar to GLuc, at the positions corresponding to the mutated sites of GLuc, an enhancement in bioluminescence intensity and a shift to a longer wavelength similar to those exhibited by GLuc were observed. This proved that the above mutation sites are common performance-improving sites of marine luciferases.
Moreover, by using the new luciferase synthesized by the present invention as a reporter gene for reporter-gene assay or mammalian two-hybrid assay, or by using the luciferases as a dissected luciferase in bioluminescent probe methods, the strong bioluminescence was also stably maintained in these assays, confirming the effect of reduced assay times and improved S/N ratios. Among these, the usage of the luciferase variants of the present invention in a reporter-gene assay was found to be a revolutionary modification technique of hitherto known assays; therefore, as a separate invention, a patent application of this technology was filed with the Japan Patent Office on the same date as the application date of the present invention.

Specifically, the present invention comprises the following items.

### [Item 1]

A luciferase variant with improved optical property obtained by replacing at least one amino acid residue among the amino acid sequence of a marine luciferases at positions corresponding to 89 to 118 in the amino acid sequence of Gaussia luciferase (GLuc), wherein an amino acid residue at a position corresponding to at least one position selected from positions 89, 90, 95, 97, 100, 108, 112, 115, and 118 in the amino acid sequence of GLuc is replaced by way of conservative amino acid replacement.

### [Item 2]

The luciferase variant according to item 1, wherein the conservative amino acid replacement includes replacement of a hydrophobic amino acid residue at a position corresponding to positions 89, 90, 97, 108, 112, 115, or 118 in the amino acid sequence of GLuc with another hydrophobic amino acid residue, or replacement of a hydrophilic amino acid residue at a position corresponding to positions 95 or 100 in the amino acid sequence of GLuc that forms a hydrogen bond with another hydrophilic amino acid residue.

### [Item 3]

The luciferase variant according to item 2, wherein the replacement of a hydrophobic amino acid residue with another hydrophobic amino acid residue includes replacement of a hydrophobic and nonpolar aliphatic amino acid residue at a position corresponding at position 90, 108, 112, 115, or 118 in the amino acid sequence of GLuc with another hydrophobic and nonpolar aliphatic amino acid residue, or replacement of a hydrophobic aromatic amino acid residue at a position corresponding to position 89 or 97 in the amino acid sequence of GLuc with another hydrophobic aromatic amino acid residue.

### [Item 4]

The luciferases variant according to item 3, wherein the replacement of a hydrophobic and nonpolar aliphatic amino acid residue with another hydrophobic and nonpolar aliphatic amino acid residue is replacement of isoleucine with an amino acid residue selected from leucine, tryptophan, and valine.

### [Item 5]

The luciferases variant according to item 3, wherein the replacement of a hydrophobic aromatic amino residue with another hydrophobic aromatic amino residue is replacement of an amino acid residue at a position corresponding to position 89 or 97 in the amino acid sequence of GLuc with tryptophan (W).

### [Item 6]

The luciferase variant according to item 2, wherein the replacement of a hydrophilic amino acid residue at a position corresponding to position 95 or 100 in the amino acid sequence of GLuc that forms a hydrogen bond with another hydrophilic amino acid residue is replacement of an amino acid residue at a position corresponding to position 95 in the amino acid sequence of GLuc with glutamic acid (E), or replacement of an amino acid residue at a position corresponding to position 100 in the amino acid sequence of GLuc with asparagine (N).

### [Item 7]

A luciferase variant obtained by way of conservative amino acid replacement that includes replacement of an amino acid residue at a position corresponding to position 90 in the amino acid sequence of GLuc among the amino acid sequences of marine luciferase with leucine.

### [Item 8]

The luciferase variant according to item 7, wherein the conservative amino acid replacement further includes replacement of an amino acid residue corresponding to position 89 in the amino acid sequence of GLuc with tryptophan, or replacement of an amino acid residue corresponding to position 115 5 in the amino acid sequence of GLuc with leucine.

### [Item 9]

The luciferase variant according to item 7 or 8, wherein the conservative amino acid replacement further includes replacement of an amino acid residue at a position corresponding to position 95 in the amino acid sequence of GLuc with glutamic acid or glutamine, and replacement of an amino acid residue at a position corresponding to position 97 in the amino acid sequence of GLuc with tryptophan.

### [Item 10]

The luciferase variant according to any one of items 1 to 9, wherein the marine luciferase is s one luciferase selected from Gaussia luciferase (GLuc) and Copepod luciferases (MLuc, MpLucl, and MpLuc2).

### [Item 11]

A DNA encoding the luciferase variant according to any one of items 1 to 10.

### [Item 12]

An expression vector comprising the DNA according to stem 11.

### [Item 13]

A transformed cell in which the DNA according to item 11 is introduced in a manner such that the DNA can be expressed.

### [Item 14]

A method for improving luminescent optical property of marine luciferase, comprising replacing at least one of the amino acid residues among the amino acid sequences of marine luciferase at positions corresponding to positions 89 to 118 in the amino acid sequence of Gaussia luciferase (GLuc), wherein an amino acid residue at a position corresponding to at least one position selected from positions 89, 90, 95, 97, 100, 108, 112, 115, and 118 in the amino acid sequence of GLuc is replaced by way of conservative amino acid replacement.

### [Item 15]

A bioluminescent probe with improved optical property that adopts a working mechanism of resurrecting luminescent function by reconstituting dissected luciferases, the probe using the luciferase variant according to any one of items 1 to 10 as the luciferase.

### [Item 16]

A DNA encoding a bioluminescent probe according to item 15.

### Advantageous Effects of Invention

The present invention successfully improved the activity of luminescence enzyme, i.e., enhanced bioluminescence intensity in a longer wavelength region due to enhanced bioluminescence intensity of luciferase or a shift to the longer wavelength side, and also attained an enhanced stability, only by replacing at least one amino acid residue in the luciferase active region and in the vicinity thereof conserved in a wide range of general marine luciferases including GLuc, with a hydrophobic amino acid residue, an aromatic amino acid residue and/or an amino acid residue that forms a hydrogen bond.
By being used as an element of a luciferase for hitherto well-known reporter-gene assay, yeast two-hybrid assay, mammalian two-hybrid assay, protein splicing assay, protein complementation assay, circular permutation assay, bioluminescence resonance energy transfer (BRET) assay (Non-patent Literature 3 to 12) or the like, the superluminescent marine luciferase of the present invention can create a luminescent probe with high bioluminescence intensity and stability, thereby significantly improving measurement efficiency in these assays.
It should be noted that the disclosures of the prior art documents and specifications of patent applications referred to in the present invention are included in the disclosure of the specification of the present invention.

### [Brief Description of Drawings]

[Fig. 1A] A graph showing hydrophobicity distribution in each amino acid sequence of various marine luciferases.
[Fig. 1B] A comparative chart of amino acid sequences of various marine luciferases. The dotted box denotes a putative enzyme active site.
[Fig. 2A] A chart showing the entire amino acid sequence of Gaussia luciferase ((GLuc). The sequence of GLuc was compared with the sequence of luciferase derived from Metridia longa (MLuc). The arrow indicates the enzyme dissection sites in the present study.
[Fig. 2B] A chart showing a known crystallographic structure showing the active site of Renilla luciferase. The chemical substance in the middle shows a molecular structure of the specific substrate (caelenterazine). The respective positions of amino acids shows that the enzyme active site resides between the group of hydrophobic amino acids (single lined circle) and the group of hydrophilic amino acids (double lined circle).
[Fig. 3A] A graph showing recovery level of bioluminescence intensity at each dissection site of GLuc.
[Fig. 3B] A chart showing distribution of hydrophobic amino acids of GLuc. An hydrophilic region covers the vicinity of amino acid numbers 90 to 100.
[Fig. 3C] A drawing for comparing chromophores of fluorescent protein and luminescence protein. Section A shows the crystal structure of fluorescent protein and the chemical structure of the chromophore. Section B shows, from left to right, the crystal structure of firefly luciferases, the chemical structure of coelenterazine, and the crystal structure of Renilla luciferases.
[Fig. 4A] A map showing mutation sites in the amino acid sequence of GLuc.
[Fig. 4B-1] A graph (1) showing a comparison among different GLuc variants with respect to bioluminescence intensities. The graph compares peak values of luminescence spectrum measured by a spectrophotometer, showing relative bioluminescence intensities upon comparison with the bioluminescence intensity of native GLuc (the leftmost value). Here, the cell dissolution time was set to 5 minutes, the time of which is not enough to dissolve organelles.

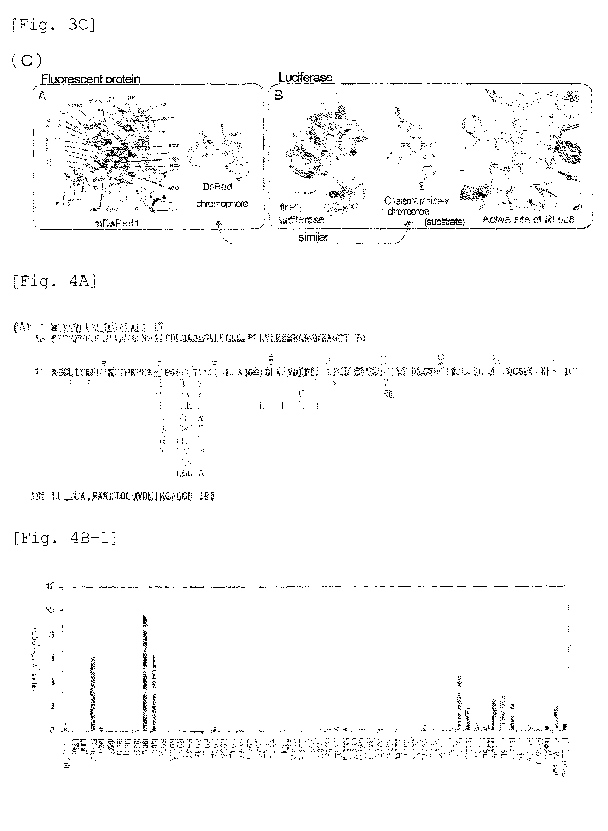

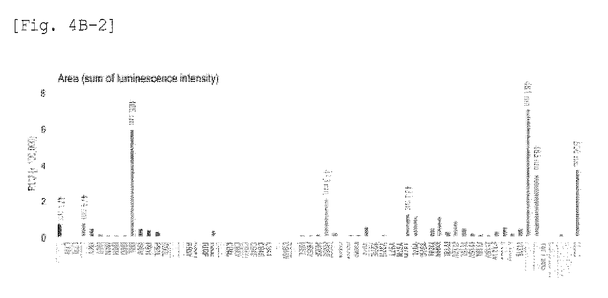

[Fig. 4B-2] A graph (2) showing a comparison among different GLuc variants in terms of bioluminescence intensity. The graph compares areas of luminescence spectrum measured by a luminescence plate reader, showing relative bioluminescence intensities based on the original bioluminescence intensity (the leftmost value). Here, the cell dissolution time was set to 20 minutes, thereby dissolving organelle to obtain uniform lysis solutions. The graph shows maximum values of luminescence wavelengths for the variants with high bioluminescence intensities.

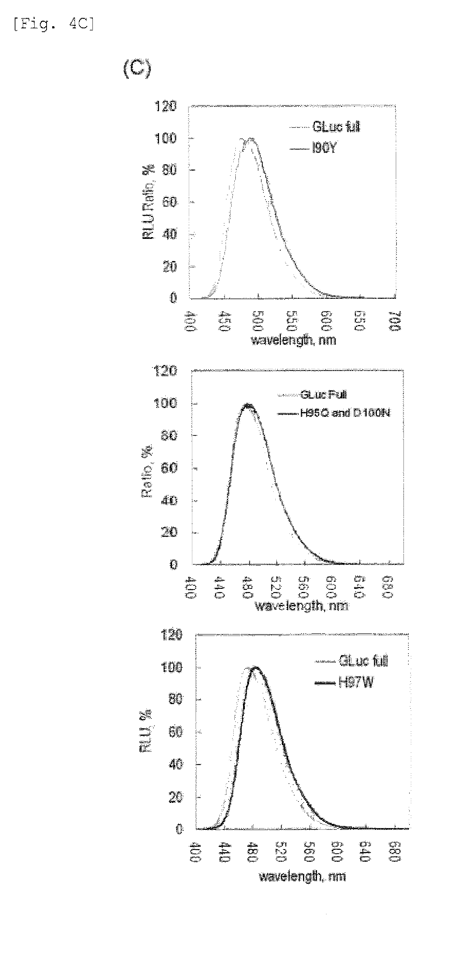

[Fig. 4C] A graph showing a redshift of bioluminescence spectrum of GLuc variants. The upper graph shows mutation of 190 into Tyr(Y), the middle graph shows mutation of H95 and D100 into Q and N, respectively, and the lower graph shows mutation of H97 into W.

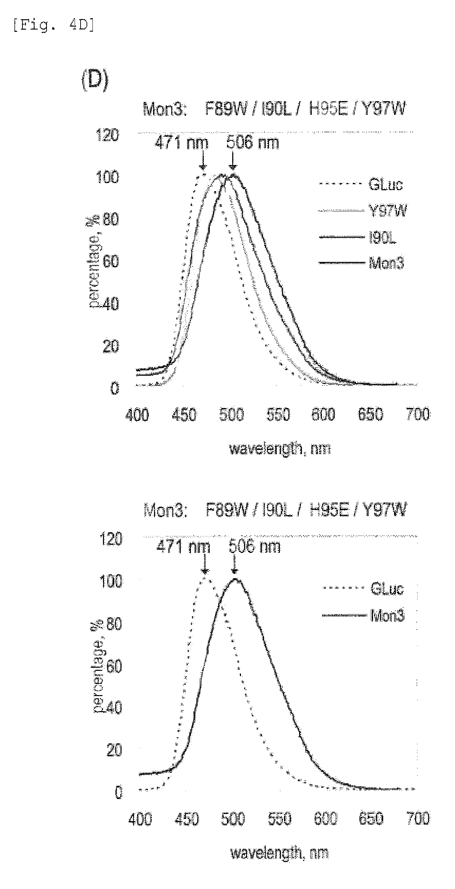

[Fig. 4D] A graph showing redshifts of bioluminescence spectra of GLuc variants. The graph shows each luminescence spectrum of Y97W, I90L, and F89W/I90L/H95E/Y97W (Mon3), compared with a conventional GLuc.

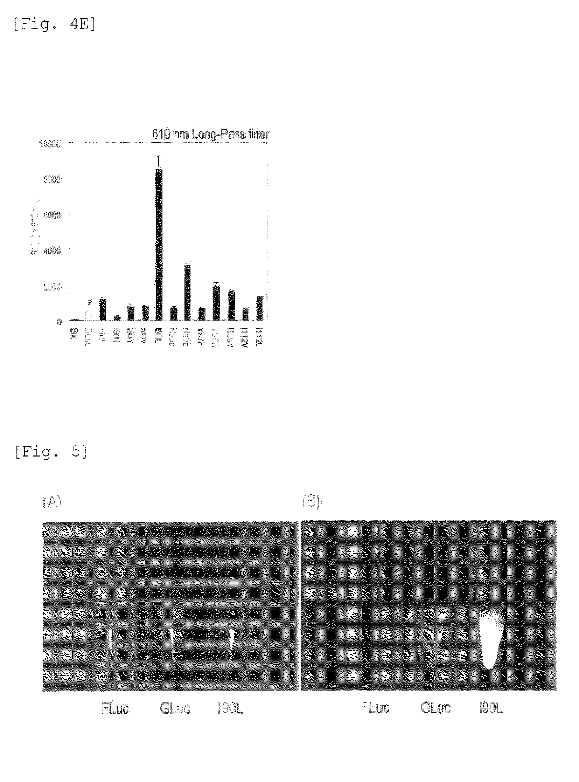

[Fig. 4E] graph showing relative long wavelength bioluminescence intensities measured by a 610 nm long-pass filter. The graph shows respective wavelengths of luminescence variants compared with a known GLuc (white bar). The graph reveals that the variants show strong bioluminescences at long wavelengths.
[Fig. 5] Photos comparing bioluminescence intensities of GLuc variants with significantly strong bioluminescences.
   (A) A digital photo showing the positions of luminescent tubes. The photo shows lysis solutions expressing, from left to right, firefly luciferase (FLuc), known Gaussia luciferase (GLuc), and I90L Variant of GLuc.
   (B) A photo comparing luminescence levels of respective tubes. The photo reveals that the variant I90L of GLuc on the far right has the strongest bioluminescence.

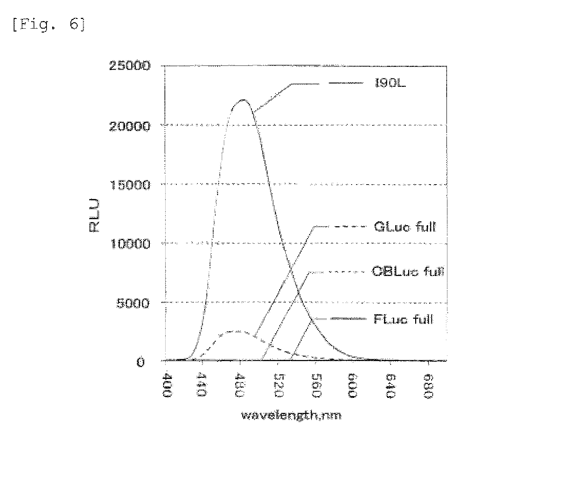

[Fig. 6] A graph comparing bioluminescence spectrums of typical luciferases. The graph reveals that the bioluminescence intensity of the GLuc variant (solid line) is far stronger than those of known GLuc (dotted line), FLuc, and click beetle luciferase (CBLuc).
[Fig. 7AB] Graphs showing changes in bioluminescence intensity with time.
   (A) The graph shows a result of a short-time (5 seconds) observation of changes in bioluminescence with time after introducing a substrate (dissolved in a PBS buffer) into each variant. The graph shows that I90L, H95E, and Y97W exhibit relatively stable bioluminescences.
   (B) The graph shows a result of a relatively long-time (10 minutes) observation of changes in bioluminescence with time after introducing the substrate (dissolved in a PBS buffer) into each variant. The graph reveals that the bioluminescence intensity (white circle) of the original GLuc significantly decreased, whereas the bioluminescence intensities of I90L (black square) and I90V (gray triangle) are relatively stable.
[Fig. 7CD] Graphs showing changes in bioluminescence intensity with time.
   (C) The graph shows a result of a short-time (5 seconds) observation of changes in bioluminescence with time after introducing the substrate (dissolved in a modified Matthew's buffer into each variant.
   (D) The graph shows a result of observation of changes in bioluminescence with time that was conducted per second for each stage after introducing the substrate (dissolved in a modified Matthew's buffer) into each variant stepwise. The graph reveals that all variants are very stable, showing strong bioluminescences.

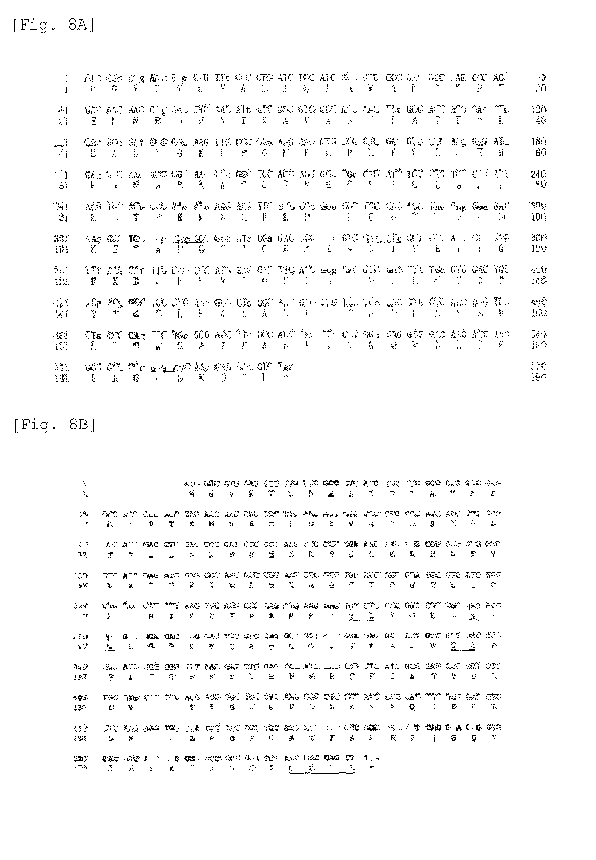

[Fig. 8A] A drawing showing a base sequence modification example (1) of a GLuc variant suitable for expression in mammals including human. Fig. 8(1) shows a base sequence of a variant I90L of GLuc consisting of codons suitable for mammals including human, to which a restriction enzyme was introduced. The underline shows the restriction enzyme site, and the italicized letters indicate the mutation sites.
[Fig. 8B] A drawing showing a base sequence modification example (2) of a GLuc variant suitable for expression in mammals including human. Fig. 8(2) shows a base sequence of GLuc enzyme variant (Mon3 with four mutations including I90L) consisting of codons suitable for mammals including human, to which an EcoRV restriction enzyme was introduced. The underline shows the restriction enzyme site, and the italicized letters indicate the mutation sites.
[Fig. 9AB] Tables showing substrate selectivities of the GLuc variants of the present invention.
   (A) The table shows comparison between the respective variants in terms of substrate selectivity using their bioluminescence intensities as index. The table shows levels of relative bioluminescence intensities based on the bioluminescence intensity of the standard substrate (coelenterazine; CTZ) shown as "++++."
   (B) The table shows the maximum luminescence wavelength (λmax) of luminescence spectrum by each synthetic coelenterazine. The table reveals that each variant has a different luminescence color depending on the type of synthetic coelenterazine.

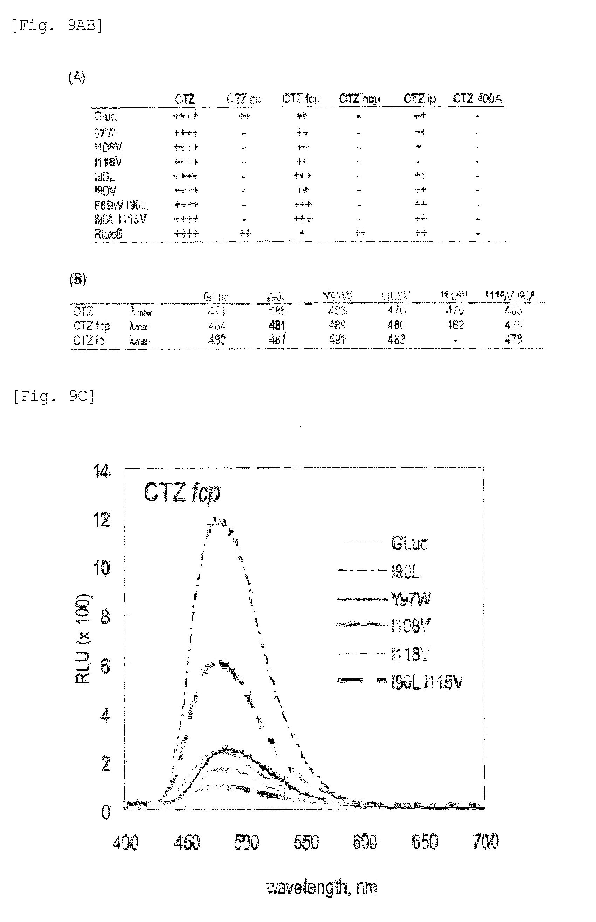

[Fig. 9C] Graph showing substrate selectivity spectrums of the GLuc variants of the present invention. The graph shows, as an example, bioluminescence spectrums of the variants in the presence of coelenterazine fcp.

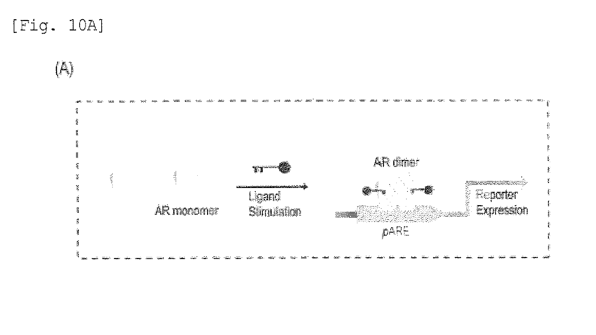

[Fig. 10A] A drawing showing reporter-gene assay based on the GLuc variants of the present invention.
   (A) The drawing shows the principle of the assay. The androgen receptor is activated by ligand stimulation (androgen), duplicated, and then bound to an androgen responsive element (ARE), thereby expressing the reporter gene (GLuc variant).
[Fig. 10BC] The graphs showing a reporter-gene assay based on the GLuc variants of the present invention.
   (B) The graph shows bioluminescence intensities in the presence and absence of androgen. The graph reveals that the GLuc variant (I90L) shows an excellent signal-to-noise ratio (S/N ratio) compared with a known GLuc.
   (C) The graph shows changes in bioluminescence with time of the expressed reporter protein. The graph reveals that both the intensity and stability of the bioluminescence were superior when I90L was incorporated.
[Fig. 10DE] Graphs showing a reporter-gene assay based on the GLuc variants of the present invention.
   (D) The graph shows a comparison in terms of bioluminescence intensity in the reporter-gene assay between the case of using a known Gaussia luciferase (GLuc) and the case of using GLuc variant (I90L) of the present invention. The graph reveals that the area of the bioluminescence intensity was greater in case of using I90L.
   (E) The graph shows comparison of luminescence areas obtained in (D).
[Fig. 11AB] A graph showing a mammalian two-hybrid assay based on the GLuc variants of the present invention.
   (A) The drawing shows the principle of two-hybrid assay. Src SH2 domain and ER LBD were used as the model proteins. The SH2 domain was ligated to Ga14, and the ER LBD was ligated to VP16. They were bound to each other by stimulation of an estrogen. In response to this, the GAL responsive element of pG5 induced expression of the reporter protein (GLuc variant).
   (B) The graph shows changes in bioluminescence intensity depending on the estrogen stimulation time. A sufficient bioluminescence was observed by 6-hour estrogen stimulation when using GLuc variant of the present invention.
[Fig. 11CD] A graph showing mammalian two-hybrid assay based on the GLuc variants of the present invention.
   (C) The graph shows changes in bioluminescence intensity with time when using the original GLuc (pG5-GLuc).
   (D) The graph shows changes in bioluminescence intensity with time when using the GLuc variant of the present invention. The graph reveals that the absolute luminescence of the Gluc variant was higher than GLuc, and that the bioluminescence thereof was efficiently enhanced even by a short-time estrogene stimulation.

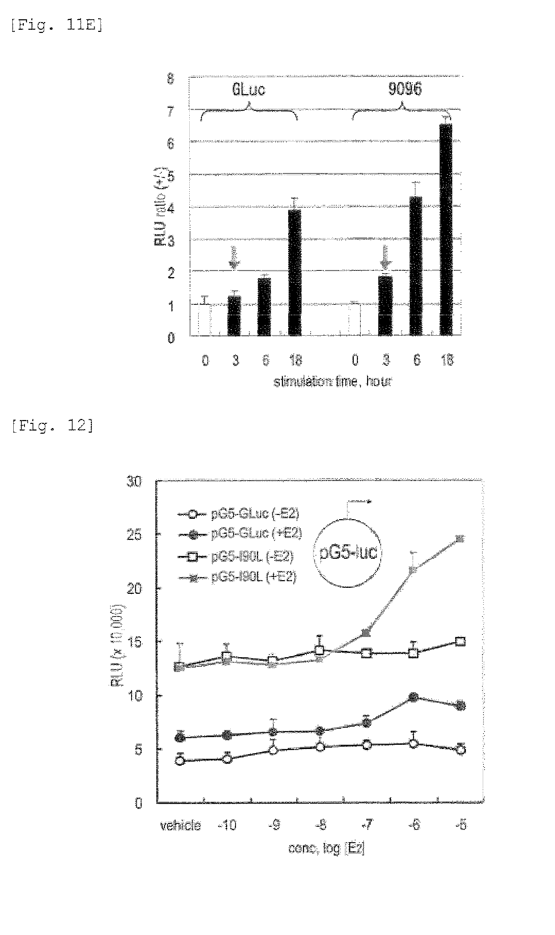

[Fig. 11E] A graph showing a mammalian two-hybrid assay based on the GLuc variants of the present invention.
   (E) The graph shows bioluminescence intensities (measured by a luminometer) after (after 0 hour, 3 hours, 6 hours, and 18 hours) estrogene stimulation when incorporating Glue and GLuc variant (9096) in a pG5 vector.
[Fig. 12] A graph showing the results of a mammalian two-hybrid assay using the GLuc variant of the present invention. The graph shows that the bioluminescence intensity of the GLuc variant of the present invention was significantly enhanced depending on the concentration of estrogene.
[Fig. 13AB] A drawing showing a single-molecule-format bioluminescent probe using the GLuc variant of the present invention.
   (A) The GLuc variant was dissected into two fragments, and a stress hormone receptor (glucocorticoid receptor) and a LXXLL motif were inserted between the fragments. A GLuc-based probe ("SimGR3") was used as a control. In the figure, "8990N" denotes a GLuc variant having mutations at F89W/I90L, and "Mon3N" denotes a GLuc variant having mutations at F89W/I90L/H95E/Y97W.
   (B) The drawing shows a molecular structure diagram of single-molecule-format bioluminescent probes containing GLuc variants of the present invention.
[Fig. 13CD] Graphs showing data regarding single-molecule-format bioluminescent probes containing GLuc variants of the present invention.
   (C) The graph shows a response to stress hormone for each single-molecule-format bioluminescent probe containing the GLuc variant of the present invention. "8990N" exhibited a relatively strong bioluminescence in response to stress hormone.
   (D) The graph shows changes in bioluminescence intensity with time of "8990N." "8990N" exhibited strong bioluminescence in the presence of stress hormone.

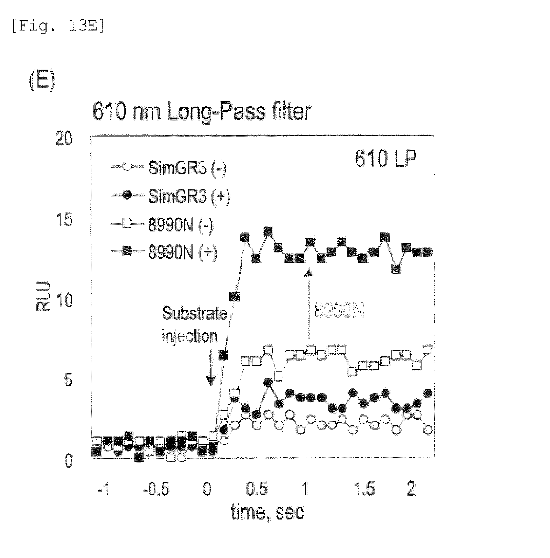

[Fig. 13E] A graph showing data regarding single-molecule-format luciferase probes containing GLuc variants of the present invention.
   (E) The graph shows measurement of relative stress hormone sensitivities of SimGR3 and 8990N measured by a luminescence plate reader equipped with a 610-nm long-pass filter. The bioluminescence of "8990N" was greater, showing a higher sensitivity to stress hormone stimulation.
[Fig. 14AB] Graphs showing luminescence characteristics of MpLuc1 (Metridia pacifica-derived luciferases) variant.
   (A) The graph shows luminescence spectrums of MpLuc1 itself, a I114L variant of MpLuc1 (I114L), and a Y113W/I114L/H119E/Y121W variant of MpLuc1 (MpLuc4).
   (B) The graph shows measurement of bioluminescence intensity of MpLuc1 variant with respect to a long wavelength (610 nm or more) using a 610-nm long-pass filter.

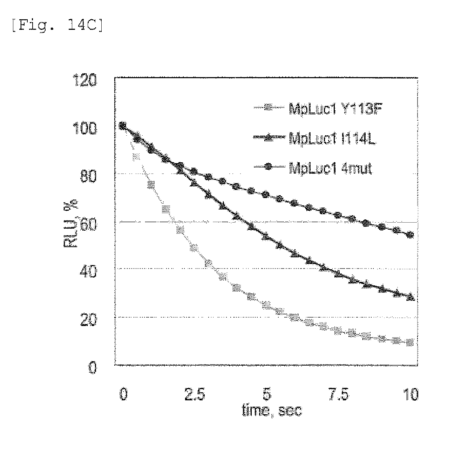

[Fig. 14C] The graph shows measurements of luminescence stability of the MpLuc1 variants. The graph reveals that both I114L and MpLuc4 variants showed more stable bioluminescences compared with the known MpLuc1.
[Fig. 15AB] Graphs showing luminescence characteristics of MLuc variants (Metridia longa-derived luciferases).
   (A) The graph shows luminescence spectrums of MLuc itself, a I123L variant of MLuc (I123L), and a Y122W/I123L/H128E/Y130W variant of MLuc (MLuc4).
   (B) The graph shows measurement of luminescence stabilities of MLuc variants. The graph reveals that the Mluc4 variant showed more stable bioluminescence compared with the known MLuc itself.
[Fig. 15CD] Graphs showing luminescence characteristics of MLuc variant.
   (C) The graph shows measurement of bioluminescence intensities of MLuc variants with respect to a long wavelength (610 nm or more) using a 610-nm long-pass filter.
   (D) The graph shows changes in bioluminescence intensity with time around substrate introduction.

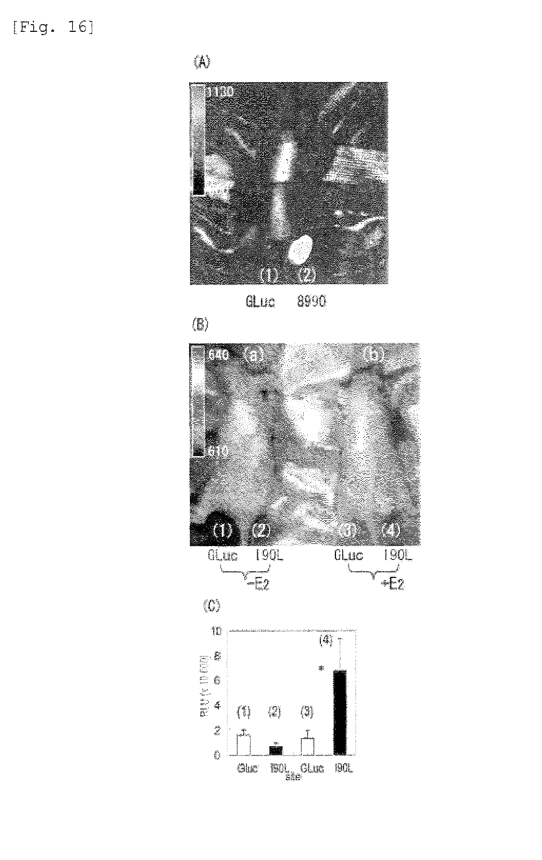

[Fig. 16] (A) A photograph for observation of the animal tissue permeability of bioluminescence of a GLuc variant (8990N) of the present invention having mutations at F89W/190L. A conventional GLuc and 8990N were expressed at the subcutaneous layers of the left back and the right back, respectively, of a mouse. The observation revealed that the bioluminescence from 8990N (right) was stronger than that from GLuc (left).
   (B) A photograph exhibiting bioluminescence intensities from a two-hybrid assay system in the tissues of living animals. Transformed cells, that was obtained by cotransfecting pG5-GLuc or pG5-90L into a COS-7cell, as well as pACT-SH2 or pBIND-ER LBD, was implanted into the subcutaneous layers of the left back or the right back portion of a mouse. The measurement was performed in the presence and absence of estrogene. A stronger bioluminescence was observed in the tissue where pG5-90L was introduced (right: expression of I90L variant of GLuc).
   (C) A graph comparing relative bioluminescence intensities at the respective transplantation portions of the mouse in the above experiment (B). The values correspond to the sites (1), (2), (3), and (4), respectively, from left to right.

### Description of Embodiments

### 1. Marine Luciferase

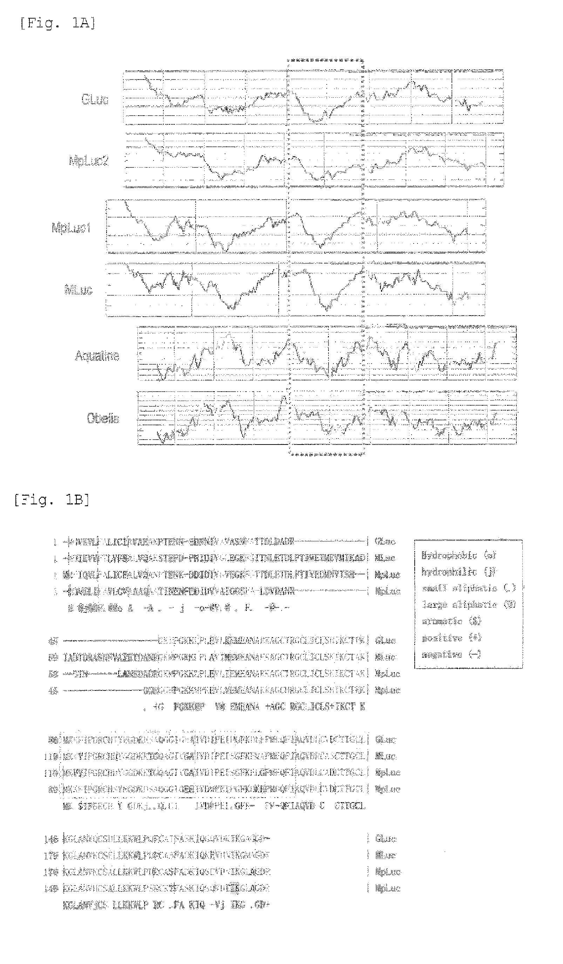

In the present invention, "marine luciferase" designates a luciferase produced by a "luminescent marine animal", which can, in a broad sense, include Obelin, a kind of luminescent plankton, aqualine Pleuromanma, Oplophorus and the like, as well as Gaussia, Renilla reniformis, Cypridina, Metridia etc. Gaussia luciferase (GLuc) and Metridia luciferase (such as MLuc derived from *Metridia longa,* or MpLuc1 and MpLuc2 which are luciferase groups derived from Metridia pacifica) are very similar in terms of distribution of hydrophilic and hydrophobic amino acids in the entire enzyme; moreover, they have high similarity in amino acid sequence in the putative enzymatic activie cite (Fig. 1). Therefore, the luciferase groups are simply called "marine luciferase", or specifically called "Gaussia-like luciferase". The luciferase group also includes luciferases variants with amino acid sequences and/or encoded by the base sequences having a sequence homology of 80% or more, preferably 90% or more, more preferably 95% or more with the amino acid sequence of GLuc (Gene Bank Assession Number: AY015993), MLuc (Gene Bank Assession Number: AY364164), MpLuc1 (Gene Bank Assession Number: AB195233), or MpLuc2 (Gene Bank Assession Number: AB195234). These Gaussia-like luciferases can be easily obtained by a homology search for the base sequence of the aforementioned GLuc, MLuc, MpLuc1, or MpLuc2 in the PSI-BLAST database or a similar database. It is also possible to establish those luciferases from natural Gaussia-like marine animal genes using a probe or primer based on those base sequences.
MLuc, MpLuc1, and MLuc2 as Gaussia-like luciferases are slightly different from GLuc, as they have a greater molecular weight than GLuc; however, they have very similar sequences, as explained above. Further, in terms of enzymatic characteristics such as substrate or luminescence activity, they are almost identical to GLuc. Therefore, the following description mainly discusses a typical Gaussia luciferase (GLuc). Any finding regarding GLuc may also be applied to other Gaussia luciferases.
Hereinafter, the mutation site is indicated based on the amino acid sequence of GLuc, unless otherwise specified. For example, "amino acid 90 of GLuc" corresponds to the amino acid 123 of MLuc. It also corresponds to the amino acid 114 of MpLuc1 and 93 of MpLuc2.
Gaussia luciferase (GLuc) has the following characteristics.
(i) GLuc is the smallest luciferase ever discovered. Therefore, the use of GLuc for molecular imaging is much less burdensome for the host cell or host protein.
(ii) GLuc has significant resistance to pH, surfactant, and chemical denaturant.
(iii) GLuc has the highest bioluminescence intensity among luciferases.
(iv) GLuc has a quick turnover cycle of enzyme, seven times or more than that of known firefly luciferase.
On the other hand, the bioluminescence of GLuc considerably depends on the reaction conditions, and significant decreases in intensity can easily occur, thereby degrading its stability. Therefore, GLuc was considered inappropriate as a reporter (index) for luminescence analysis that strictly requires stability.

### 2. Modification of Marine Luciferase of the Present Invention to Improve Optical Properties

### (2-1) Enhancement in Optical Properties of Marine Luciferases

In the present invention, "enhancement in optical properties of marine luciferases" mainly means enhancement in bioluminescence intensity and improvement in stability of observation of reporter gene expression. However, it also encompasses a shift of the luminescence wavelength to the longer wavelength side. This is an important nature to expand the utilization of the reporter gene, because the shift to the long wavelength side enhances transmittance through cells or skin.
The two-dimensional distribution of bioluminescence intensity can be obtained by measuring bioluminescence intensity in a specific wavelength range after addition of the substrate using a known luminescence spectrophotometer. By performing such measurement with time, luminescence stability with time may also be measured. A luminescence plate reader, which has superior sample processing property, may also be used to obtain more accurate data. Here, the areas of respective luminescence spectrums are measured after sufficient cell lysis time (about 20 minutes). The shift to a long wavelength side may be measured by a wavelength scanning method, or using a long wavelength filter.

### (2-2) Modification Method

The modification of the amino acid sequence of the marine luciferase of the present invention may be performed by causing a chemical change directly on the desired amino acid residue or residues of the amino acid sequence; however, the modification is generally performed by point mutation of the base corresponding to the target amino acid among the base sequence encoding the enzyme. The mutation of the base may be performed by a well-known method, such as site mutation. The following description discusses the case of introducing point mutation according to the "Quick-Change method" (Non-patent Literature 20); however, the present invention is not limited to this method.
More specifically, the introduction of mutation is conducted by (i) performing PCR reaction in the co-existence of a pair of sense primer and antisense primer of about 30 bases prepared to have a mutation point with a E-coli-derived plasmid template containing DNA coding GLuc, thereby inducing site-specific mutation. (ii) DpnI enzyme treatment, which enables decomposition of only *E-coli-derived* plasmid, is performed to decompose the plasmid used as the template contained in the PCR product. (iii) The presence or absence of mutation is confirmed by performing gene sequence assay.
For the template DNA encoding GLuc, the base sequence (GENE ACCESSION #: FJ010198) registered in a known database may be used without modification, or with partial modification. Here, as stated below in Section 3, it is possible to use a modified DNA for further improvement of the function as a template DNA for the Quick-Change method. To synthesize the DNA encoding the GLuc or a modified DNA, a set of multiple primers are prepared first. Then, the primers are set to the sense and antisense positions to be subjected to PCR reaction, thereby extending the two fragments to create a DNA template containing the entire length of Gaussia luciferase gene. This template may be mass-produced by, for example, introducing the template into eukaryotic expression vectors pcDNA3.1(+) and culturing them in procaryotic cells such as Escherichia coli.

### (2-3) Modification Strategy for Improving Optical Properties

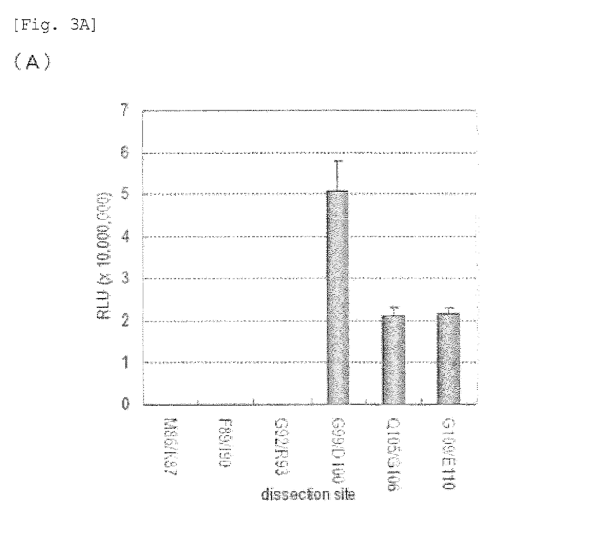

To determine the replacement sites of amino acid residues in marine luciferase, the present inventors first focused attention on the following points.
(i) According to a hydrophobic sequence assay of the entire amino acid sequence of GLuc, there is a hydrophilic amino acid region in the central region of the enzyme (Fig. 4B). The hydrophilic region in the central region of a protein denotes that the portion is externally exposed. Therefore, the inventors presumed that the portion is a region where the substrate can easily approach; more specifically, the portion is an interaction site with a substrate, i.e., the region includes the active center of the enzyme.
(ii) According to a report (Non-patent Literature 15) regarding the crystal analysis of Renilla-derived luciferase (Renilla luciferase; RLuc), which is a marine luciferase, a hydrophobic amino acid, such as isoleucine (Ile), is present at regular intervals in the vicinity of the hydrophilic region, which is the presumed enzyme active site (as explained in (i)). Since such regular appearance of hydrophobic amino acids is also observed in GLuc, the inventors speculated that the region near the isoleucine (Ile) repeat sequence is the critical area of enzymatic activity where the interaction with the substrate takes place.
(iii) In the past, the present inventors conducted a series of divisional studies in which six portions of the central region of GLuc are cleaved, and thereby found that the regions in the vicinity of amino acids No. 93 and 98 are the critical area of enzymatic activity (Non-patent Literature 14) (Fig. 3A). This finding is consistent with the findings described in (i) and (ii) above. Following a thorough study of the above findings (i) to (iii), the inventors speculated that the area corresponding to 74 to 131 of GLuc among the central region of the amino acid sequence of marine luciferase is the active center of enzyme. The inventors further speculated that the area near the hydrophobic amino acid repeat sequence is the portion where the interaction with the substrate actually takes place.
Therefore, in the following, point mutation is introduced mainly into hydrophobic amino acids at positions 74 to 131 in the GLuc sequence.
The amino acids to be replaced were selected according to the three hypothesized interactions with the substrate in the active center, i.e., (a) a hydrogen bond with the substrate (b) an aromatic interaction with the phenyl group of the substrate, and (c) an interaction between respective hydrophobic residues of the substrate and the enzyme. The inventors hypothesized that the enzyme active group could be improved by adjusting these three interactions.
Further, in the replacement of amino acid residues, the inventors set the major condition "conservative amino acid replacement," in which the candidate is selected from a group of similar amino acid residues, so as to avoid changes in the environment of the enzyme active center where enzymatic activity takes place.
More specifically, the amino acids that presumably form a hydrogen bond with the substrate as stated in (b) are amino acids irrelevant to the peptide bond, or polar amino acids having carboxy group, amide group or hydroxyl group; namely, asparagine, serine, threonine, glutamine, cysteine or like neutral amino acids; asparagic acid, and glutamic acid or like acidic amino acids; and arginine, histidine, and lysin or like basic amino acids. Among these amino acids, asparagine, threonine, serine and glutamine as typical neutral amino acids, glutamic acid as typical acidic amino acid, and histidine as typical basic amino acid were selected to replace amino acids at positions 90, 93 to 95, 97, 99, and 100.
Specific examples of amino acids capable of aromatic interaction with the phenyl group of the substrate as stated in (b) include aromatic amino acids or heterocyclic amino acids having a π-electron, namely, phenylalanine (Phe), thyrosine (Tyr), tryptophan (Trp), and histidine (His). Using these amino acids, the amino acids at positions 90, 93 to 95, 97 and 130 are replaced. Among them, thyrosine (Tyr) and histidine (His) belong to the aforementioned amino acid group (a), and phenylalanine (Phe) and tryptophan (Trp) belong to the amino acid group (c).
Specific examples of amino acids capable of interaction between respective hydrophobic residues of the substrate and the enzyme as stated in (c) are hydrophobic nonpolar amino acid residues, namely, Leu (leucine), Ile (isoleucine), Val (valine), Ala (alanine), Phe (phenylalanine), Pro (proline), Met (methionine), Trp (tryptophan), and Gly (glycine). As typical examples of these amino acids, Leu (leucine), Ile (isoleucine), Val (valine), phenylalanine (Phe), tryptophan (Trp), and glycine (Gly) were used to replace the amino acids at positions 74, 77, 90, 93 to 95, 97, 108, 112, 115, 118, 121, 130, and 131. Phenylalanine (Phe) and tryptophan (Trp) belong to the aforementioned amino acid group (b).

### (2-4) Luciferase Variant of the Present Invention with Improved Luminescent Function

According to the results of the experiment based on the aforementioned strategies (i), (ii) and (iii), improvement in optical properties was observed in amino acids having mutations at positions 89 to 118 among the amino acids 74 to 131 of GLuc. More specifically, conservative amino acid replacement was performed with respect to an amino acid at a position corresponding to position 89, 90, 95, 97, 100, 108, 112, 115, or 118 in the amino acid sequence of GLuc, thereby enhancing the bioluminescence intensity of luciferase and/or stabilizing the bioluminescence intensity, and/or causing a shift to the longer wavelength side. Thus, improvement in luminescent function of luminescence enzyme was accomplished. The replacement that rendered the greatest improvement was a conservative amino acid replacement at a position corresponding to position 90, 108, 112, 115, or 118 in the amino acid sequence of GLuc based on the aforementioned strategy (iii), or a conservative amino acid replacement at a position corresponding to position 89 or 97 in the amino acid sequence of GLuc based on the aforementioned strategy (ii). The replacement enhanced the intensity of luciferase, stabilized the luminescence, and caused a shift to the longer wavelength side. More specifically, the replacement of the hydrophobic nonpolar (in particular, aliphatic) amino acid residue (more specifically, isoleucine) at position 90, 108, 112, 115, or 118 with other hydrophobic nonpolar (in particular, aliphatic) amino acid residue (more specifically, leucine, tryptophan, or valine); and the replacement of the aromatic amino acid residues at position 89 or 97 (more specifically, phenylalanine at position 89 or thyrosine at position 97) with other aromatic amino acid residue (more specifically, tryptophan) particularly enhanced and stabilized the bioluminescence intensity, while causing a shift to the longer wavelength side. In particular, the variant (I90L) obtained by replacing isoleucine at position 90 with leucine was a superior luciferase variant having significantly high and stable bioluminescence intensity, and a shift to a long wavelength side.
The replacement of leucine at position 90 with thyrosine is also regarded as a replacement between hydrophobic amino acid residues, and therefore may also be regarded as a conservative amino acid replacement. This replacement caused a shift to long wavelength, even though no enhancement in bioluminescence intensity was observed. According to the fact that the replacement of phenyl alanine at position 89 or thyrosine at position 97 with tryptophan is also considered replacement between hydrophobic amino acid residues, the above effect can also be expressed such that "replacement of hydrophobic amino acid residues corresponding to position 89, 90, 97, 108, 112, 115, or 118 in GLuc respectively with another hydrophobic amino acid residue improves activity of a luminescence enzyme; such improvement is specifically substantiated by enhancement and stabilization of bioluminescence intensity and/or a shift to the longer wavelength side.
Further, since the replacement of a hydrophilic amino acid residue at a position corresponding to position 95 or 100 in the amino acid sequence of GLuc that forms a hydrogen bond with another hydrophilic amino acid residue (more specifically, replacement of histidine at position 95 with glutamic acid, or replacement of asparagic acid at position 100 with asparagine) causes a shift to the longer wavelength side, it was confirmed that the aforementioned strategy (i) is also effective to cause a shift of luminescence wavelength to a long wavelength side. A shift to a long wavelength side is equivalent to an enhancement in light transmittance in the observation through cell membrane or skin. Therefore, this is a desired characteristic.

Furthermore, it was also confirmed that the optical property was further improved by introducing multiple mutations according to the aforementioned strategies (i), (ii) and (iii) instead of introducing only one mutation into one of the above positions. More specifically, in the present invention, it is also effective to introduce mutations into multiple sites at the same time. For example, by combining the mutation for enhancing bioluminescence intensity and for improving luminescence stability (more specifically, the mutation of at least one position corresponding to positions 89, 90, 108, 112, 115, and 118 in the amino acid sequence of GLuc with a hydrophobic amino acid or acids) with the mutation for causing the shift of the luminescence wavelength to a long wavelength side (more specifically, the mutation of at least one position corresponding to positions 90, 95, 97 and 100 in the amino acid sequence of GLuc), it becomes possible to further enhance the bioluminescence intensity, improve luminescence stability, and cause a shift to the longer wavelength side.
More specifically, both the modified luciferase (F89W/I90L) obtained by replacing isoleucine (I) at a position corresponding to position 90 in the amino acid sequence of GLuc with leucine (L), and replacing phenylalanine (F) at a position corresponding to position 89 in the amino acid sequence of GLuc with tryptophan (W); and a modified luciferases (I90L/I115L) obtained by replacing isoleucine (I) at a position corresponding to position 90 in the amino acid sequence of GLuc with leucine (L), and replacing isoleucine (I) at a position corresponding to position 115 in the amino acid sequence of GLuc with leucine (L) were revolutionary luciferases that have very high and stable bioluminescence intensity, and cause a shift to the longer wavelength side.

In order to obtain a variant with further improved luminescent function, a modified luciferases having mutations at "F89W/I90L" was further mutated by replacing histidine (H) at a position corresponding to position 95 in the amino acid sequence of GLuc with glutamic acid (E) or glutamine (Q), and replacing thyrosine (Y) at a position corresponding to position 97 in the amino acid sequence of GLuc with tryptophan (W) (F89W/I90L/H95E/Y97W); and a further shift to the long wavelength side was observed while maintaining the improved bioluminescence intensity and stability. In particular, the modified luciferase "F89W/I90L/H95E/Y97W" showed a great shift to the long wavelength side, which was shown as an increase of the peak value from 471 nm (absorbency), which indicates the maximum bioluminescence intensity of the original GLuc, to 506 nm (absorbency).

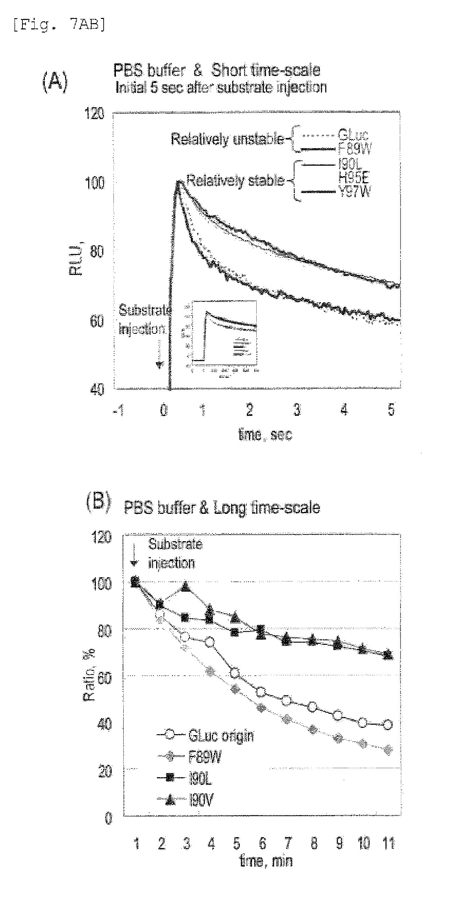

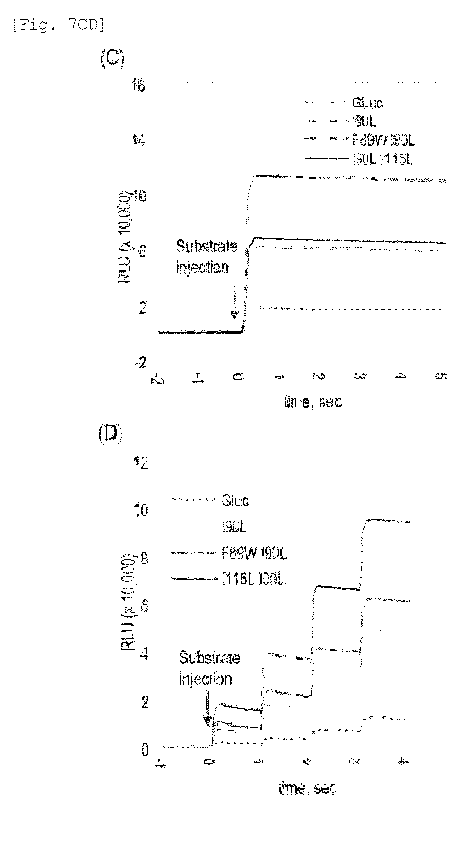

The stability of the bioluminescence intensity is confirmed by introducing a substrate (dissolved in a PBS buffer) into each variant, and observing its changes in bioluminescence with time. As a result of a 5-second observation of I90L, H95E, and Y97W that are typical GLuc variants, it was found that they had stable bioluminescence intensities (Fig. 7A). Further observation (10 minutes) revealed that, whereas the decrease in bioluminescence intensity of the original GLuc (white circle) was significant, the bioluminescence intensity of I90L (black square) and the bioluminescence intensity of I90V (gray triangle) were relatively stable (Fig. 7B). The same result was obtained from a short-time (5 seconds) observation of each variant for its changes in bioluminescence with time after introducing a substrate (dissolved in a modified Matthew's buffer) (Fig. 7C). Furthermore, another observation of 1 minute for each stage of changes in bioluminescence with time after introducing a substrate (dissolved in a modified Matthew's buffer) stepwise into each variant also revealed that each variant had very stable and strong bioluminescence (Fig. 7D).

### (2-5) Other Gaussia-like Luciferase Variants

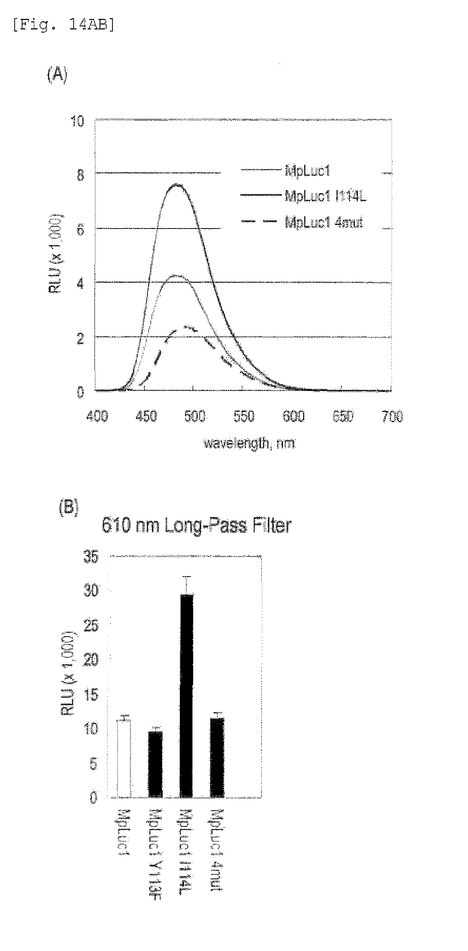

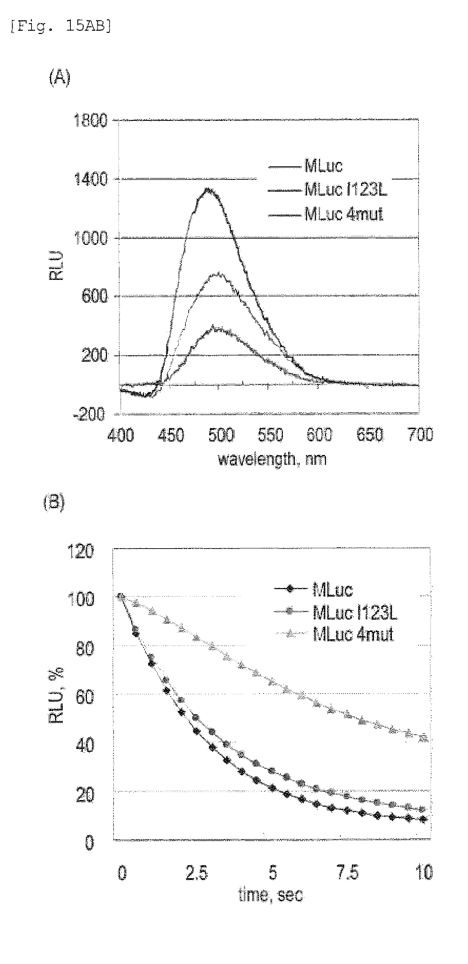

Using a Gaussia luciferases derived from a Copepod *(Metridia pacifica* or *Metridia longa*), which is a Gaussia-like marine animal very similar to Gaussia, namely, luciferases of MpLuc1 (derived from *Metridia pacifica*) and MLuc (derived from *Metridia longa*)*,* a MLuc-I123L variant and a MpLuc1-I114L variant were obtained by carrying out conservative amino acid replacements of an amino acid at position 123 of MLuc and an amino acid at position 114 of MpLuc1 corresponding to the amino acid at position 90 of GLuc; also obtained were a Y122W/I123L/H128E/Y130W variant (MLuc4) of MLuc and a Y113W/I114L/H119E/Y121W variant (MpLuc4) of MpLuc1 corresponding to a F89W/I90L/H95E/Y97W variant (Mon3) of GLuc. The luminescence characteristics of these variants were measured, with the result that their bioluminescence intensities were enhanced as in the GLuc variant. The results also revealed an enhancement in stability and a shift to the long wavelength side (Fig. 14A to 14C and Fig. 15A to 15D). This confirmed that the findings regarding GLuc are also applicable to all of the other Gaussia luciferases.

### (2-6) Luciferase Variant with Improved Luminescent Function used for the Present Invention

The above results revealed that the marine luciferase of the present invention, in particular, the Gaussia luciferase, is obtained by "conservative amino acid replacement" of at least one amino acid residue selected from the amino acid residues at a position corresponding to positions 89, 90, 95, 97, 100, 108, 112, 115, and 118 among the amino acid sequence at positions corresponding to positions 89 to 118 of Gaussia luciferase (GLuc). More specifically, such replacement includes replacement of a hydrophobic amino acid residue at a position corresponding to position 89, 90, 97, 108, 112, 115, or 118 in the amino acid sequence of GLuc with another hydrophobic amino acid residue, or replacement of a hydrophilic amino acid residues at a position corresponding to position 95 or 100 in the amino acid sequence of GLuc that forms a hydrogen bond with another hydrophilic amino acid residue.
This hydrophobic amino acid is preferably a hydrophobic amino acid having a greater molecular weight than a small-size hydrophobic amino acid such as glycine (Gly). An aromatic amino acid, such as tryptophan (Trp), may be used insofar as it is hydrophobic and nonpolar. In particular, among the hydrophobic amino acids, aliphatic amino acids, such as leucine (Leu), valine (Val), isoleucine (lie) or tryptophan (Trp), are preferable. Leucine (Leu), valine (Val), and tryptophan (Trp) are particularly preferable.
Further, when the replacement of a hydrophobic amino acid residue with another hydrophobic amino acid residue is replacement of a hydrophobic nonpolar aliphatic amino acid residue at a position corresponding to position 90, 108, 112, 115, or 118 in the amino acid sequence of GLuc with another hydrophobic nonpolar aliphatic amino acid residue (in particular, the replacement of isoleucine with one amino acid residue selected from leucine, tryptophan and valine), or replacement of a hydrophobic aromatic amino residue at a position corresponding to position 89 or 97 with another hydrophobic aromatic amino residue (the replacement of phenylalanine (Phe) at a position corresponding to position 89 or thyrosine (Tyr) at a position corresponding to positions 97 with tryptophan (Trp)), the resulting luciferase variant has an enhanced bioluminescence intensity, as well as stable bioluminescence intensity or a shift to the longer wavelength side. Thus, the enhancement in activity of luciferase can be accomplished. The most preferable combination of the mutation position and the mutating amino acid for such replacement is replacement of a position corresponding to position 89 in the amino acid sequence of GLuc with tryptophan (Trp), replacement of a position corresponding to position 90 in the amino acid sequence of GLuc with Leu (leucine) or Val (valine), and replacement of position 108 with Val (valine).
Further, the replacement of a hydrophilic amino acid residue that forms a hydrogen bond with another hydrophilic amino acid residue is replacement of histidine (His) at position 95 in the amino acid sequence of GLuc with glutamic acid (Glu), or replacement of asparagic acid (Asp) at position 100 in the amino acid sequence of GLuc with asparagine (Asn). Such a replacement causes a shift to the longer wavelength side of the resulting modified luciferase.
Among the obtained modified luciferases, the modified luciferases (GLuc-I90L, MpLuc1-I114L, MpLuc1-I114L) having a mutation caused by replacement of isoleucine corresponding to position 90 in the amino acid sequence of GLuc had the highest intensity, and improved stability, and a shift to the long wavelength side. It is more preferable to produce a variant by replacing an amino acid corresponding to position 89 in the amino acid sequence of GLuc with tryptophan, replacing an amino acid at position 90 with leucine or valine, replacing an amino acid at position 95 with glutamic acid, replacing an amino acid at position 97 with tryptophan, replacing an amino acid at position 108 with valine, replacing an amino acid at position 112 with leucine or valine, replacing an amino acid at position 115 with valine, or replacing an amino acid at position 118 with leucine or valine, so as to further significantly enhance bioluminescence intensity and cause a shift to the longer wavelength side. In particular, the leucine variants (GLuc-I90L, MpLuc1-I114L, MpLuc1-I114L) having mutation at a position corresponding to position 90 in the amino acid sequence of GLuc are excellent modified luciferases with not only a high, bioluminescence intensity but also a significant shift to the longer wavelength side.
In the present invention, "a luciferase variant (modified luciferase)" preferably denotes a variant basically having no mutation with respect to the amino acid sequence of the original Gaussia luciferase other than the aforementioned "conservative amino acid replacement", in the region corresponding to positions 89 to 118 in the amino acid sequence of GLuc, i.e., in the active center of the amino acid sequence. However, as long as the specified region has no mutation other than the conservative amino acid replacement, the variant may have mutation in other positions on the condition that the mutation is not conducive to a severe change in the entire insteric structure. In particular, the variant may contain a deletion at a part of the N- or C-terminal region, for example, deletion of 1 to 50, preferably 1 to 30, more preferably 1 to 20, further preferably 1 to 10 amino acid residues in the N- or C-terminal region. More specifically, the variant may mean a modified Gaussia-like luciferase having an amino acid sequence with homology of 70% or more, preferably 80% or more, more preferably 90% or more, most preferably 95%, except for the region corresponding to positions 89 to 118 in the amino acid sequence of GLuc.

### 3. Change of Marine Luciferase Gene of the Present Invention to Optimal Codons for Mammalian

The usages of the marine luciferase of the present invention are described in Section 4 below. Typically, the usages include those as a luciferase component of a known bioluminescent probe, and, owing to its high bioluminescence intensity, as a substitute for a reporter gene for fluorescent imaging in vino. Since analysis methods using reporter genes, such as the reporter-gene assay or the two-hybrid method, are performed in the hitherto-established host cell systems, the series of procedures of producing a vector by binding the luciferase variant gene of the present invention with the promoter of the target gene, and culturing the host cell incorporating the vector may be performed according to well-known methods. Examples of host cells used herein include yeast cells, bacteria cells such as *Escherichia coli,* and insect cells, as well as mammalian cells (COS cell, CHO-K1 cell, HeLa cell, HEK293 cell, NIH3T3 cell) used for general gene recombination. The present invention is mainly used in mammalian cells, such as humans, in *vivo* or in *vitro.*
Further, the luciferase variant gene of the present invention is preferably modified to have codons suitable for the target cell, thereby modifying the base sequence into a sequence that is more easily expressed in the host cell. The luciferase variant gene of the present invention also needs a modification to contain a restriction enzyme site to be introduced into a vector. These modifications may also be performed according to known methods. As one example, a modification strategy of a variant assumed to be used in mammalian cells, such as humans in vivo or in mammalian cells in vitro, is described below.
More specifically, the modifications for improving other functions include modification of the codons corresponding to the amino acid into those suitable for mammalian so that the gene has a base sequence more easily expressed in the bodies of mammalian such as humans, and a modification to introduce a restriction enzyme site at a desired position. Accordingly, the present invention adopts such modifications as required. The modification strategies for improving functions are specifically described below. However, the present invention is not limited to these examples.

(i) The modification was conducted by taking into consideration the fact that organisms have a common molecular mechanism for expressing the same DNA, but may have a different gene codon proportion depending on the species. For example, an animal cell has a higher proportion of G or C in the nucleic acid than that in *Escherichia* coli, or tends to express genes with a higher proportion of G and C. Therefore, in order to ensure effective expression of GLuc in an animal cell, the known gene sequence of the GLuc is modified by optimizing the codons to have a higher proportion of G or C.
(ii) The Kozak sequence (GCCACC**ATG**G) is introduced in the first position of the gene so as to ensure high expression in eukaryotic cells.
(iii) The original extracellular secretion signal (1-17AA) on the upstream of GLuc gene, which is irrelevant to the luminescence activity, is deleted or replaced with another known extracellular secretion signal or intracellular signal. Alternatively, an additional intracellular signal may be provided in the C-terminal end while leaving the secretion signal as is.

In the embodiments of the present invention, a template of DNA encoding GLuc was designed according to the sequence information of GLuc, in consideration of the above points (i) to (iii). A primer set for synthesizing the DNA was constructed, and the template of GLuc gene was completed by PCR reaction. The template was ligated into pcDNA3.1(+) of a eukaryotic cell-expression vector to carry out subcloning.
Using the above expression vector as a template, a point mutation was introduced according to the aforementioned "Quick-Change method." More specifically, a PCR reaction was performed by coexisting a sense primer and an antisense primer of about 30 bases prepared by containing a mutation point, thereby inducing site-specific mutation. Then, only the E. coli-derived plasmid containing the GLuc gene template was decomposed by DpnIenzyme, and the gene sequence was analyzed to confirm the presence or absence of mutation.

### 4. Method for Confirming Enzymatic Activity of Mutant Enzyme of the Present Invention

The enzymatic activity of mutated GLuc may be verified, for example, in the following manner.
First, using a known lipid reagent for gene introduction, an expression vector having a GLuc variant is introduced into African monkey cells (COS-7); while also introducing, as a control, an expression vector having a known unmodified GLuc without any mutation into the cells in the same manner. At a predetermined time (from 10 to 20 hours, for example, 16 hours) after the introduction of the vector, a cell lysate is prepared using a known lysis solution.
Thereafter, the cell lysate is mixed with a known substrate solution containing coelenterazine, and its optical intensity, short-term stability in luminescence, etc., are measured.
The bioluminescence intensity may be found by measuring the intensity at a specific wavelength using a known luminescence spectrophotometer after addition of a known substrate. By performing the measurement every minute, the short-term stability in luminescence can be found. To measure a shift to the longer wavelength side, scanning of the entire wavelength is necessary.
In the embodiments of the present invention, the variant obtained by replacing hydrophobic amino acids at positions corresponding to positions 89, 90, 108, 112, 115, and 118 in GLuc with other hydrophobic amino acids had enhanced bioluminescence intensity. More specifically, a variant obtained by replacing the amino acid 89 with tryptophan, 90 with leucineor valine, 108 with valine, 112 with leucineor valine, 115 with valine, and 118 with leucineor valine showed an intense luminescence spectrum peak (luminescence spectrophotometer). In the measurement of the area of luminescence spectrum using a luminescence plate reader, the glutamic acid variant having a mutation at position 95 and the tryptophan variant having a mutation at position 97 showed a significant enhancement in bioluminescence intensity, as well as the leucine variant having a mutation at position 90; further, these variants all caused a shift to the longer wavelength side. In particular, the leucine variant (I90L) having a mutation at position 90 is a superior luciferase variant that has a high luminescence intensity and a significant shift to the longer wavelength side. Further, the variant with two simultaneous mutations (F89W/I90L or I90L/I115L), i.e., the leucine replacement at position 90, and tryptophan replacement at position 89 or leucine replacement at position 115 at the same time; and the variant with four simultaneous mutations (F89W/I90L/H95E/Y97W), i.e., leucine replacement at position 90, tryptophan replacement at position 89, glutamic acid replacement at position 95, and tryptophan replacement at position 97 at the same time, both showed a significant bioluminescence intensity and a significant shift to the long wavelength side. Thus, they both were revolutionary luciferase variants.
Further, the variant having replacement with glutamine (Gln) or asparagine (Asn), which is a neutral amino acid forming a hydrogen bond, at position 95 or 100 showed a shift of bioluminescence to the long wavelength side. Similarly, it was confirmed that the variants having mutations at positions 90 and 97 showed a shift of bioluminescence to the longer wavelength side, regardless of whether the mutation introduces leucine (Leu), which is a hydrophobic amino acid, thyrosine (Tyr), which is an amino acid forming a hydrogen bond and also an aromatic amino acid, or tryptophan (Trp), which is an aromatic amino acid and also a hydrophobic amino acid. The shift to the longer wavelength side was substantiated by the luminescence peaks of variant Y97W and variant 190Y, which were 486 nm and 492 nm, respectively, relative to the luminescence peak of GLuc, which was 471 nm. Further, the variants I90L, F89W/I90L, and I90L/I115L also showed significant shifts to the long wavelength side, as they had luminescence peaks of 486 nm, 481 nm, and 483 nm, respectively. In particular, the luminescence peak of the variant F89M/I90L/H95E/Y97W was 506 nm, showing a revolutionary shift to the longer wavelength side. Such a long wavelength bioluminescence improved transmittance through living tissues, and is thereby highly useful as a valuable analytical signal.

### 5. Use of the Present Invention as Modified Luciferase Gene

The gene of the present invention encoding a .luciferase variant is most expected to be used as a reporter gene for various analysis systems, as with the conventional luciferase genes, and as a bioluminescent probe that is currently under research and development.

### (5-1) Reporter-gene assay

The marine luciferase variant of the present invention may be used as the widely known reporter-gene assay (reporter-gene assay), yeast two-hybrid assay, mammalian two-hybrid assay, protein splicing assay, protein complementation assay, circular permutation assay, bioluminescence resonance energy transfer assay (BRET) or the like as a core element, and is conducive to significant improvement in measurement efficiency in these assays. The marine luciferase variant of the present invention can completely overcome the following drawbacks of conventional firefly luciferase:
(i) Due to its large molecular weight, it takes a long time to start expression, thereby imposing a great burden on the host cells.
(ii) Due to the low bioluminescence intensity of firefly luciferase, it generally takes 1 to 2 days after stimulation to obtain a sufficient accumulation of luciferase (reporter).
Since the use of the superluminescent luciferase of the present invention ensures a significantly high bioiuminescence intensity of the reporter (in particular, the variant I90L has a bioluminescence intensity 14000 times greater than that of a known firefly luciferase), it has an advantage of very prompt measurement after the stimulation. Accordingly, the measurement time can be greatly reduced while ensuring high short-term stability in luminescence, thereby enabling luminescence measurement even for a cell strain with insufficient gene introduction. Further, the redshifted luminescence improves transmittance through cell membrane or skin, thereby ensuring high measurement sensitivity.
More specifically, the superluminescent luciferase of the present invention is adopted to these reporter-gene assays in such a manner that the luciferase is linked to a known eukaryotic cell expression vector containing a special promoter in an upsteam portion, and the vector is then introduced into a eukaryotic cell. After a predetermined time, the measurement is performed using the luciferase either in the presence or absence of signal (stimulation) (Non-patent Literature 13). A known p-TransLucent vector can be used as this expression vector for reporter-gene assay that can carry the lucifierase of the present invention; the luciferase can easily be incorporated therein using a known method. The commercially available pG5Luc vector can be used as this vector for the two-hybrid assay; the luciferase can easily be incorporated therein using a known method.

### (5-2) Application to luminescent probe

Further, by incorporating the superluminescent luciferase of the present invention into the single-molecule-format luminescent probe (Non-patent Literature 9, Non-patent Literature 10, Non-patent Literature 11, Non-patent Literature 12, Japanese Patent Application No. 2007-005144, 2007-202308, 2007-332253, 2008-116098, and 2009-025229, PCT/JP2008/050370, US12/025532, US12/343830), or the two-molecule-format luminescent probe (Non-patent Literature 4, Non-patent Literature 6, and Non-patent Literature 7) which are recited in the pending patents applied by the present inventors, the presence or absence of ligand and the intensity of ligand activity can be observed with high bioluminescence intensity. By comprising, as the probe components, (i) the dissected luciferase (N- and C-terminal fragments), and (ii) a ligand-binding protein responsive to the target ligand and (iii) a recognition protein that recognizes the bond of the ligand with the ligand-binding protein, which are linked to the vicinity of the dissected luciferase, it is possible to form a high-performance luminescent probe. This luminescent probe functions such that, as the recognition protein recognizes the bond of the ligand binding of the ligand-binding protein, the two fragments of the dissected enzyme complement compensate each other and thereby change the enzymatic activity. Here, due to the high bioluminescence intensity and stability of the dissected enzyme, it is possible to perform reliable measurement with an improved detection limit.

In the present invention, "single molecule-format luminescent probe" denotes a known bioluminescent probe in which all components for visualization imaging are integrated in a single fusion molecule (disclosed in Patent Literature 7, etc.). For example, "single molecule-format luminescent probe" denotes a fusion protein that comprises, as fundamental components, the two fragments of N- and C-terminals obtained by dissecting the marine luciferase variant of the present invention, a ligand-binding protein, and a recognition protein for recognizing the ligand-binding protein. Similarly, "two molecule-format luminescent probe" in the present invention denotes a bioluminescent probe in which the two fragments of N- and C-terminals obtained by dissecting the marine luciferase variant of the present invention are present in the fusion protein containing the ligand-binding protein, and in the fusion protein containing the recognition protein, respectively.
As such, when the marine luciferase superluminant variant of the present invention is used for these bioluminescent probes, the variant must be dissected into a N-terminal fragment and a C-terminal fragment. The dissection portion is the same as the dissection portion of the marine luciferase used for the known "single molecule-format luminescent probe" and "two molecule-format luminescent probes".
More specifically, the GLuc variant of the present invention is dissected at a portion between 105 and 110, and the MLuc variant of the present invention is dissected at a portion between 138 and 142.
Further, in addition to the single molecule-format bioluminescent probe, the superluminant luciferases may also be incorporated in the circular permutation replacement probe (Non-patent Literature 11; Non-patent Literature 12) developed by the present inventors, thereby enabling efficient calculation of intracellular molecular phenomenon with stable and high bioluminescence intensity. In addition to 8990N and Mon3N, i.e., the single molecule-format bioluminescent probe described in Example 9 of the present invention, the circular permutation replacement probes are cPresso and cPressoMax in the sequence listing.

Regarding the actual method for using the superluminescent luciferase of the present invention as a single molecule-format luminescent probe, Patent Literature 7, and Japanese Patent Application Nos. 2007-332253 and 2009-025229 disclose the details thereof. More specifically, the marine luciferase of the present invention is dissected, and a chimera DNA encoding a luminescent probe in which a ligand-binding protein and a peptide sequence, which recognizes the change in steric structure upon binding of a ligand to the protein, are linked in a linear chain form. Generally, the chimera DNA is subcloned into a vector suitable for the cells in which the chimera DNA is intended to be expressed, and the vector is introduced into the cells to be expressed. However, the chimera DNA may be ligated to a control sequence at an upstream portion to be directly introduced into the cells. The target cells are preferably mammalian-derived cells such as human cells. Other suitable examples include cells that exist in a living subject, and culture cells that retain the native function, yeast cells, insect cells, and procaryotic cells such as *Escherichia coli.* The type of the vector is also not particularly limited. A suitable vector capable of being expressed in the target host cells is appropriately selected. The introduction of the vector into the cells are performed using known transfection methods such as a microinjection method or an electroporation method, or a transfection method using a lipid reagent (BioPORTER (Gene Therapy Systems, Inc.), Chariot (Active Motif), etc.).
Since the bioluminescent probe using the superluminescent luciferase of the present invention is introduced into cells as a chimera DNA and expressed in the cells as a fusion protein, by measuring the change in light amount emitted from the cells after subjecting the transgenic cell to ligand stimulation, the property or levels of activity of the ligand may be evaluated.

When the superlurninesoent luciferase of the present invention is incorporated in the bioluminescent probe, the "ligand-binding protein," which can be incorporated in the probe together with the luciferase, is intended to mean a protein that binds with a ligand at the ligand binding site. The ligand-binding protein may serve to, in response to the bond with the ligand, for example, change the steric structure, cause phosphorylation, or facilitate protein-protein interaction. Examples of such ligand-binding proteins include nuclear receptors (NR) to which such ligands as hormones, chemical substances, or signal transduction proteins bind; cytokine receptors; and various protein kinases. A suitable ligand-binding protein is selected depending on the target ligand. The ligand that binds to the ligand-binding protein is not particularly limited insofar as it binds to the ligand-binding protein. The ligand may be an extracellular ligand that is introduced in response to extracellular stimulus, or an intracellular ligand that is produced inside the cells. Examples thereof include agonists or antagonists of the receptor protein (for example, intranuclear receptor, or G-protein-linked receptor), signal transduction proteins such as cytokine, chemokine, or insulin, intracellular second messenger, lipid second messenger, phosphorylated amino acid residue, G-protein-linked receptor ligand and like ligands that specifically bind to proteins involved in intracellular signal transduction.
For example, when the intracellular second messenger, the lipid second messenger or the like is used as a ligand, the binding domain of each second messenger may be used as the ligand-binding protein. "Second messenger" denotes a different kind of intracellular signal transduction substance that is newly produced as a result of the bond of the extracellular signal transduction substance, such as a hormone or neurotransmission substance, with a receptor that exists in the cell membrane.
Examples of the second messengers include cGMP, AMP, PIP, PIP₂, PIP₃, inositol trisphosphate (IP₃), IP₄, Ca²⁺, diacylglycerol, and arachidonic achid. For example, for Ca2+ as the second messenger, calmodulin (CaM) may be used as the ligand-binding protein.

### (5-3) Intracellular Imaging

Further, using the gene encoding the superluminescent luciferase enables stable introduction of the luciferase into various cell strains. For example, using the gene enables stable introduction of the luciferase into the novel induced pluripotent stem cells (iPS cells). Since the induced pluripotent stem cells (iPS) do not emit light themselves, it has been very difficult to research the intracellular molecular phenomenon and tissue specificity of the cells. To address this difficulty, a molecular probe containing the luciferase is introduced into somatic cells before the embryo is formed; after the introduction of the luciferase-containing probe, the embryo is differentiated into various tissues. This enables measurement of specific molecular phenomena in respective organs at high sensitivity. This process is performed according to the method of Yamanaka et al. (Non-patent Literature 19).
Further, by linking the superluminescent luciferase of the present invention to a suitable signal peptide, the luciferase can be used for luminance imaging of various organelles. For example, by linking a GAP-43-derived MLCCMRRTKQV sequence to an N- or C-terminal of GLuc, the luciferase may be localized in cell membranes. Linking a GRKKRRQRRR sequence to a terminal enables localization in cell cytoplasm. Further, for localization in endoplasmic reticulum (ER) and cellular nucleus, KDEL and DPKKKRKV sequences, respectively, are linked to a terminal. Furthermore, by linking to HIS-tag (HHHHHH), FLAG-tag (DYKDDDDK), Myc-tag (EQKLISEEDL), HA-tag (YPYDVPDYA), V5-tag (GKPIPNPLLGLDST), T7-tag (MASMTGGQQMG) or like antigen sites, the luciferase can be used for immunostaining or separation/refinement in acellular systems. In these usages, known immunostaining technologies or immunocytochemistry may be adopted.

### 6. Other Remarks

The other terms and concepts used in the present invention are specifically defined in the descriptions of embodiments and examples. The terms are generally selected from the IUPAC-IUB Commission on Biochemical Nomenclature, or based on the interpretations of idiomatic terms and words in the related field. Further, except for the technologies with apparent sources, the various technologies used to carry out the present invention can be easily and consistently performed by persons skilled in the art in reference to published documents, etc. For example, genetic engineering and molecular biological techniques can be carried out according to J. Sambrook, E. F. Fritsch & T. Maniatis, "Molecular Cloning: A Laboratory Manual (2nd edition)," Cold Spring Harbor Laboratory Press, Cold Spring Harbor, New York (1989); D. M. Glover et al. ed., "DNA Cloning," 2nd ed., Vols. 1 to 4, (The Practical Approach Series), IRL Press, Oxford University Press (1995); Ausubel, F. M. et al., Current Protocols in Molecular Biology, John Wiley & Sons, New York, N.Y., 1995; *"*Zoku Seikagaku Jikken Koza 1 (Continuation of Biochemistry Experimental Series 1), Idensi Kenkyu Ho (Gene Study Method) II" edited by Tokyo Kagaku Dojin (1986); "Shin Seikagaku Jikken Koza 2 (New Biochemistry Experimental Series 2), Kakusan (Nucleic Acid) III (DNA Recombinant Technology)" edited by Tokyo Kagaku Dojin (1986); Japanese Biochemical Society, Tokyo Kagaku Dojin (1992); R. Wu ed., "Methods in Enzymology," Vol. 68 (Recombinant DNA), Academic Press, New York (1980); R. Wu et al. ed., "Methods in Enzymology," Vols. 100 (Recombinant DNA, Part B) & 101 (Recombinant DNA, Part C), Academic Press, New York (1983); R. Wu et al. ed., "Methods in Enzymology," Vols. 153 (Recombinant DNA, Part D), 154 (Recombinant DNA, Part E) & 155 (Recombinant DNA, Part F), Academic Press, New York (1987), etc.; the methods mentioned in the documents referenced in these documents, or other various similar methods and modified methods thereof that are substantially the same as the disclosed methods.
Further, the various proteins, peptides, and DNAs encoding them used in the present invention may be obtained from existing databases (URL: http://www.ncbi.nlm.nih.gov, etc.).

### Examples

Hereinbelow, the present invention is specifically described in detail with reference to examples; however, the present invention is not limited to these examples.
Note that the disclosures of the technical documents, patent publications, and patent application specifications are incorporated into the specification by reference.

### Example 1 Constriction of Gene Sequence Encoding GLuc

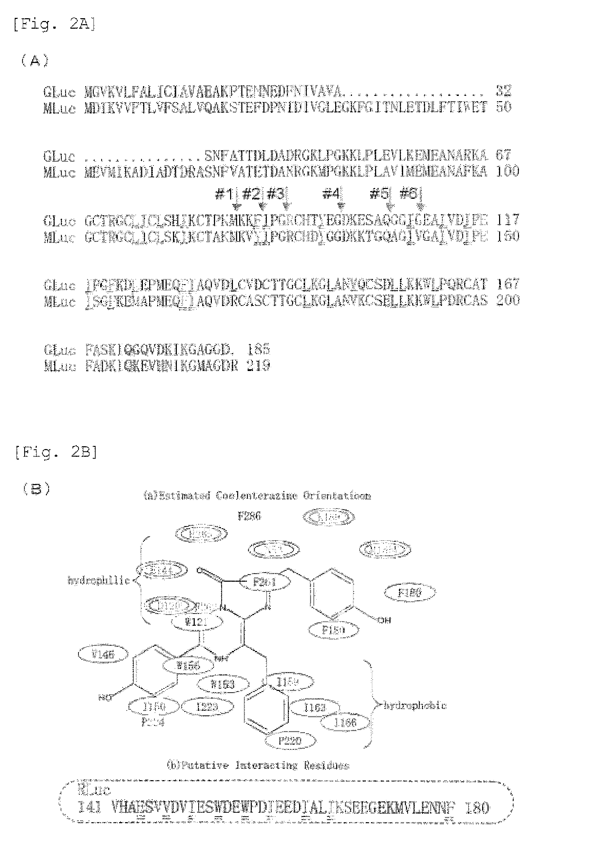

In the determination of a new gene sequence based on a known gene sequence (GENE ACCESSION #: FJ010198) of Gaussia luciferase (GLuc), codons were optimized for known mammalian cells. Specifically, codons that contain many Gs and Cs must be produced for optimal expression in mammalian cells. For example, of two bases, TTT and TTC, encoding Phe among the amino acids, TTC is more suitable for mammalian cells. Additionally, in order to increase the level of protein expression, a known Kozak sequence (GCCRCC**ATG**G) was inserted before and after the translation initiation codon (AUG).
In order to generate the above gene sequence, sense and antisense primers were palindromically placed, and a PCR reaction was performed to synthesize the full-length sequence. The full-length GLuc was used as a genetic template upon introduction experiments of other mutations. Fig. 2 shows the full length.

### Example 2 Search for Enzyme Active Site in GLuc and Introduction of Mutation

### (2-1) The following inferences were made in the introduction of a mutation into Gaussia luciferase (GLuc).

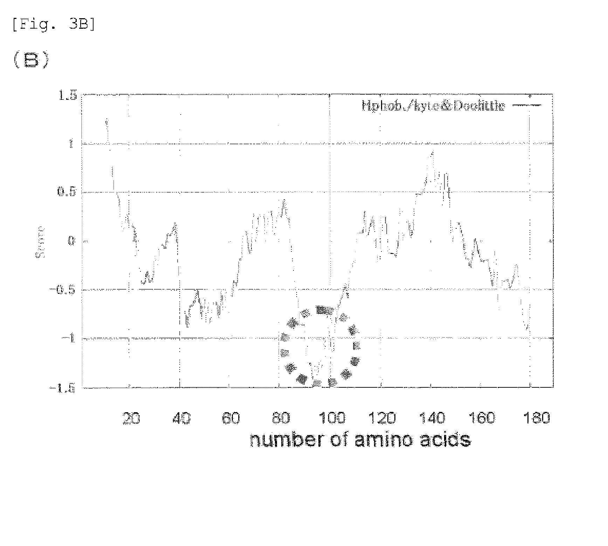

**Inference 1:** Through a detailed observation of the crystallographic structure (Fig. 3C) formed by Renilla luciferase (RLuc) upon binding to coelenterazine, which is a common substrate of marine luciferases, it was found that, in the active site of RLuc, (1) there is a region where hydrophobic amino acids are found at certain intervals (2 to 4 amino acid residues), and (2) the active site has a structure in which the substrate is sandwiched between hydrophilic and hydrophobic amino acid sites (Fig. 2B).
   Through an examination of GLuc amino acid sequence, it was found that, in the region at a similar site (amino acid numbers 74-161), hydrophobic amino acids (isoleucine (I), leucine (L), valine (V), phenylalanine (F), tryptophan (W), and the like) appear at certain intervals (2 to 4 amino acid residues); and it was inferred that, also in GLuc, this region is the substrate-binding region, which is the key site that influences luciferase activity.
**Inference 2:** First, in order to estimate the active site in the luciferase, several sites in the center of the enzyme were fragmented, and calmodulin (CaM) was inserted between the fragments, thereby examining whether the enzymatic activity is reconstituted. As a result, hardly any enzyme activity was observed at dissection sites #1-#3. On the other hand, a very strong bioluminescence was observed at dissection site #4 (Fig. 3A).
   It was confirmed from the study on the dissection sites in GLuc that the amino acid sequences near the dissection sites #3 and #4 are the key regions in GLuc that influence the enzyme activity of GLuc.
**Inference 3:** Further, the hydrophobic sequence search of GLuc revealed that the above site is the region of intersection of hydrophilic and hydrophobic amino acid domains (Fig. 3B).
**Inference 4:** Conventional studies on the chrompphores of fluorescent protein were examined, and the following techniques were brought into focus:
   (1) Introduction of amino acids (tyrosine (Y), serine (S), and the like) forming a hydrogen bond with a fluorescent chromophore into a position near the chromophore can produce an effect of extending a n-bond of the chromophore, as well as a color change to the longer wavelengths and a change in the bioluminescence intensity (Non-patent Literature 16).
   (2) Because the fluorescent chromophore contains a phenyl group in its skeleton, it was possible to make a better fluorescent protein by introducing an amino acid containing a phenyl group as a side chain into a position near the chromophore so as to interact with the phenyl group of the chromophore (Non-patent Literature 17).
   (3) A stable and high-intensity fluorescent color was achieved by introducing a hydrophobic amino acid into a position near a fluorescent chromophore so as to hydrophobically interact with the fluorescent chromophore (Non-patent Literature 18).

   As described above, it was found that, in regard to the fluorescent protein, there is a case where the introduction of a mutation into an amino acid near a fluorescent protein chromophore effectively improves the fluorescent protein.
   The comparison of the substrate of marine luciferases with the fluorescent protein chromophore reveals that their structures are closely similar to each other. Therefore, it was inferred that they share chromophores having very similar chemical structures; and that the fluorescent protein embeds the chromophore inside of the molecule, whereas the luciferase recruits the chromophore outside of the molecule in form of the substrate (Fig. 3C).
**Inference 5:** In a previous study by the present inventors on luciferase, there is a case of successful improvement of luminescence properties by partially overlapping the sequences near the enzyme active site. From this case, improvement in the properties of luciferase can also be expected by a technique of partially overlapping the sequences near the enzyme active site.

(2-2) Based on the above five inferences, a strategy for introduction of various mutations by conservative amino acid replacement into 18 positions including a putative luciferase active site and the vicinity thereof was examined (Fig. 4A), and a DNA encoding 57 GLuc variants was prepared in accordance with the strategy (Fig. 4). In the figure, "L74I," for example, indicates that a DNA mutation that replaces L (Leu) at position 74 with I (Ile) in the amino acid sequence of GLuc was introduced into GLuc gene; and "F89W, I90L" refers to a variant that replaced F (Phe) at position 89 with W (Trp) and, simultaneously, I (Ile) at position 90 with L (Leu).

### Example 3 Comparison of Bioluminescence intensities of GLuc variants into which Amino Acid Mutation has been Intraduced

A pcDNA3.1(+) vector into which the DNA of GLuc variants prepared in Example 2 was inserted was transfected into COS-7 cells cultured in a 12-well plate, followed by incubation for 16 hours.
Subsequently, cell lysates were prepared from the COS-7 cells in each well (5-minute lysis), and a spectrum was measured in the presence of a luciferase-specific substrate (coelenterazine), using a fluorescence spectrophotometer (F-7000, Hitachi), thereby examining the effect of the introduced mutation. Figs. 4B-1 and 4C show the results.
According to Figs. 4B-1 and 4C, some variants were found to exhibit longer-wavelength bioluminescence compared to the original GLuc, and a variant having remarkably high bioluminescence intensity was also discovered. Specifically, first, in the case of an introduction of a mutation that potentially induces an interaction between the substrate and a phenyl group, a redshift of about 10 nm was observed with a variant (Y97W) in which tyrosine (Y) at position 97 was mutated to tryptophan (W). From this result, it can be analyzed that a π-bond was extended because a bond was formed between the substrate and the phenyl group.
Further, as a result of a series of introductions of amino acid mutations that form a hydrogen bond with the above substrate, a redshift of about 7 nm in the spectrum was observed when histidine (H) at position 95 and aspartic acid (D) at position 100 were multiply mutated to glutamine (Q) and asparagine (N), respectively. From this result, it can be analyzed that a π-bond was partially extended because a hydrogen bond was formed between the substrate and each amino acid. Further, when F89W/I90L/H95E/Y97W mutations were introduced into the original GLuc, a variant whose peak luminescence wavelength was shifted to the longer wavelength side as much as 35 nm was obtained (Fig. 4D). Note that, although it is not shown in Figs. 4C or 4D, it has been confirmed that I90L variant also causes a shift of 15 nm at maximum to the long wavelength side (Fig. 4B-2).
The above-described variants are advantageous particularly in terms of the observation of bioluminescence in *vivo* because the variants show long-wavelength bioluminescence. Fig. 4E shows the results of the observations using a 610 nm long-pass filter on bioluminescence of 610 nm or longer (high tissue permeability) emitted by each variant. As predicted, it was found that I90L and the like exhibit a much higher optical intensity compared to the original GLuc.

Also, a mutant enzyme having high bioluminescence intensity was found when a hydrophobic amino acid mutation was introduced into a putative enzyme active site, resulting in the enhancement of the optical intensities. Fig. 4B-1 shows a graph formed using a luminescence spectrophotometer (F-7000, Hitachi) with the peak of each luminescence spectrum as the index. Additionally, in the preparation of cell samples, the samples are immersed in a lysis solution for 5 minutes. This allows preparation of non-uniform samples of cells whose organelles are not lysed with this quick manipulation procedure. As shown in the figure, GLuc variants having particularly high bioluminescence intensity are obtained when the original GLuc is mutated to be F89W, I90L, I90V, I108V, I112L, I115V, I118L, and I118V.
From this result, it was found that the bioluminescence intensities are greatly increased as a result of the introduction of amino acids into the mutation sites that hydrophobically interact with the substrate of GLuc. For example, F89W was 9-times, I90L was 14-times, I108V was 6.7-times, and I118L was 4.2-times higher than GLuc in terms of the bioluminescence intensity. In the case of I90L in particular, one amino acid mutation resulted in bioluminescence 14-times higher than the conventional case. This bioluminescence is 14,000 times higher than that of firefly luciferase (FLUC), which is currently the most used commercially.
Further, transformed COS-7 cells with genes of GLuc variants comprising additional mutation sites were prepared, and the above-described experiment for measurement of bioluminescence intensity was repeated under the same experimental conditions, including preparation of the cell lysate (Fig. 4B-2). Note that Fig. 4B-2 shows GLuc variants having particularly high bioluminescence intensity along with the wavelengths that showed maximum bioluminescence intensity. In this case, a protocol for immersing the lysate samples in lysis solution for 20 minutes was employed (uniform samples of cells whose organelles are lysed). The area of luminescence spectrum measured by a luminescence plate reader was used as the index of the bioluminescence intensity. According to Fig. 4B-2, particularly high bioluminescence intensity was shown when the original GLuc was mutated to be I90L, H95E, Y97W, F89W/I90L, I90L/I115L, and F89W/I90L/H95E/Y98W. The figure also shows that each of these variants was shifted to the longer wavelength side.
The above result confirmed that the bioluminescence increases its intensity to a very high level as a result of the introduction of amino acid mutations that hydrophobically interact with the substrate into the region corresponding to the active center of GLuc. For example, I90L was 7.1 times (hereinafter, based on the area ratio), H95E was 3.0 times, Y97W was 2.1 times, F89W/I90L was 10.2 times, I90L/I115L was 4.9 times, and F89W/I90L/H95E/Y98W was 4.4 times higher than GLuc in terms of the bioluminescence intensity. I90L, in particular, demonstrated bioluminescence that is 7.1 times higher than that of the original GLuc and 10,000 times higher than that of FLuc, as shown in the experimental results in Fig. 4B-2.

Further, in order to visually compare the GLuc variant having high bioluminescence intensity with other luciferases with respect to the bioluminescence intensity, a substrate (coelenterazine) was added to tubes containing solutions of FLuc, original GLuc, and the GLuc variant exhibiting high bioluminescence intensity (Fig. 5). As a result, it was visually observed that the very strong bioluminescence from the Superluminescent variant of GLuc (I90L) was demonstrated.
Fig. 6 shows bioluminescence intensities of the spectra, where the intensities of the GLuc variant (I90L) of the present invention were compared with those of known luciferases (firefly luciferase (FLuc), Gaussia luciferase (GLuc), click beetle luciferase (CBLuc), and the like). This figure also indicates that the bioluminescence intensity of the GLuc variant is remarkably high.

### Example 4 Measurement of Stability of Mutant Luciferases

The bioluminescence stability as well as the high bioluminescence intensity is an important enzymatic property of ideal luciferase. Therefore, in order to examine the stability of the bioluminescence of the luciferase variants having high bioluminescence intensity, which was obtained in the present invention, time-course of the bioluminescence intensity after the introduction of the substrate were measured (Fig. 7).
First, a gene encoding each GLuc variant was cloned into pcDNA3.1(+), and the aliquots of the plasmids were introduced into COS-7 cells. The cells were collected 16 hours after the introduction, lysed with a cell lysis buffer, and then transferred to a microplate reader. A PBS buffer in which coelenterazine is dissolved was set to a substrate injector of the plate reader. At the measurement, after the substrate was automatically injected into each well, changes in the bioluminescence intensities were measured every 0.05 seconds. Specifically, the enzymatic activities of GLuc itself, F89W, I90L, H95E, and Y97W were measured. As a result, variants such as I90L, H95E, and Y97W showed relatively stable bioluminescence. In contrast, GLuc and F89W showed relatively unstable bioluminescence intensity (Fig. 7A).
Further, in order to examine changes in the bioluminescence intensity over a longer time span, the bioluminescence intensities by I90L, F89W, and I90V were observed during 10 minutes after introduction of the substrate, and the optical values relative to the original GLuc were illustrated (Fig. 7B). As a result, it was observed that while I90L and I90V were stable, F89W and native GLuc itself were relatively unstable, and the bioluminescence intensities thereof were decreased by about 60% within 10 minutes after introduction of the substrate. From this result, it was confirmed that not only high bioluminescence intensity but also high stability are ensured when isoleucine (I) at position 90 is mutated to leucine (L) or valine (V).
Further, in order to examine the stability of the bioluminescence intensity, optical properties of each of the variants, I90L, F89W/I90L, and I90L/I115L, were examined using a substrate solution based on a modified Matthew's buffer. Fig. 7C shows the results exhibiting the time-courses of the bioluminescence intensities during a short time span (5 seconds). Fig. 7D shows the results, after stepwise introduction of the substrate, exhibiting time-courses of the bioluminescence intensities every second at each stage. The results show that each of the variants (I90L, F89W/I90L, and I90L/I115L) demonstrated a very quick response time after the introduction of the substrate, and the bioluminescence intensity was also much higher than that of the original GLuc. These experimental results also indicate that each of these variants shows a very stable and strong bioluminescence.
Note that the composition of the modified Matthew's buffer contains the following components:
100 mM potassium phosphate, 500 mM sodium chloride, 1 mM ethylenediaminetetraacetic acid (EDTA), 0.15 mg/ml porcine gelatin, pH 5.0, 1% Nonylphenol ethoxylates (Tergitol NP-9) and 1% Antifoam, 25 mM thiourea, and 0.1% DMSO.

### Example 5 Synthesis of Further Modified Variants

The above synthetic GLuc was synthesized based on the above study on mutation to create a further modified gene (Fig. 8). The I90L variant of the synthetic GLuc shown in Fig. 8A was: (A) modified to resemble codons of human genes (GC-rich) for optimal expression in human cells, based on the information on the proportion of human codons acquired from GenBank at NCBI; and (B) restriction enzyme site was added to the middle or end of the gene in order to enhance the availability of the gene as a GLuc reporter. As shown in Fig. 8A, SacII and EcoRV were introduced at base positions 312 and 340, respectively. These sites were found as appropriate dissection sites by the present inventors in their previous study (Non-patent Literature 12), and are the optimal insertion sites for protein insertion. Additionally, BamHI was introduced at the end of the gene in order for this site to be effectively used as a multicloning site when binding to DNAs of other proteins.
Based on the findings obtained from the experimental results in Examples 1 to 7, there are four modification sites including I90L for desired GLuc mutations. Fig. 8B shows the preparation of a GLuc enzyme variant (Mon3: F89W/I90L/H95E/Y97W) with the mutations introduced into all four sites, using human-optimized codons. A specific modification strategy for Mon3 is as follows.
(A) First, because GLuc itself has an extracellular secretion signal at the N-terminal, KDEL as an ER localization signal must be linked to the end of the sequence in order to retain GLuc in the cells.
(B) It has been found that the putative enzyme active site of GLuc is located in the center of the enzyme, and that the key sites that are particularly involved in the activity are distributed from F89 to I131.
(C) Amino acids that are particularly important in the enzyme activity and properties of GLuc are indicated by underlines in the sequence.
(D) A restriction enzyme site must be provided at the center (enzyme active site) of the DNA sequence encoding GLuc enzyme so as to facilitate easy preparation of a bioluminescence probe that can be cleaved by the enzyme at a later date. For this reason, introducing an EcoRV site within the D114 and I115 region is recommended.
(E) Introducing a BamHI site at the end of GLuc for facilitating fusion with other gene sequences is also effective.
Examples of experiments that used Mon3 are shown in Figs. 4B and 4D. These figures show that Mon3 has an extremely high bioluminescence activity level, and demonstrated a shift to the long wavelength side (a long-wavelength shift of 35 nm at maximum).

### Example 6 Examination of Substrate Selectivity of Each GLuc Variant

The substrate selectivity of the GLuc variants obtained in the present invention were examined (Fig. 9). COS-7 cells were cultured in a 24-well plate. Thereafter, a plasmid encoding each GLuc variant was introduced into the cells, further followed by incubation for 16 hours. Subsequently, 30 µL of cell lysate was added to the cells. Afterward, the bioluminescence intensity of each variant was measured under various conditions of coexistence of substrates. Fig. 9A shows the results.
The results confirmed that each variant has substrate selectivity. Specifically, each of the variants, including the original GLuc itself, exhibited strong bioluminescence only in the coexistence of coelenterazine (CTZ), coelenterazine fcp, or coelenterazine ip. On the other hand, strong bioluminescence was not shown in the coexistence of coelenterazine cp, coelenterazine hcp, or coelenterazine 400A.
Fig. 9B shows the typical spectra, and Fig. 9C shows the highest bioluminescence intensity according to each substrate. The results show that the spectrum of each of the variants, including the original GLuc itself, is influenced by the type of substrate. Depending on the substrate, some variants showed a shift of the bioluminescence intensity to the long wavelength side, while other showed a shift to the short wavelength side. From these results, it was found that the luminescence of each variant can be shifted to the longer or shorter wavelength sides by changing the substrate.

### Example 7 Reporter-Gene Assay Carrying GLuc Variant

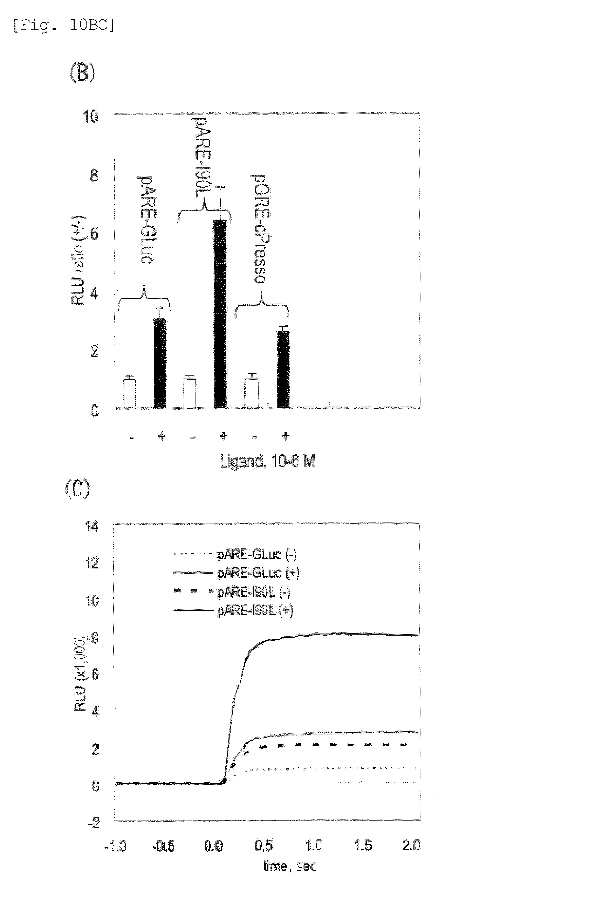

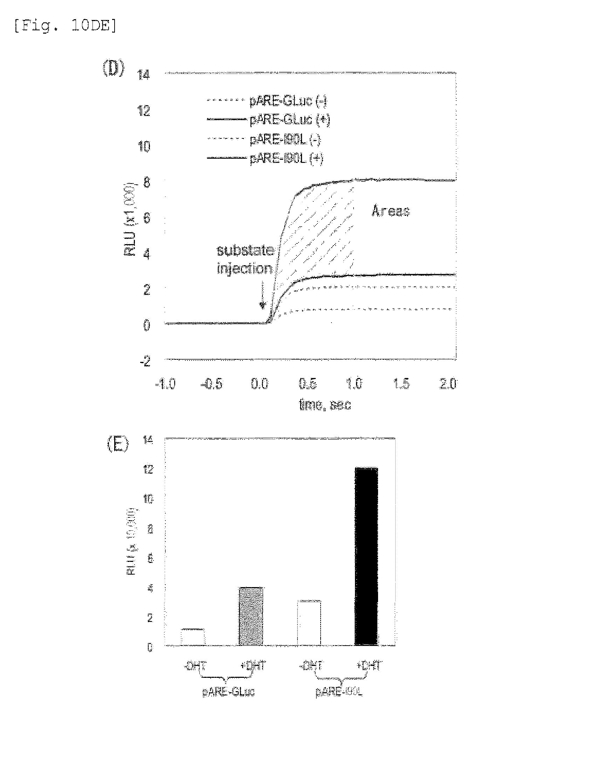

In order to prove the usability of the GLuc variants developed in the present invention, a novel reporter-gene assay system was constructed by adding the GLuc variants to pG5Luc (Promega), which is a conventional reporter-gene assay system (Fig. 10). First, a vector system having an androgen response element (ARE) in the upstream was created, and a gene encoding the original GLuc or GLuc variant of the present invention was linked to the vector system at the downstream by genetic engineering (Fig. 10A). This novel vector was introduced into COS-7 cells, and the difference in the bioluminescence intensities was measured using a luminometer under the conditions of presence and absence of androgen (male hormone) (Fig. 10B).
As a result, the reporter-gene assay system carrying the GLuc variant of the present invention indicated a better signal-to-noise ratio than the reporter-gene assay system carrying the original GLuc, and also showed an absolute bioluminescence intensity about 5 times higher than the conventional case (Fig. 10B).
Further, the kinetics of the bioluminescence intensity was observed using a plate reader under similar experimental conditions (Fig. 10C). Specifically, after COS-7 cells transfected with the above vector were stimulated for 16 hours under conditions of presence and absence of male hormone (androgen), cell lysate was prepared, and a specific substrate (coelenterazine) was added thereto. Then, the difference in the bioluminescence intensity was immediately measured using a plate reader.
In Fig. 10C above, changes in the bioluminescence intensity can be quantitatively compared by the area from when the substrate was introduced to when a certain time span (1 second) has passed. The activity level of male hormone being tested can be accurately quantified from the change in the bioluminescence intensity of the reporter gene (I90L) by subtracting the area defined by the control (GLuc) from the area defined by the reporter gene (I90L) (Fig. 10D). Further, the areas on the bioluminescence intensity graph in Fig. 10D were compared. As a result, it was confirmed that the transformed cells transfected with pARE-I90L has a larger area (higher bioluminescence intensity) (Fig. 10E).

### Example 8 Two-Hybrid Assay Carrying GLuc Variant

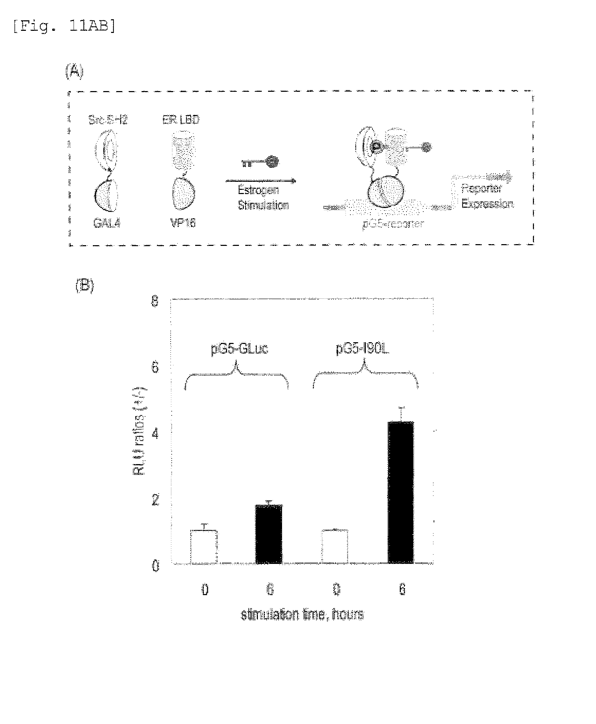

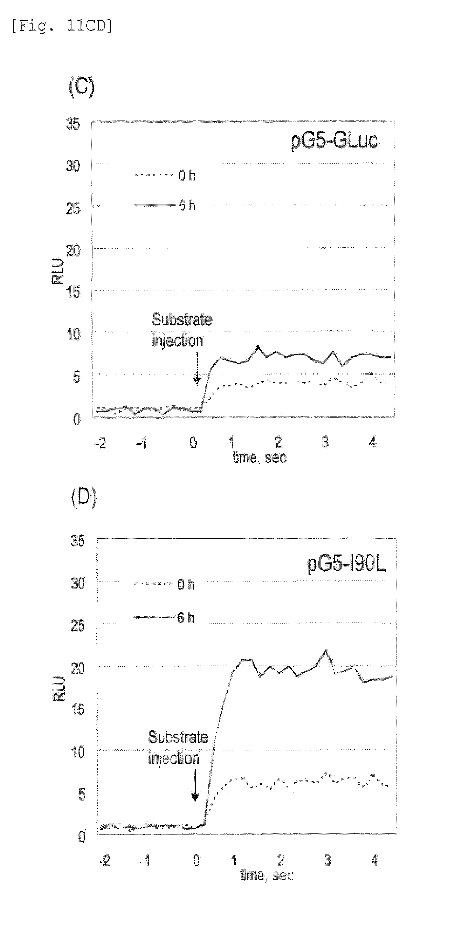

In order to prove the usability of the GLuc variants, an eukaryotic two-hybrid assay system carrying the GLuc variants was constructed, and an experiment to measure the protein-protein interaction was performed (Fig. 11).
In order to perform the experiment, first, the following plasmids were prepared based on pACT, pBIND, and pG5Luc, which are the conventional plasmids for a typical two-hybrid assay: (1) plasmid encoding ER LBD and BIND, (2) plasmid encoding SH2 domain and ACT, and (3) pG5 plasmid carrying the conventional GLuc or the highly luminescent variant (I90L) of the present invention as a reporter gene.
Living cells into which the above plasmids (1), (2), and (3) were concurrently introduced were stimulated with the solvent itself (0.5% DMSO) and woman sex hormone (estrogen), separately, for 0 hours and 6 hours. Subsequently, the cells were collected, and the bioluminescence intensities were measured using a luminescence spectrophotometer (Fig. 11B). As a result, the cells having the plasmid carrying the GLuc variant of the present invention indicated a superior S/N ratio when stimulated with woman sex hormone. For example, in the case of the plasmid carrying GLuc variant, the bioluminescence intensity was raised up to 4-fold by the 6-hour stimulation; however, in the case of the conventional plasmid, the increase in the bioluminescence intensity was not significant.
Further, time courses in the bioluminescence intensity were measured using a plate reader (Figs. 11C and 11D). As a result, an increase in the very strong bioluminescence was observed in the case of the plasmid carrying the GLuc variant.
Additionally, the difference in the bioluminescence depending on the estrogen concentration was observed (Fig. 12). For this experiment, COS-7 cells were cultured in a 96-well plate. In addition to the genes encoding ERLBD-BIND and SH2-ACT, pG5-GLuc that expresses the original GLuc itself was introduced into the control group.
In contrast, in addition to the genes encoding ERLBD-BIND and SH2-ACT, pG5-I90L that expresses the relevant GLuc variant was introduced into the experimental group. After cultivation for 12 hours, estrogen stimulation was performed, followed by cultivation for an additional 8 hours. Subsequently, a lysis buffer was added to the cells, and the bioluminescence intensity was measured for 2 seconds after the substrate was introduced with the automated program.
As a result, in comparison to the control group, the experimental group showed a significant difference in the bioluminescence intensity even by 10⁻⁷ M estrogen stimulation. The degree of luminescence was more remarkable as the concentration of estrogen was higher. As a result, the superior luminescence properties of the relevant GLuc variant led to an improvement in the detection limit.
Next, the living cells into which the above plasmids (1), (2), and (3) were concurrently introduced were stimulated with the solvent itself (0.5% DMSO) or woman sex hormone (estrogen) for 0 hours, 3 hours, 6 hour, and 18 hours. Subsequently, the cells were collected, and the bioluminescence intensities were measured using a luminescence spectrophotometer (Fig. 11E). As a result, the transformed cells having the plasmid carrying one of the GLuc variants of the present invention indicated a superior S/N ratio when stimulated with a woman sex hormone. For example, in the case of the plasmid carrying one of the GLuc variants, the bioluminescence intensity was raised up to 2-fold by the 3-hour stimulation; however, in the case of the plasmid carrying the conventional enzyme, an increase in the bioluminescence intensity was indistinctive. This result shows that, because the GLuc variant prepared by the present inventors has high bioluminescence intensity, a distinctive increase in strong luminescence was observed even when the stimulation was performed for a significantly reduced time span (i.e., even when the expression level was low) compared to the conventional case.

### Example 9 Development of Single-Molecule-Format Bioluminescent Probes Carrying GLuc Variants

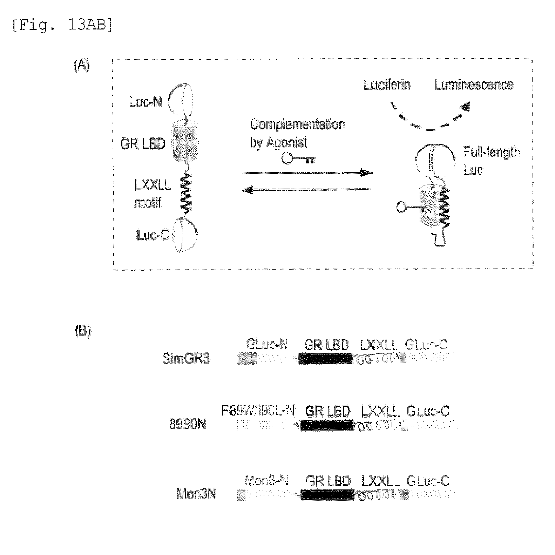

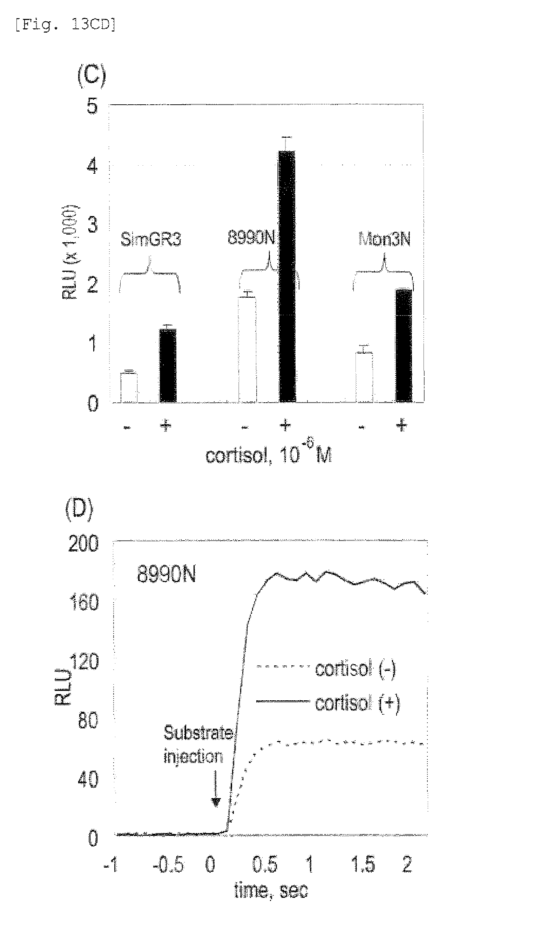

In order to further examine the usability of the highly-luminescent variants of the present invention, a single-molecule-format bioluminescent probe was synthesized based on this variant (Fig. 13). For the synthesis, first, the variant having high bioluminescence intensity (GLuc variant having F89W/I90L mutations) and GLuc variant having four mutations (F89W/I90L/H95E/Y97W) were both dissected into two fragments, and AR LBD and an LXXLL motif were inserted into the space (a probe having the former variant was named 8990N, and a probe having the latter variant was named Mon3N). These probes were inserted into living cells, and the difference in the bioluminescence intensity was measured under conditions of presence and absence of male hormone. Further, a control probe was prepared by dissecting the original GLuc itself into two fragments in a similar manner and inserting GR LBD and an LXXLL motif into the space (named SimGR3), and the S/N ratio was measured under conditions of presence and absence of stress hormone (cortisol).
Fig. 13A shows the operating principle of this probe. First, a stress hormone receptor recognizes the stress hormone, and causes an alteration in the structure. As a result, the receptor binds to the adjacent LXXLL motif, which thereby causes recombination of the fragments of the dissected luciferase. At this time, the activity of the stress hormone can be measured using the reversed enzyme activity as the index.
The sensitivity of SimGR3, 8990N, and Mon3N prepared for this experiment to the stress hormone was measured (Fig. 13B). As a result, 8990N carrying the GLuc variant of the present invention showed strong bioluminescence, and the sensitivity thereof was not inferior to that of the probe carrying the original GLuc. The luminescence properties of 8990N also showed similar results in the measurement of time courses in the bioluminescence intensity (Fig. 13C). Further, as a result of the measurement of the luminescent values of the conventional SimGR3 and 8990N of the present invention using a 610 nm long-pass filter, 8990N showed luminescence properties superior to those of the original SimGR3. In contrast, in the case of the conventional SimGR3, the difference in the bioluminescence intensities was not significant enough to distinguish between the presence and absence of stress hormone (Fig. 13D).

### Example 10 Measurement of Luminescence Activities of MpLuc1 and its Variants

Further, to test the general applicability of the enzyme active site and the mutated sites, which were found in the present invention, on marine luciferases, a group of novel bioluminescence enzyme variants based on Metridia pacifica luciferases (MpLuc1), which is an example of marine animals, was prepared (Fig. 14).
Specifically, the following were synthesized by genetic engineering: original MpLuc1 itself, MpLuc1 with a mutation introduced into Y113 (hereinafter named "Y113F"; this corresponds to F89 of GLuc), MpLuc1 with a mutation introduced into I114 (hereinafter named "I114L"; this corresponds to 190 of GLuc), and a variant transfected with four mutations including I114L of MpLuc1 (this variant carries Y113W, I114L, H119E, and Y121W mutations, and is named "MpLuc4"; and these mutations correspond to F89, I90, H95, and Y97 of GLuc, respectively). These variants were individually introduced in equal amounts into living cells, and their relative bioluminescence intensities were measured.
First, the results of the measurement of the luminescence spectrum of each variant show that I114L exhibits a higher bioluminescence intensity compared to the original MpLuc1. It was also observed that MpLuc4 showed a spectrum shift to the longer wavelength side, compared to the original MpLuc1 (Fig. 14A). Further, as a result of the examination of the bioluminescence intensity of the above variant using a 610 nm long-pass filter, the variant was found to show stronger bioluminescence, compared to the original MpLuc1 (Fig. 14B). Still further, time courses in the luminescence properties after the introduction of the substrate were observed using a microplate reader in order to examine the luminescence properties of each variant, and the results showed that each variant demonstrates stable bioluminescence, compared to the original MpLuc1 (Fig. 14C). Unlike other variants, MpLuc4 in particular was found to sustain 60% of its initial bioluminescence intensity in 10 seconds after the introduction of the substrate.

### Example 11 Measurement of Luminescence Activities of MLuc and its Variants

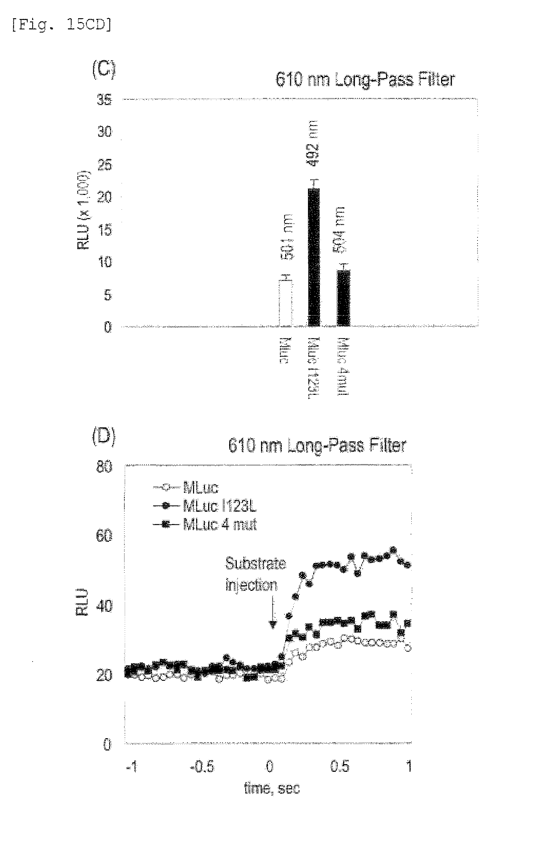

To test the general applicability of the enzyme active site and the mutated sites, which were found in the present invention, on marine luciferases, a novel luciferase variant based on Metridia longa luciferase (MLuc), which is an example of marine animals, was prepared (Fig. 15).
Specifically, the following were synthesized by genetic engineering: original MLuc itself, MLuc with I123L mutation introduced (hereinafter named "I123L"; this corresponds to I90 of GLuc), and a variant transfected with four mutations including I123L of MLuc (named "MLuc4"; four mutation sites are Y122W, I123L, H128E, and Y130W, which correspond to F89, I90, H95, and Y97 of GLuc, respectively). These variants were individually introduced in equal amounts into living cells, and their relative luminescence spectra were measured. As a result, it was found that the bioluminescence intensity of I123L was increased compared to the original MLuc because of the introduction of the mutation (Fig. 15A). It was also found that the position of the maximum bioluminescence intensity (λ max) also varies due to the introduction of the mutation. In order to examine the luminescence stability of the variants produced in the present invention, time courses in the bioluminescence intensity after the introduction of the substrate were measured every 0.5 seconds (Fig. 15B). As a result, MLuc variant (MLuc4) with mutations introduced into 4 positions showed the most stable bioluminescence. In contrast, the original MLuc was found to lose 90% of the initial bioluminescence intensity only 10 seconds after the introduction of the substrate. Further, in order to examine the long-wavelength bioluminescence intensities of the variants, the bioluminescence intensities of the variants were measured using a 610 nm long-pass filter. The results show that I123L produces strong bioluminescence in the red wavelength. Results similar to the above tendency were obtained from the measurement of the bioluminescence intensities before and after the introduction of the substrate (Fig. 15D). It was found that the enzyme variants into which the variants were introduced based on the findings of the present invention show bioluminescence properties superior to those of the original type.

### Example 12 In Vivo Imaging Using GLuc Variants

Taking advantage of the improved properties (high bioluminescence intensity, a longer-wavelength shift, stability, and the like) of the GLuc variants, the applicability thereof to the biological system was examined (Fig. 16).
First, pcDNA3.1 plasmid encoding 8990, which is a GLuc variant having high bioluminescence intensity, was produced and introduced into COS-7 cells. At the same time, as a control, pcDNA3.1 plasmid encoding the original GLuc itself was similarly introduced into COS-7 cells. Subsequently, both of these transformed cells were transplanted into subcutaneous tissues of the back on the left and right sides of BALB/c nude mice (5-week-old female). After waiting 12 hours to allow stabilization of the transplanted cells, an equal amount of substrate (coelenterazine) was injected. Subsequently, the developed bioluminescence intensities were compared (Fig. 16A). As a result, stronger bioluminescence was observed at the site into which the transformed cells that express 8990 were transplanted.

Further, living cells carrying a mammalian two-hybrid system that expresses the GLuc variant were prepared, and an in *vivo* imaging system was established using the prepared living cells (Fig. 16B).
First, two plasmids (pBIND and pACT) in which a woman sex hormone receptor (ER LBD) binds to BIND and Src SH2 domain binds to ACT, respectively, were prepared. At the same time, as a signal responsive system, a plasmid containing a Gal4-responsive element in the upstream and expressing I90L was also prepared (pG5-I90L). As a control, a plasmid that expresses the original GLuc itself was prepared (pG5-GLuc).

The above three plasmids (pBIND, pACT, and pG5-I90L or pG5-GLuc) were co-transfected into COS-7 cells, thereby preparing transformed cells. The transformed cells were transplanted into subcutaneous tissues of the back on the left and right sides of BALB/c nude mice (5-week-old female). At this time, the control cells (pG5-GLuc) were transplanted into the left back side, and the transformed cells carrying pG5-I90L were transplanted into the right back side. A 12-hour waiting time was further allowed for stabilization of the transplanted cells.
Subsequently, woman sex hormone or a solvent (0.1% DMSO) was injected into each mouse, and the bioluminescence intensity was observed 6 hours after the injection (Fig. 16B). As a result, the mouse stimulated with the female hormone showed stronger bioluminescence. Additionally, in the mouse stimulated with the female hormone, the right back side (expressing I90L) showed stronger bioluminescence than the left side (expressing GLuc). This result can be analyzed as confirming that even when the same amount of GLuc or I90L was expressed by woman sex hormone stimulation, I90L showed stronger bioluminescence because the luminescence of I90L is higher and more stable.

### Industrial Applicability

The measurement efficiency of assays is dramatically improved by using luciferase of the present invention in measurements of ligands based on the conventionally widely used reporter-gene assays, or by using them as an alternative of bioluminescence enzymes in the conventional bioluminescence probes. Accordingly, the luciferase of the present invention can be widely used in applications such as basic biological studies, and development of diagnostic reagents in medical and pharmaceutical sciences, and analytical chemistry.

## Claims

1. A luciferase variant with improved optical property obtained by replacing at least one amino acid residue among the amino acid sequence of a marine luciferase at positions corresponding to 89 to 118 in the amino acid sequence of Gaussia luciferase (GLuc), wherein an amino acid residue at a position corresponding to at least one position selected from positions 89, 90, 95, 97, 100, 108, 112, 115, and 118 is replaced by way of conservative amino acid replacement.

2. The luciferase variant according to claim 1, wherein the conservative amino acid replacement includes replacement of a hydrophobic amino acid residue at a position corresponding to positions 89, 90, 97, 108, 112, 115, or 118 in the amino acid sequence of GLuc with another hydrophobic amino acid residue, or replacement of a hydrophilic amino acid residue at a position corresponding to positions 95 or 100 in the amino acid sequence of GLuc that forms a hydrogen bond with another hydrophilic amino acid residue.

3. The luciferase variant according to claim 2, wherein the replacement of a hydrophobic amino acid residue with another hydrophobic amino acid residue includes replacement of a hydrophobic and nonpolar aliphatic amino acid residue at a position corresponding at position 90, 108, 112, 115, or 118 in the amino acid sequence of GLuc with another hydrophobic and nonpolar aliphatic amino acid residue, or replacement of a hydrophobic aromatic amino acid residue at a position corresponding to position 89 or 97 in the amino acid sequence of GLuc with another hydrophobic aromatic amino acid residue.

4. The luciferase variant according to claim 3, wherein the replacement of a hydrophobic and nonpolar aliphatic amino acid residue with another hydrophobic and nonpolar aliphatic amino acid residue is replacement of isoleucine with an amino acid residue selected from leucine, tryptophan, and valine.

5. The luciferase variant according to claim 3, wherein the replacement of a hydrophobic aromatic amino residue with another hydrophobic aromatic amino residue is replacement of an amino acid residue at a position corresponding to position 89 or 97 in the amino acid sequence of GLuc with tryptophan (W).

6. The luciferase variant according to claim 2, wherein the replacement of a hydrophilic amino acid residue at a position corresponding to position 95 or 100 in the amino acid sequence of GLuc that forms a hydrogen bond with another hydrophilic amino acid residue is replacement of an amino acid residue at a position corresponding to position 95 in the amino acid sequence of GLuc with glutamic acid (E), or replacement of an amino acid residue at a position corresponding to position 100 in the amino acid sequence of GLuc with asparagine (N).

7. A luciferase variant obtained by way of conservative amino acid replacement that includes replacement of an amino acid residue at a position corresponding to position 90 in the amino acid sequence of GLuc among the amino acid sequences of marine luciferase with leucine.

8. The luciferase variant according to claim 7, wherein the conservative amino acid replacement further includes replacement of an amino acid residue corresponding to position 89 in the amino acid sequence of GLuc with tryptophan, or replacement of an amino acid residue corresponding to position 115 in the amino acid sequence of GLuc with leucine.

9. The luciferase variant according to claim 7 or 8, wherein the conservative amino acid replacement further includes replacement of an amino acid residue at a position corresponding to position 95 in the amino acid sequence of GLuc with glutamic acid or glutamine, and replacement of an amino acid residue at a position corresponding to position 97 in the amino acid sequence of GLuc with tryptophan.

10. The luciferase variant according to any one of claims 1 to 9, wherein the marine luciferase is one luciferase selected from Gaussia luciferase (GLuc) and Copepod luciferase (MLuc, MpLuc1, and MpLuc2).

11. A DNA encoding the luciferase variant according to any one of claims 1 to 10.

12. An expression vector comprising the DNA according to claim 11.

13. A transformed cell in which the DNA according to claim 11 is introduced in a manner such that the DNA can be expressed.

14. A method for improving luminescent optical property of marine luciferase, comprising replacing at least one of the amino acid residues among the amino acid sequences of marine luciferase at positions corresponding to positions 89 to 118 in the amino acid sequence of Gaussia luciferase (GLuc), wherein an amino acid residue at a position corresponding to at least one position selected from positions 89, 90, 95, 97, 100, 108, 112, 115, and 118 in the amino acid sequence of GLuc is replaced by way of conservative amino acid replacement.

15. A bioluminescent probe with improved optical property that adopts a working mechanism of resurrecting luminescent function by reconstituting dissected luciferase, the probe using the luciferase variant according to any one of claims 1 to 10 as the luciferase.

16. A DNA encoding a bioluminescent probe according to claim 15.
